# EUROPEAN PATENT APPLICATION

(11) **EP 2 130 838 A2**
(43) Date of publication of application: **09.12.2009**
(21) Application number: 09006667.1
(22) Date of filing: 26.07.2006
(51) Int. Cl.: C07K 14/00

(54) **Zinc finger binding domains for CNN**

(30) Priority: 11.08.2005 US 707839 P
(62) Divisional of application: 06836095.7
(71) Applicant: The Scripps Research Institute, La Jolla, CA 92037 (US)
(72) Inventor: Barbas, Carols F., III, Solana Beach, CA 92075 (US); Dreier, Birgit, 8106 Regensdorf 2 Adlikon ZH (CH)
(74) Representative: Leifert & Steffan

(57) **Abstract**

Polypeptides that contain zinc finger-nucleotide binding regions that bind to nucleotide sequences of the formula CNN are provided. Compositions containing a plurality of polypeptides, polynucleotides that encode such polypeptides and methods of regulating gene expression with such polypeptides, compositions and polynucleotides are also provided.

## Description

### CROSS REFERENCE TO RELATED APPLICATIONS

This application is related to the earlier patent application, United States Patent Application Serial No. 10/487,268, filed June 18, 2004, by Barbas et al., entitled "Zinc Finger Binding Domains for CNN," which is the United States national stage of PCT Application No. PCT/US02/26388, filed August 20, 2002, which in turn claimed priority from Provisional Patent Applications Serial Nos. 60/313,864 and 60/313,693, filed Aug. 20, 2001, the disclosures of which are all incorporated herein by reference. This application also claims priority from United States Provisional Patent Application Serial No. 60/707,839, filed August 11, 2005, by Barbas et al., entitled "Zinc Finger Binding Domains for CNN," the disclosure of which is incorporated herein by reference.

### GOVERNMENT INTERESTS

Funds used to support some of the studies reported herein were provided by the National Institutes of Health (NIH GM 53910). The United States Government, therefore, may have certain rights in the invention.

### TECHNICAL FIELD OF THE INVENTION

The field of this invention is zinc finger protein binding to target nucleotides. More particularly, the present invention pertains to amino acid residue sequences within the α-helical domain of zinc fingers that specifically bind to target nucleotides of the formula 5'-(CNN)-3'.

### BACKGROUND OF THE INVENTION

The construction of artificial transcription factors has been of great interest in the past years. Gene expression can be specifically regulated by polydactyl zinc finger proteins fused to regulatory domains. Zinc finger domains of the Cys₂-His₂ family have been most promising for the construction of artificial transcription factors due to their modular structure. Each domain consists of approximately 30 amino acids and folds into an α-helical structure stabilized by hydrophobic interactions and chelation of a zinc ion by the conserved Cys₂-His₂ residues. To date, the best characterized protein of this family of zinc finger proteins is the mouse transcription factor Zif 268 [Pavletich et al., (1991) Science 252(5007), 809-817; Elrod-Erickson et al., (1996) Structure 4(10), 1171-1180]. The analysis of the Zif 268/DNA complex suggested that DNA binding is predominantly achieved by the interaction of amino acid residues of the α-helix in position -1, 3, and 6 with the 3', middle, and 5' nucleotide of a 3 bp DNA subsite, respectively. Positions 1, 2 and 5 have been shown to make direct or water-mediated contacts with the phosphate backbone of the DNA. Leucine is usually found in position 4 and packs into the hydrophobic core of the domain. Position 2 of the α-helix has been shown to interact with other helix residues and, in addition, can make contact to a nucleotide outside the 3 bp subsite (Pavletich et al., (1991) Science 252(5007), 809-817; Elrod-Erickson et al., (1996) Structure 4(10), 1171-1180; lsalan, M. et al., (1997) Proc Natl Acad Sci USA 94(11), 5617-5621].

The selection of modular zinc finger domains recognizing each of the 5'-(GNN)-3' DNA subsites with high specificity and affinity and their refinement by site-directed mutagenesis has been demonstrated (U.S. Pat. No. 6,140,081, the disclosure of which is incorporated herein by reference). These modular domains can be assembled into zinc finger proteins recognizing extended 18 bp DNA sequences which are unique within the human genome or any other genome. In addition, these proteins function as transcription factors and are capable of altering gene expression when fused to regulatory domains and can even be made hormone-dependent by fusion to ligand-binding domains of nuclear hormone receptors. To allow the rapid construction of zinc finger-based transcription factors binding to any DNA sequence it is important to extend the existing set of modular zinc finger domains to recognize each of the 64 possible DNA triplets which are assigned meaning in the genetic code, including the three triplets that code for protein termination. This aim can be achieved by phage display selection and/or rational design. Due to the limited structural data on zinc finger/DNA interaction, rational design of zinc proteins is very time-consuming and may not be possible in many instances. In addition, most naturally occurring zinc finger proteins consist of domains recognizing the 5'-(GNN)-3' type of DNA sequences. The most promising approach to identify novel zinc finger domains binding to DNA target sequences of the type 5'-(NNN)-3' is selection via phage display. The limiting step for this approach is the construction of libraries that allow the specification of a 5' adenine, cytosine or thymine in the subsite recognized by each module. Phage display selections have been based on Zif268 in which different fingers of this protein were randomized [Choo et al., (1994) Proc. Natl. Acad. Sci. U.S. A. 91 (23), 11168-72; Rebar et al., (1994) Science (Washington, D.C., 1883-) 263(5147), 671-3; Jamieson et al., (1994) Biochemistry 33, 5689-5695; Wu et al., (1995) PNAS 92, 344-348; Jamieson et al., (1996) Proc Natl Acad Sci USA 93, 12834-12839; Greisman et al., (1997) Science 275(5300), 657-661]. A set of 16 domains recognizing the 5'-(GNN)-3' type of DNA sequences has previously been reported from a library where finger 2 of C7, a derivative of Zif268 [Wu et al., (1995) PNAS 92, 344-348 Wu, 1995], was randomized [Segal et al., (1999) Proc Natl Acad Sci USA 96(6), 2758-2763]. In such a strategy, selection is limited to domains recognizing 5'-(GNN)-3' or 5'-(TNN)-3' due to the Asp² of finger 3 making contact with the complementary base of a 5' guanine or thymine in the finger-2 subsite [Pavletich et al., (1991) Science 252(5007), 809-817; Elrod-Erickson et al., (1996) Structure 4(10), 1171-1180].

The present approach is based on the modularity of zinc finger domains that allows the rapid construction of zinc finger proteins by the scientific community and demonstrates that the concerns regarding limitation imposed by cross-subsite interactions only occurs in a limited number of cases. The present disclosure introduces a new strategy for selection of zinc finger domains specifically recognizing the 5'-(CNN)-3' type of DNA sequences. Specific DNA-binding properties of these domains were evaluated by a multi-target ELISA against all sixteen 5'-(CNN,)-3' triplets. These domains can be readily incorporated into polydactyl proteins containing various numbers of 5'-(CNN)-3' domains, each specifically recognizing extended 18 bp sequences. Furthermore, these domains can specifically alter gene expression when fused to regulatory domains. These results underline the feasibility of constructing polydactyl proteins from predefined building blocks. In addition, the domains characterized here greatly increase the number of DNA sequences that can be targeted with artificial transcription factors.

### BRIEF SUMMARY OF THE INVENTION

In one aspect, the present invention provides an isolated and purified zinc finger nucleotide binding polypeptide that contains a nucleotide binding region of from 5 to 10 amino acid residues, which region binds preferentially to a target nucleotide of the formula CNN, where N is A, C, G or T. Preferably, the target nucleotide has the formula CAA, CAC, CAG, CAT, CCA, CCC, CCG, CCT, CGA, CGC, CGG, CGT, CTA, CTC, CTG or CTT. In one embodiment, a polypeptide of the invention contains a binding region that has an amino acid sequence with the same nucleotide binding characteristics as any of SEQ ID NOs: 1-28, 35-45, 48, 54, 57-82, 85-130, and 134-157. Such a polypeptide competes for binding to a nucleotide target with any of SEQ ID NOs: 1-28, 35-45, 48, 54, 57-82, 85-130, and 134-157. That is, a preferred polypeptide contains a binding region that will displace, in a competitive manner, the binding of any of SEQ IDS NOs: 1-28, 35-45, 48, 54, 57-82, 85-130, and 134-157. Means for determining competitive binding are well known in the art. Preferably, the binding region has the amino acid sequence of any of SEQ ID NOs: 1-28, 35-45, 48, 54, 57-82, 85-130, and 134-157. More preferably, the binding region has the amino acid sequence of any of SEQ ID NOs: 54, 57-82, 85-130, and 134-157. Still more preferably, the binding region has the amino acid sequence of any of SEQ ID NOs: 2, 14, 18, 63, 64, 65, 66, 67, 68, 69, 70, 71, 72, 73, 75, 77,78,79, 80, 81, 82,134, and 153. Even more preferably, the binding region has the amino acid sequence of any of SEQ ID NOs: 2, 14, 18, 63, 66, 71, 72, 73, 75, 77, 78, 79, 80, 81, 82, 134, and 153. Still even more preferably, the binding region has the amino acid sequence of any of SEQ ID NOs: 2, 14, 18, 63, 66, 71, 72, 73, 75, 77, 78, 79, 80, 81, and 82.

In another aspect, the present invention provides a polypeptide composition that contains a plurality of and, preferably from about 2 to about 12 of zinc finger nucleotide binding domains as disclosed herein. The domains are operatively linked such as linked via a flexible peptide linker of from 5 to 15 amino acid residues. Operatively linked preferably occurs via a flexible peptide linker such as that shown in SEQ ID NO:30. Such a composition binds to a nucleotide sequence that contains a sequence of the formula 5'-(CNN)ₙ-3', where N is A, C, G or T and n is 2 to 12. Preferably, the polypeptide composition contains from about 2 to about 6 zinc finger nucleotide binding domains and binds to a nucleotide sequence that contains a sequence of the formula 5'-(CNN)ₙ-3', where n is 2 to 6. Binding occurs with a K_{D} of from 1 µM to 10 µM. Preferably binding occurs with a K_{D} of from 10 µM to 1 µM, from 10 pM to 100 nM, from 100 pM to 10 nM and, more preferably with a K_{D} of from 1 nM to 10 nM. In preferred embodiments, both a polypeptide and a polypeptide composition of this invention are operatively linked to one or more transcription regulating factors such as a repressor of transcription or an activator of transcription.

In yet another aspect, the invention further provides an isolated heptapeptide having an α-helical structure and that binds preferentially to a target nucleotide of the formula CNN, where N is A, C, G or T. Preferred target nucleotides are as described above.

Preferably, the heptapeptide has the amino acid sequence of any of SEQ ID NOs: 1-28, 35-45, 48, 54, 57-82, 85-130, and 134-157. More preferably, the heptapeptide has the amino acid sequence of any of SEQ ID NOs: 54, 57-82, 85-130, and 134-157. Still more preferably, the heptapeptide has the amino acid sequence of any of SEQ ID NOs: 2,14,18, 63, 64, 65, 66, 67, 68, 69, 70, 71, 72, 73, 75, 77, 78, 79, 80, 81, 82, 134, and 153. Even more preferably, the heptapeptide has the amino acid sequence of any of SEQ ID NOs: 2, 14, 18, 63, 66, 71, 72, 73, 75, 77, 78, 79, 80, 81, 82, 134, and 153. Still even more preferably, the heptapeptide has the amino acid sequence of SEQ ID NOs: 2, 14, 18, 63, 66, 71, 72, 73, 75, 77, 78, 79, 80, 81, and 82.

The present invention further provides polynucleotides that encode a polypeptide or a composition of this invention, expression vectors that contain such polynucleotides and host cells transformed with the polynucleotide or expression vector.

The present invention further provides a process of regulating expression of a nucleotide sequence that contains the target nucleotide sequence 5'-(CNN)-3'. The target nucleotide sequence can be located anywhere within a longer 5'-(NNN)-3' sequence. The process includes the step of exposing the nucleotide sequence to an effective amount of a zinc finger nucleotide binding polypeptide or composition as set forth herein. In one embodiment, a process regulates expression of a nucleotide sequence that contains the sequence 5'-(CNN)ₙ-3', where n is 2 to 12. The process includes the step of exposing the nucleotide sequence to an effective amount of a composition of this invention. The sequence 5'-(CNN)ₙ-3' can be located in the transcribed region of the nucleotide sequence, in a promoter region of the nucleotide sequence, or within an expressed sequence tag. The composition is preferably operatively linked to one or more transcription regulating factors such as a repressor of transcription or an activator of transcription. In one embodiment, the nucleotide sequence is a gene such as a eukaryotic gene, a prokaryotic gene or a viral gene. The eukaryotic gene can be a mammalian gene such as a human gene, or, alternatively, a plant gene. The prokaryotic gene can be a bacterial gene.

In yet another embodiment, the invention provides a pharmaceutical composition comprising:
(1) a therapeutically effective amount of a polypeptide, polypeptide composition, or isolated heptapeptide according to the present invention as described above; and
(2) a pharmaceutically acceptable carrier.

In yet another embodiment, the invention provides a pharmaceutical composition comprising:
(1) a therapeutically effective amount of a nucleotide sequence that encodes a polypeptide, polypeptide composition, or isolated heptapeptide according to the present invention as described above; and
(2) a pharmaceutically acceptable carrier.

### BRIEF DESCRIPTION OF THE DRAWINGS

The following invention will become better understood with reference to the specification, appended claims, and accompanying drawings, where:

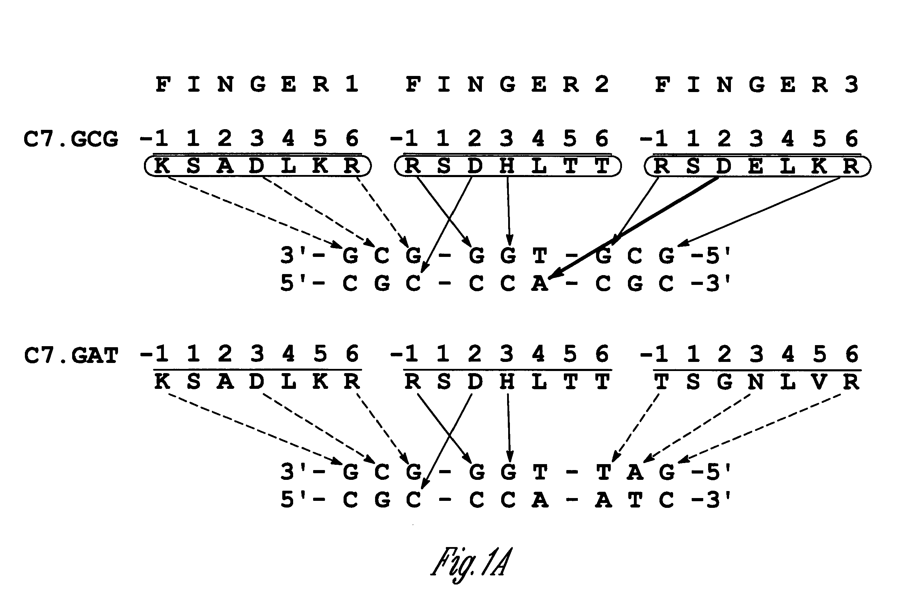

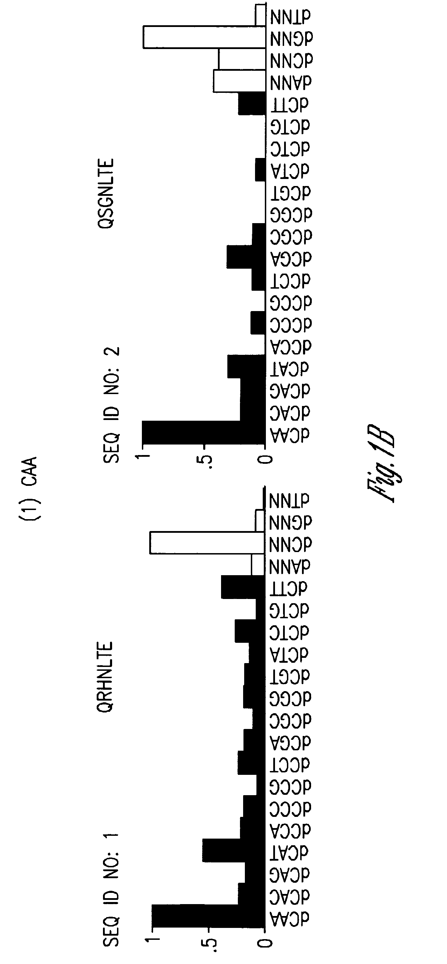

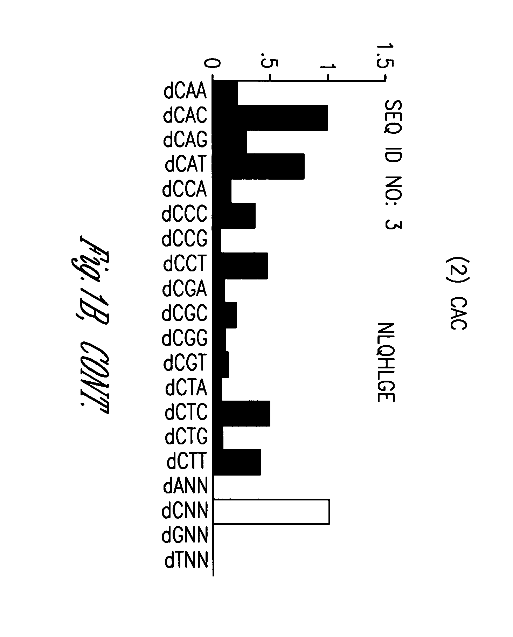

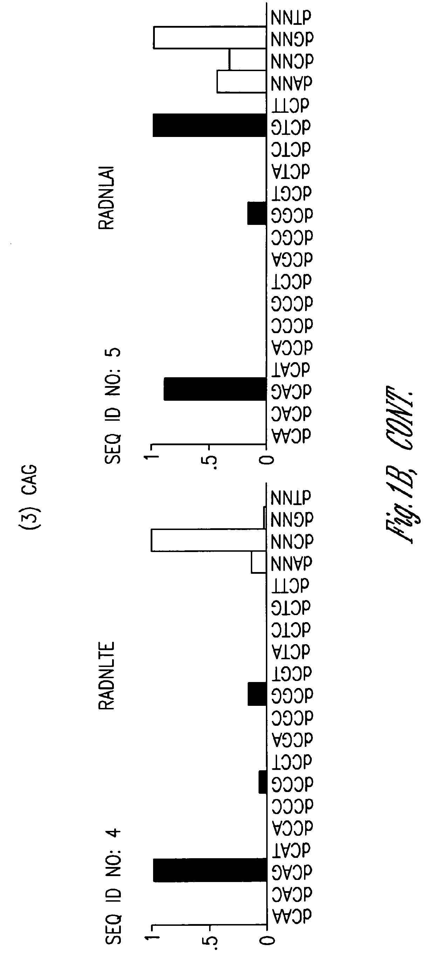

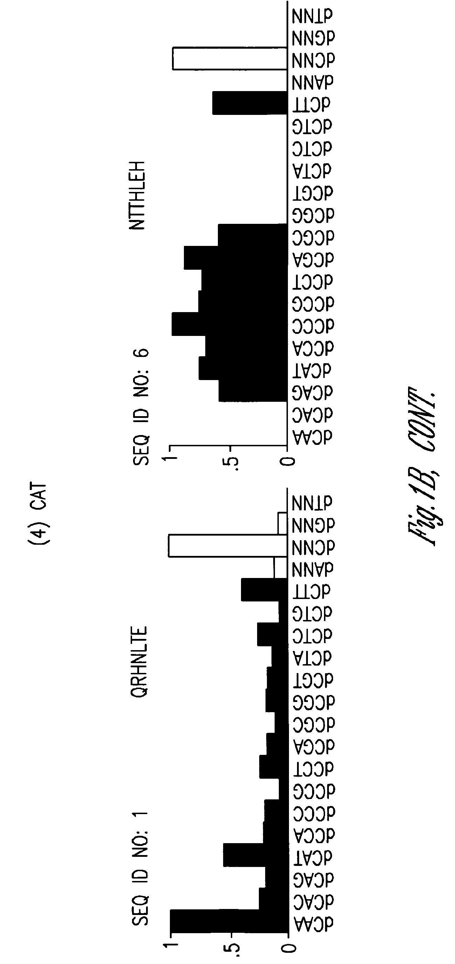

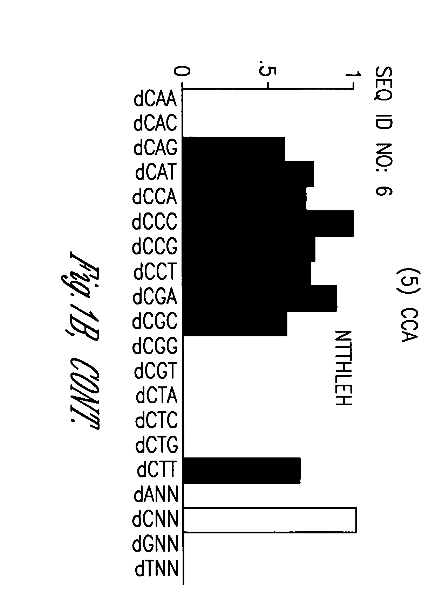

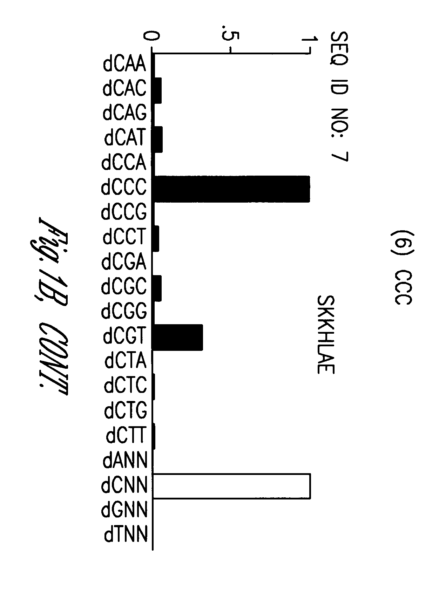

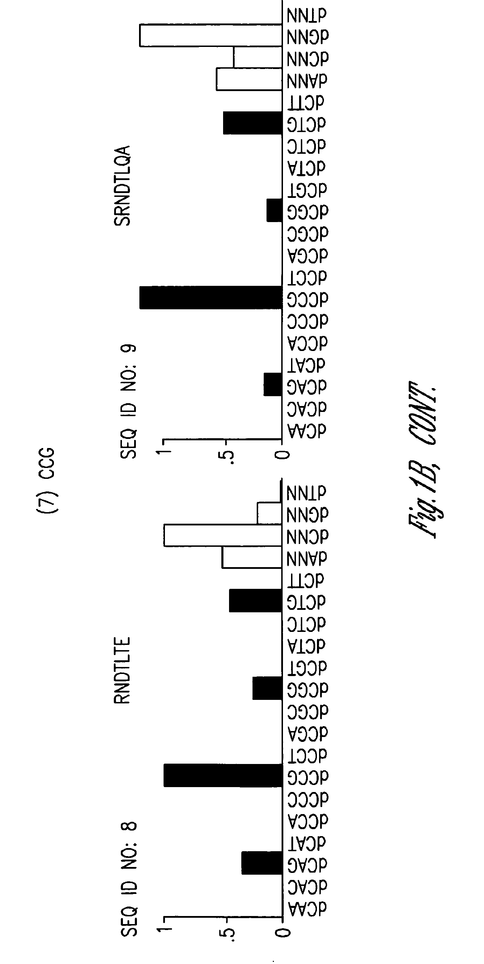

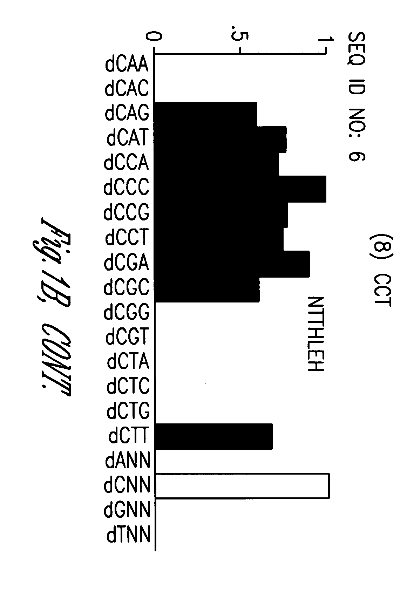

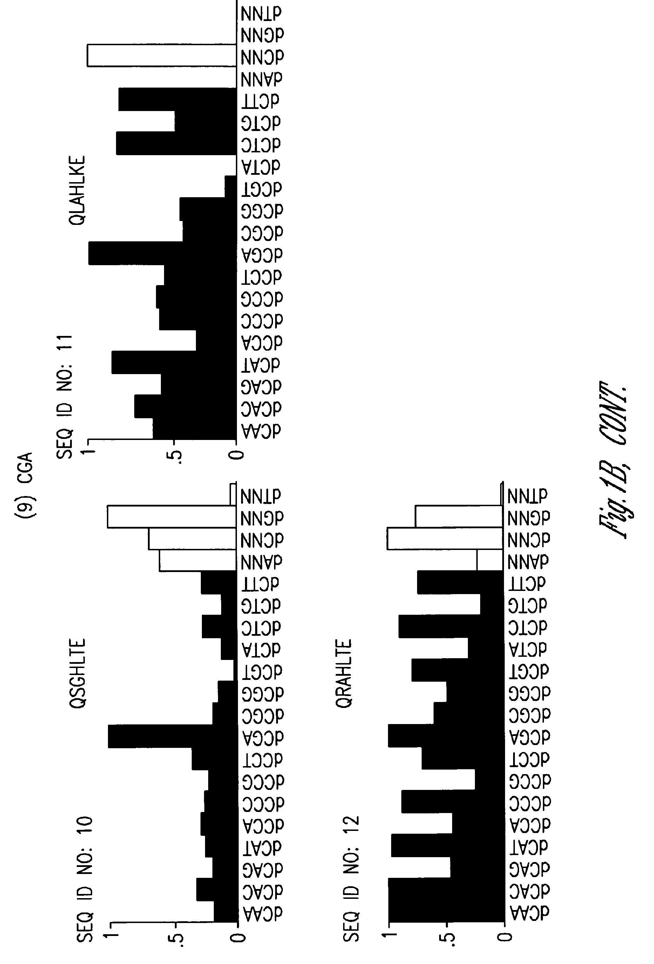

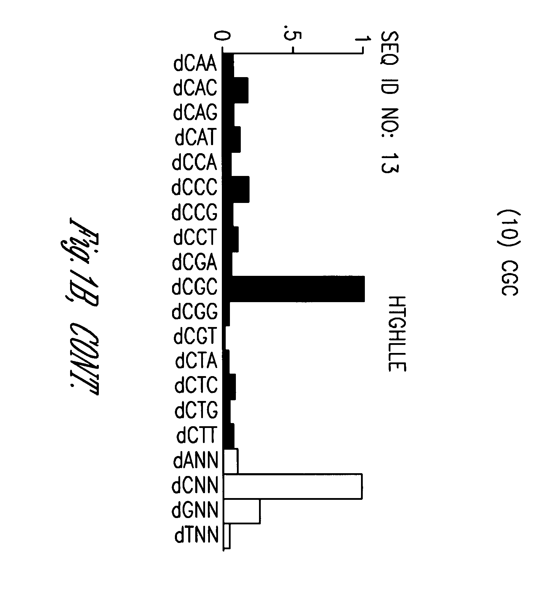

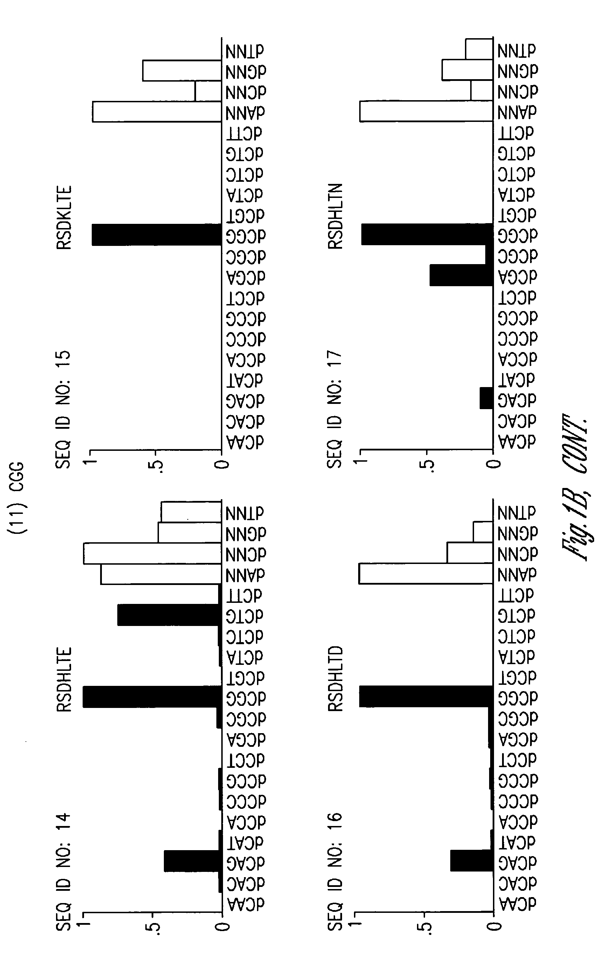

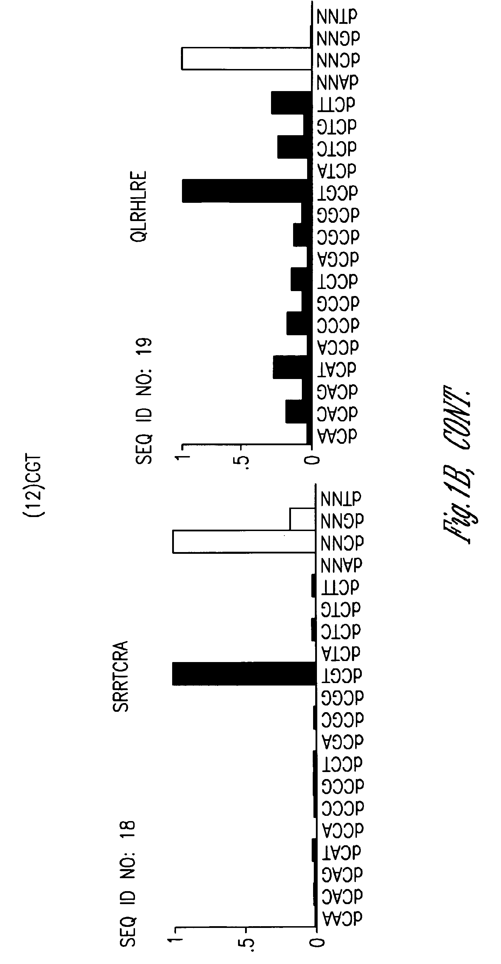

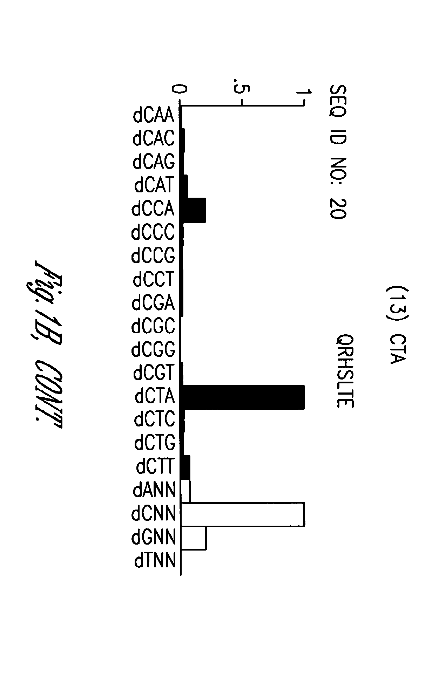

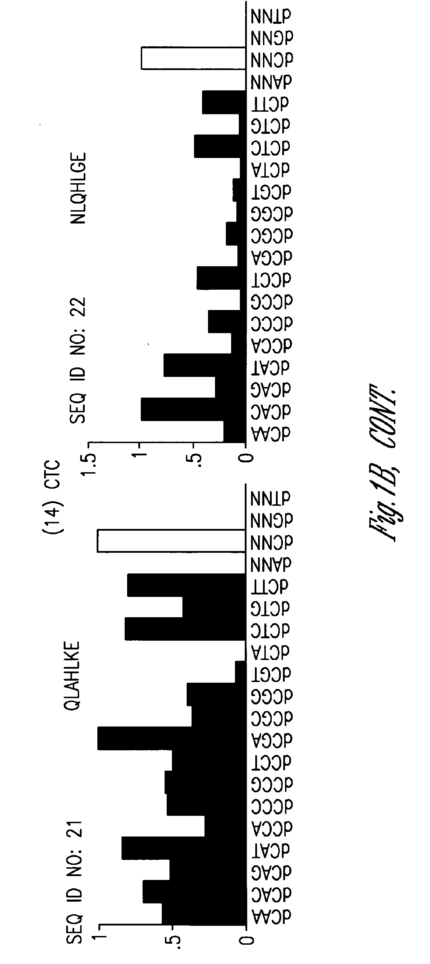

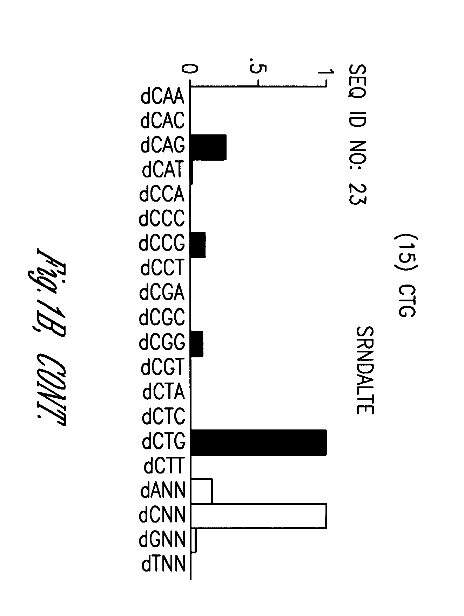

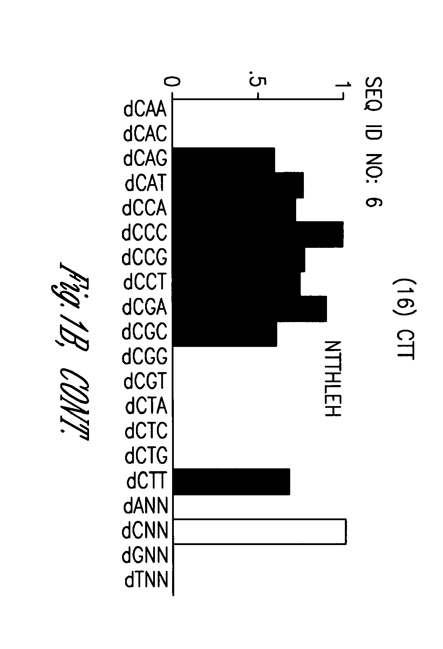

FIG. 1 shows, in two panels designated 1 A and 1B, schematically, construction of the zinc finger phage display library (A) and multitarget specificity ELISA for the C7 proteins (B): (1) 5'-(CAA)-3'; (2) 5'-(CAC)-3'; (3) 5'-(CAG)-3'; (4) 5'-(CAT)-3'; (5) 5'-(CCA)-3'; (6) 5'-(CCC)-3'; (7) 5'-(CCG)-3'; (8) 5'-(CCT)-3'; (9) 5'-(CGA)-3'; (10) 5'-(CGC)-3'; (11) 5'-(CGG)-3'; (12) 5'-(CGT)-3'; (13) 5'-(CTA)-3'; (14) 5'-(CTC)-3'; (15) 5'-(CTG)-3'; (16) 5'-(CTT)-3'.

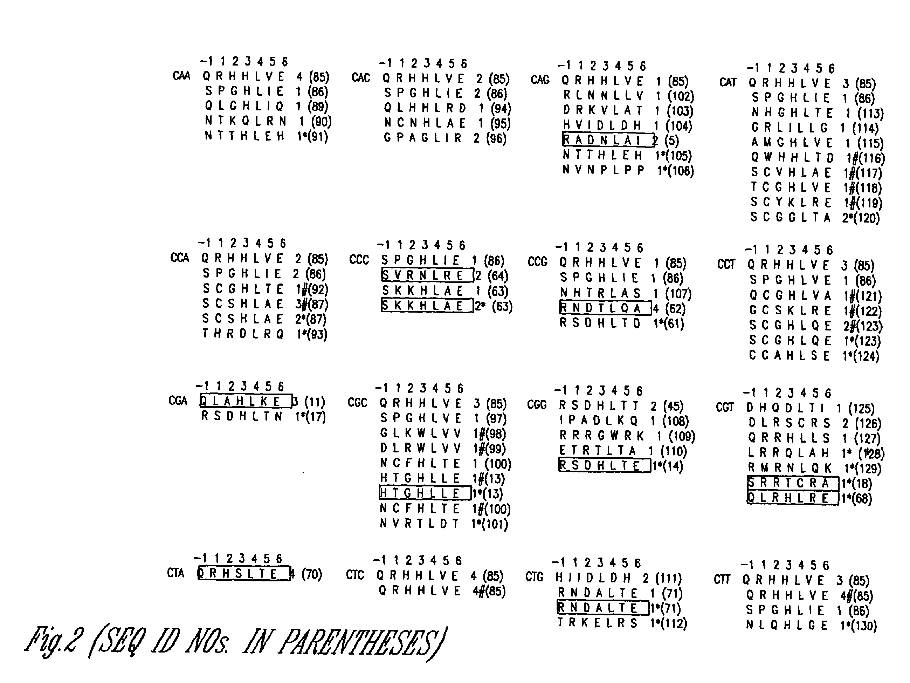

FIG. 2 shows the amino acid sequences of finger-2 recognition helices from selected clones.

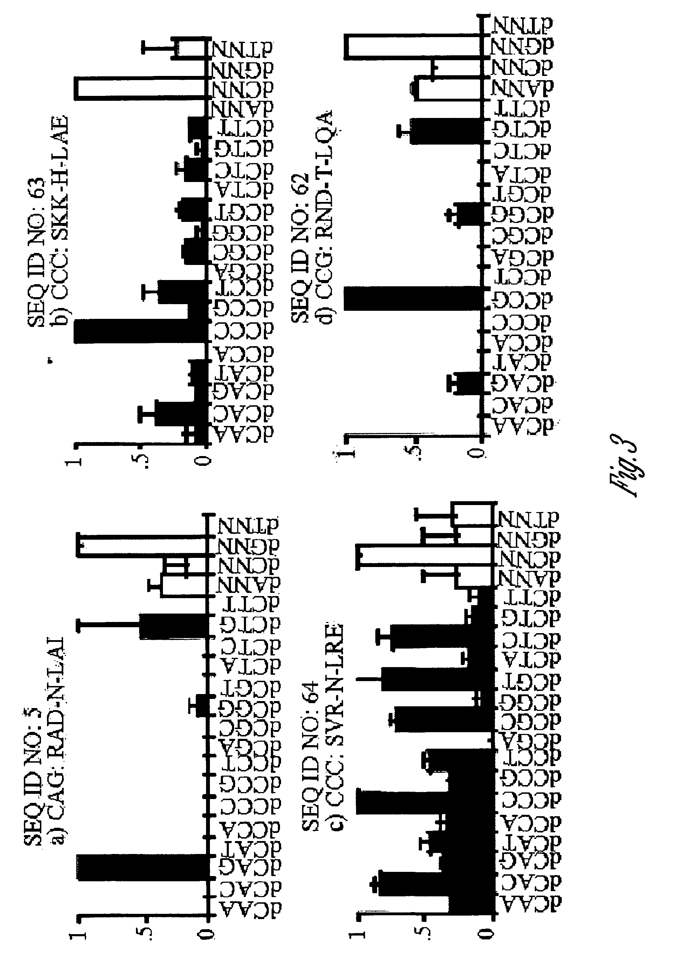

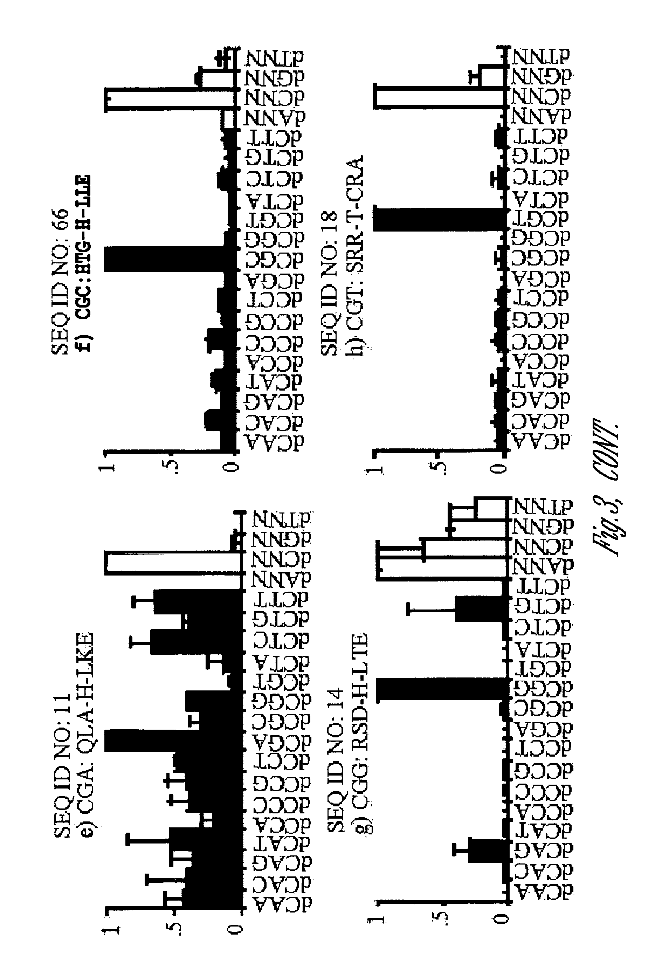

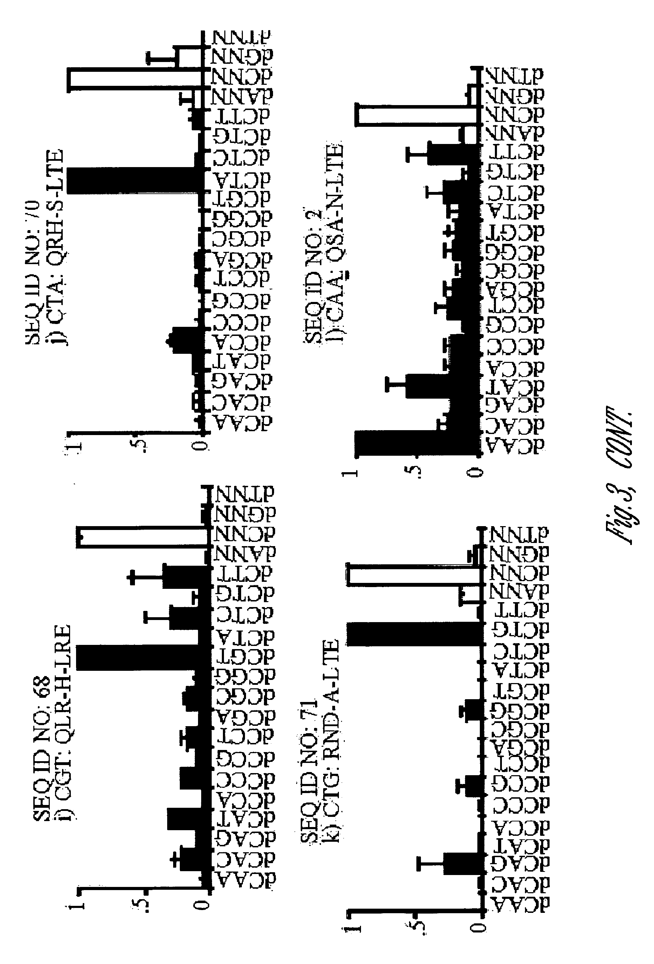

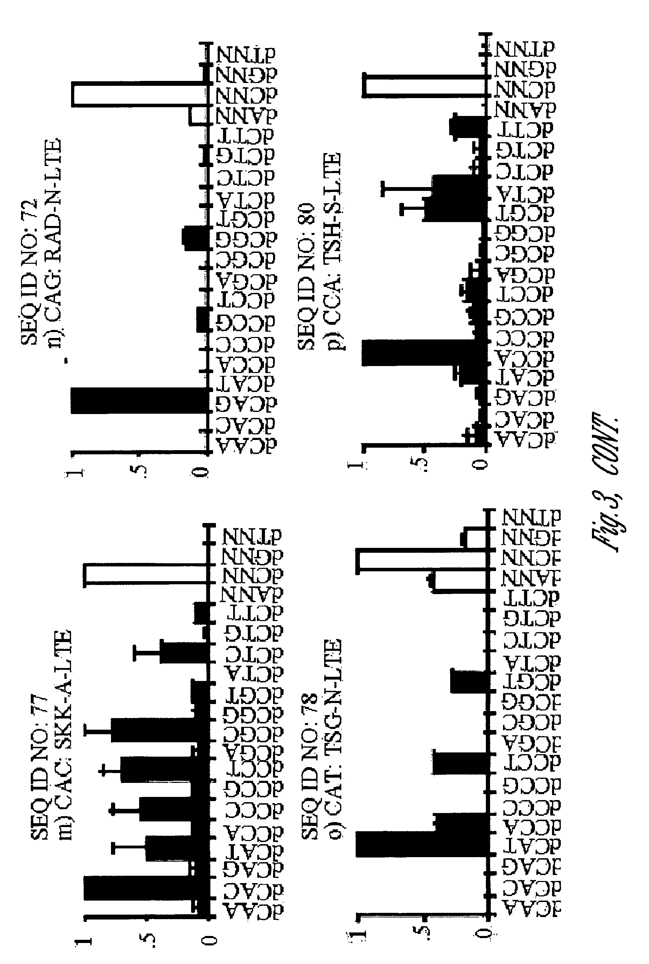

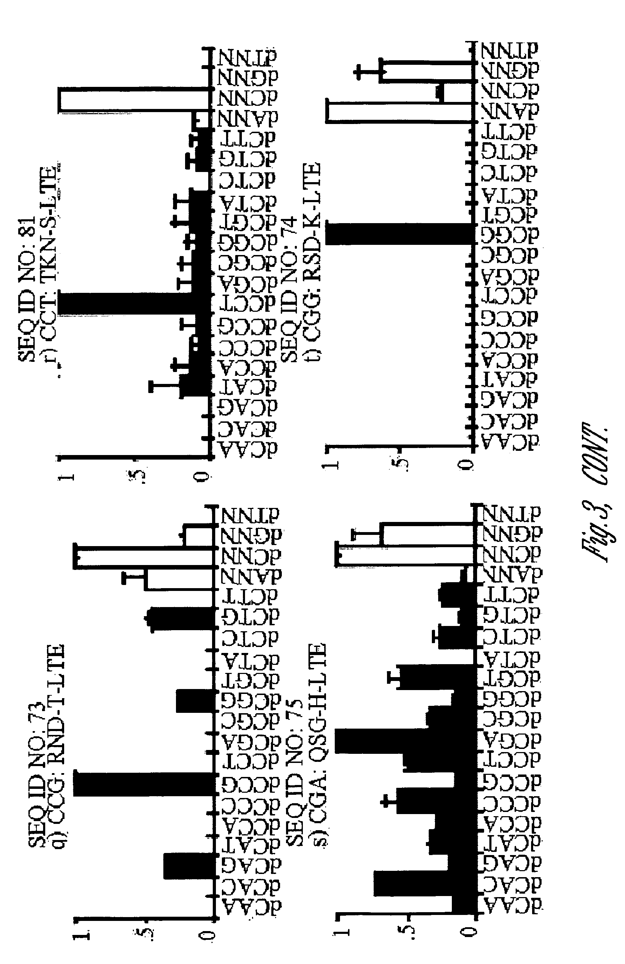

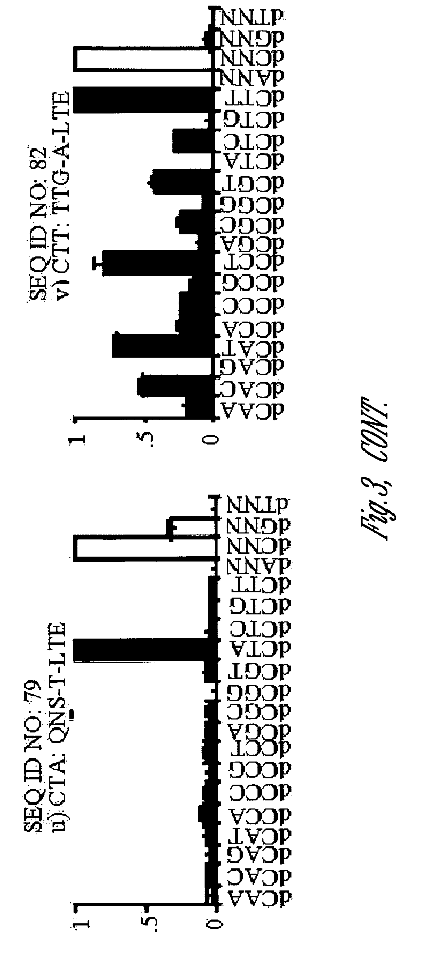

FIG. 3 is a graph showing multitarget specificity assay to study DNA-binding properties of selected domains and domains derived by site-directed mutagenesis as follows: (a) RAD-N-LAI (SEQ ID NO: 5); (b) SKK-H-LAE (SEQ ID NO: 63); (c) SVR-N-LRE (SEQ ID NO: 64); (d) RND-T-LQA (SEQ ID NO: 62); (e) QLA-H-LKE (SEQ ID NO: 11); (f) HTG-H-LLE (SEQ ID NO: 66); (g) RSD-H-LTE (SEQ ID NO: 14); (h) SRR-T-CRA (SEQ ID NO: 18(i) QLR-H-LRE (SEQ ID NO: 68); (j) QRH-S-LTE (SEQ ID NO: 70); (k) RND-A-LTE (SEQ ID NO: 71); (I) QSG-N-LTE (SEQ ID NO: 2); (m) SKK-A-LTE (SEQ ID NO: 77); (n) RAD-N-LTE (SEQ ID NO: 72); (o) TSG-N-LTE (SEQ ID NO: 78); (p) TSH-S-LTE (SEQ ID NO: 80); (q) RND-T-LTE (SEQ ID NO: 73); (r) TKN-S-LTE (SEQ ID NO: 81); (s) QSG-H-LTE (SEQ ID NO: 75); (t) RSD-K-LTE (SEQ ID NO: 74); (u) ONS-T-LTE (SEQ ID NO: 79); (v) TTG-A-LTE (SEQ ID NO: 82).

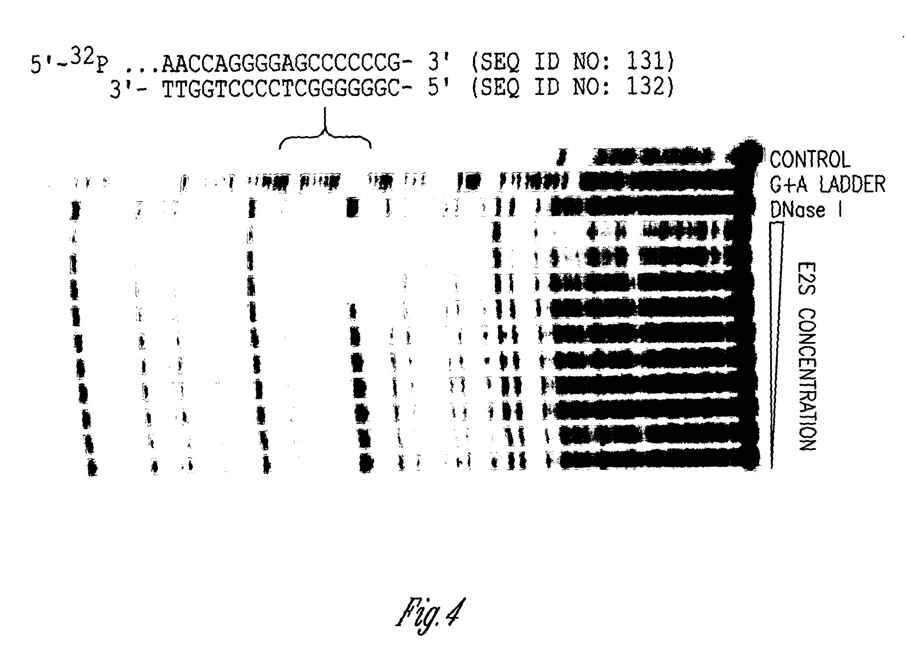

FIG. 4 is an autoradiograph showing the *DNase* I footprint of the six-finger protein pE2S containing domains recognizing 5'-CNN-3' DNA sequences.

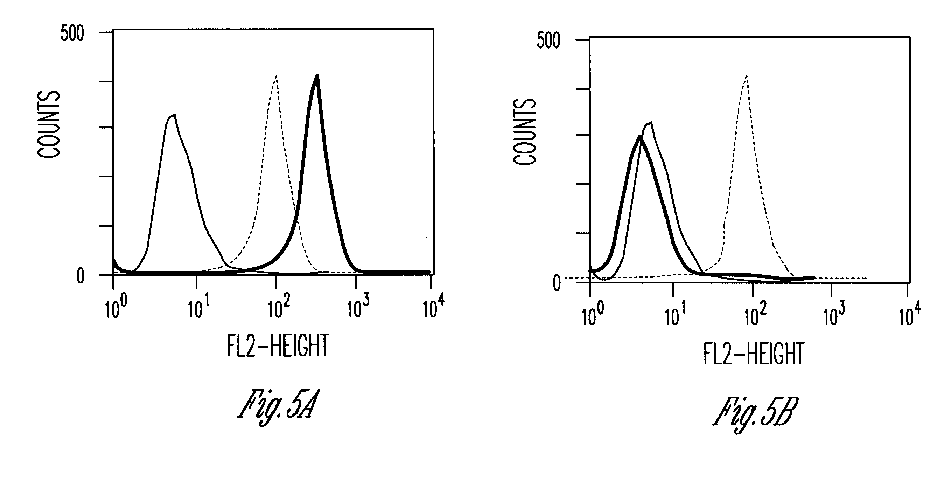

FIG. 5 is a set of graphs obtained by flow cytometry showing the results of retrovirus-mediated gene targeting to study the effect of an artificial transcription factor according to the present invention to regulate expression of an endogenous gene: Thin line, staining with secondary antibody alone; stippled line, specific staining of mock-transduced cells; solid lines, specific staining of cells transduced to express the transcription factors E2S-VP64 (A) or E2S-KRAB (B).

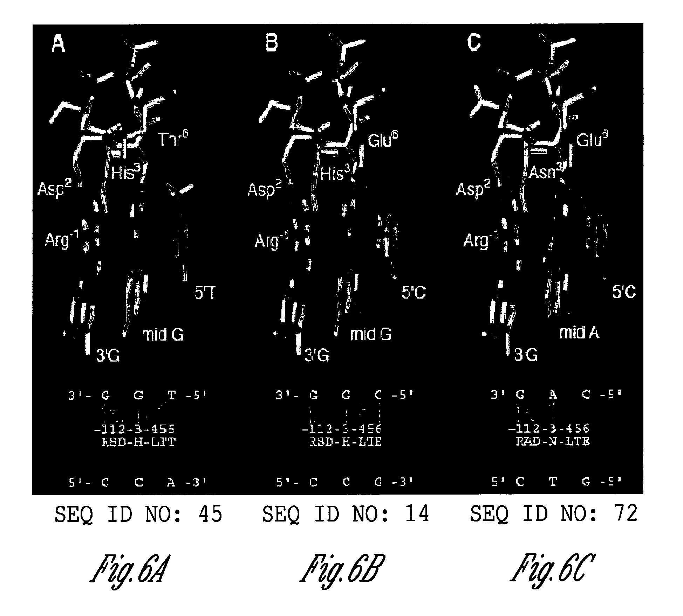

FIG. 6 is a graph showing computer models of finger-2 domains suggesting 5'-cytosine recognition. Select oxygen (red), nitrogen (blue), and phosphate (purple) atoms are colored for clarity. Green dotted lines indicate suggested hydrogen bonds. The sequence of each helix, the DNA subsite, and proposed interactions are summarized below each model. Green lines indicate hydrogen bonds. Arrows indicate hydrogen acceptors. A, finger-2 of Zif268; B, pCGG (Fig. 3g); C, pmCAG (Fig. 3n).

### DETAILED DESCRIPTION OF THE INVENTION

Definitions

Unless defined otherwise, all technical and scientific terms used herein have the same meaning as is commonly understood by one of skill in the art to which this invention belongs.

As used herein, the term "nucleic acid," "nucleic acid sequence," "polynucleotide," or similar terms, refers to a deoxyribonucleotide or ribonucleotide oligonucleotide or polynucleotide, including single- or double-stranded forms, and coding or non-coding (e.g., "antisense") forms. The term encompasses nucleic acids containing known analogues of natural nucleotides. The term also encompasses nucleic acids including modified or substituted bases as long as the modified or substituted bases interfere neither with the Watson-Crick binding of complementary nucleotides or with the binding of the nucleotide sequence by proteins that bind specifically, such as zinc finger proteins. The term also encompasses nucleic-acid-like structures with synthetic backbones. DNA backbone analogues provided by the invention include phosphodiester, phosphorothioate, phosphorodithioate, methylphosphonate, phosphoramidate, alkyl phosphotriester, sulfamate, 3'-thioacetal, methylene(methylimino), 3'-N-carbamate, morpholino carbamate, and peptide nucleic acids (PNAs); see Oligonucleotides and Analogues, a Practical Approach, edited by F. Eckstein, IRL Press at Oxford University Press (1991); Antisense Strategies, Annals of the New York Academy of Sciences, Volume 600, Eds. Baserga and Denhardt (NYAS 1992); Milligan (1993) J. Med. Chem. 36:1923-1937; Antisense Research and Applications (1993, CRC Press). PNAs contain nonionic backbones, such as N-(2-aminoethyl) glycine units. Phosphorothioate linkages are described, e.g., by U.S. Pat. Nos. 6,031,092; 6,001,982; 5,684,148; see also, WO 97/03211; WO 96/39154; Mata (1997) Toxicol. Appl. Pharmacol. 144:189-197. Other synthetic backbones encompassed by the term include methylphosphonate linkages or alternating methylphosphonate and phosphodiester linkages (see, e.g., U.S. Pat. No. 5,962,674; Strauss-Soukup (1997) Biochemistry 36:8692-8698), and benzylphosphonate linkages (see, e.g., U.S. Pat. No. 5,532,226; Samstag (1996) Antisense Nucleic Acid Drug Dev 6:153-156).

As used herein, the term "transcription regulating domain or factor" refers to the portion of the fusion polypeptide provided herein that functions to regulate gene transcription. Exemplary and preferred transcription repressor domains are ERD, KRAB, SID, Deacetylase, and derivatives, multimers and combinations thereof such as KRAB-ERD, SID-ERD, (KRAB)₂, (KRAB)₃, KRAB-A, (KRAB-A)₂, (SID)₂, (KRAB-A)-SID and SID-(KRAB-A). Other transcription regulating domains" are those disclosed in PCT Patent Application Publication No. WO 03/104414 by Barbas, entitled "Artificial Transcription Factors," incorporated herein by this reference. As used herein, the term "nucleotide binding domain or region" refers to the portion of a polypeptide or composition provided herein that provides specific nucleic acid binding capability. The nucleotide binding region functions to target a subject polypeptide to specific genes. As used herein, the term "operatively linked" means that elements of a polypeptide, for example, are linked such that each performs or functions as intended. For example, a repressor is attached to the binding domain in such a manner that, when bound to a target nucleotide via that binding domain, the repressor acts to inhibit or prevent transcription. Linkage between and among elements may be direct or indirect, such as via a linker. The elements are not necessarily adjacent. Hence a repressor domain can be linked to a nucleotide binding domain using any linking procedure well known in the art. It may be necessary to include a linker moiety between the two domains. Such a linker moiety is typically a short sequence of amino acid residues that provides spacing between the domains. So long as the linker does not interfere with any of the functions of the binding or repressor domains, any sequence can be used. Exemplary linkers are provided below.

As used herein, the term "modulating" envisions the inhibition or suppression of expression from a promoter containing a zinc finger-nucleotide binding motif when it is over-activated, or augmentation or enhancement of expression from such a promoter when it is underactivated.

As used herein, the amino acids, which occur in the various amino acid sequences appearing herein, are identified according to their well-known, three-letter or one-letter abbreviations. The nucleotides, which occur in the various DNA fragments, are designated with the standard single-letter designations used routinely in the art.

In a peptide or protein, suitable conservative substitutions of amino acids are known to those of skill in this art and may be made generally without altering the biological activity of the resulting molecule. Those of skill in this art recognize that, in general, single amino acid substitutions in non-essential regions of a polypeptide do not substantially alter biological activity (see, e.g. Watson et al. Molecular Biology of the Gene, 4th Edition, 1987, Benjamin/Cummings, p. 224). In particular, such a conservative variant has a modified amino acid sequence, such that the change(s) do not substantially alter the protein's (the conservative variant's) structure and/or activity, e.g., nucleic acid binding activity, antibody activity, enzymatic activity, or receptor activity. These include conservatively modified variations of an amino acid sequence, i.e., amino acid substitutions, additions or deletions of those residues that are not critical for protein activity, or substitution of amino acids with residues having similar properties (e.g., acidic, basic, positively or negatively charged, polar or non-polar, etc.) such that the substitutions of even critical amino acids does not substantially alter structure and/or activity. Conservative substitution tables providing functionally similar amino acids are well known in the art. For example, one exemplary guideline to select conservative substitutions includes (original residue followed by exemplary substitution): Ala/Gly or Ser; Arg/Lys; Asn/Gln or His; Asp/Glu; Cys/Ser, Gln/Asn; Gly/Asp; Gly/Ala or Pro; His/Asn or Gln; Ile/Leu or Val; Leu/Ile or Val; Lys/Arg or Gin or Glu; Met/Leu or Tyr or Ile; Phe/Met or Leu or Tyr, Ser/Thr; Thr/Ser; Trp/Tyr; Tyr/Trp or Phe; Val/Ile or Leu. An alternative exemplary guideline uses the following six groups, each containing amino acids that are conservative substitutions for one another: (1) alanine (A or Ala), serine (S or Ser), threonine (T or Thr); (2) aspartic acid (D or Asp), glutamic acid (E or Glu); (3) asparagine (N or Asn), glutamine (Q or Gin); (4) arginine (R or Arg), lysine (K or Lys); (5) isoleucine (I or Ile), leucine (L or Leu), methionine (M or Met), valine (V or Val); and (6) phenylalanine (F or Phe), tyrosine (Y or Tyr), tryptophan (W or Trp); (see also, e.g., Creighton (1984) Proteins, W. H. Freeman and Company; Schulz and Schimer (1979) Principles of Protein Structure, Springer-Verlag). One of skill in the art will appreciate that the above-identified substitutions are not the only possible conservative substitutions. For example, for some purposes, one may regard all charged amino acids as conservative substitutions for each other whether they are positive or negative. In addition, individual substitutions, deletions or additions that aster, add or delete a single amino acid or a small percentage of amino acids in an encoded sequence can also be considered "conservatively modified variations" when the three-dimensional structure and the function of the protein to be delivered are conserved by such a variation.

As used herein, the term "expression vector" refers to a plasmid, virus, phagemid, or other vehicle known in the art that has been manipulated by insertion or incorporation of heterologous DNA, such as nucleic acid encoding the fusion proteins herein or expression cassettes provided herein. Such expression vectors typically contain a promoter sequence for efficient transcription of the inserted nucleic acid in a cell. The expression vector typically contains an origin of replication, a promoter, as well as specific genes that permit phenotypic selection of transformed cells.

As used herein, the term "host cells" refers to cells in which a vector can be propagated and its DNA expressed. The term also includes any progeny of the subject host cell. It is understood that all progeny may not be identical to the parental cell since there may be mutations that occur during replication. Such progeny are included when the term "host cell" is used. Methods of stable transfer where the foreign DNA is continuously maintained in the host are known in the art.

As used herein, genetic therapy involves the transfer of heterologous DNA to the certain cells, target cells, of a mammal, particularly a human, with a disorder or conditions for which such therapy is sought. The DNA is introduced into the selected target cells in a manner such that the heterologous DNA is expressed and a therapeutic product encoded thereby is produced. Alternatively, the heterologous DNA may in some manner mediate expression of DNA that encodes the therapeutic product, or it may encode a product, such as a peptide or RNA that in some manner mediates, directly or indirectly, expression of a therapeutic product. Genetic therapy may also be used to deliver nucleic acid encoding a gene product that replaces a defective gene or supplements a gene product produced by the mammal or the cell in which it is introduced. The introduced nucleic acid may encode a therapeutic compound, such as a growth factor inhibitor thereof, or a tumor necrosis factor or inhibitor thereof, such as a receptor therefor, that is not normally produced in the mammalian host or that is not produced in therapeutically effective amounts or at a therapeutically useful time. The heterologous DNA encoding the therapeutic product may be modified prior to introduction into the cells of the afflicted host in order to enhance or otherwise alter the product or expression thereof. Genetic therapy may also involve delivery of an inhibitor or repressor or other modulator of gene expression.

As used herein, heterologous DNA is DNA that encodes RNA and proteins that are not normally produced in vivo by the cell in which it is expressed or that mediates or encodes mediators that alter expression of endogenous DNA by affecting transcription, translation, or other regulatable biochemical processes. Heterologous DNA may also be referred to as foreign DNA. Any DNA that one of skill in the art would recognize or consider as heterologous or foreign to the cell in which is expressed is herein encompassed by heterologous DNA. Examples of heterologous DNA include, but are not limited to, DNA that encodes traceable marker proteins, such as a protein that confers drug resistance, DNA that encodes therapeutically effective substances, such as anti-cancer agents, enzymes and hormones, and DNA that encodes other types of proteins, such as antibodies. Antibodies that are encoded by heterologous DNA may be secreted or expressed on the surface of the cell in which the heterologous DNA has been introduced.

Hence, herein heterologous DNA or foreign DNA, includes a DNA molecule not present in the exact orientation and position as the counterpart DNA molecule found in the genome. It may also refer to a DNA molecule from another organism or species (i.e., exogenous).

As used herein, a therapeutically effective product is a product that is encoded by heterologous nucleic acid, typically DNA, that, upon introduction of the nucleic acid into a host, a product is expressed that ameliorates or eliminates the symptoms, manifestations of an inherited or acquired disease or that cures the disease. Typically, DNA encoding a desired gene product is cloned into a plasmid vector and introduced by routine methods, such as calcium-phosphate mediated DNA uptake (see, (1981) Somat. Cell. Mol. Genet. 7:603-616) or microinjection, into producer cells, such as packaging cells. After amplification in producer cells, the vectors that contain the heterologous DNA are introduced into selected target cells.

As used herein, an expression or delivery vector refers to any plasmid or virus into which a foreign or heterologous DNA may be inserted for expression in a suitable host cell-i.e., the protein or polypeptide encoded by the DNA is synthesized in the host cell's system. Vectors capable of directing the expression of DNA segments (genes) encoding one or more proteins are referred to herein as "expression vectors". Also included are vectors that allow cloning of cDNA (complementary DNA) from mRNAs produced using reverse transcriptase.

As used herein, a gene refers to a nucleic acid molecule whose nucleotide sequence encodes an RNA or polypeptide. A gene can be either RNA or DNA. Genes may include regions preceding and following the coding region (leader and trailer) as well as intervening sequences (introns) between individual coding segments (exons).

As used herein, the term "isolated" with reference to a nucleic acid molecule or polypeptide or other biomolecule means that the nucleic acid or polypeptide has been separated from the genetic environment from which the polypeptide or nucleic acid were obtained. It may also mean that the biomolecule has ben altered from the natural state. For example, a polynucleotide or a polypeptide naturally present in a living animal is not "isolated," but the same polynucleotide or polypeptide separated from the coexisting materials of its natural state is "isolated," as the term is employed herein. Thus, a polypeptide or polynucleotide produced and/or contained within a recombinant host cell is considered isolated. Also intended as an "isolated polypeptide" or an "isolated polynucleotide" are polypeptides or polynucleotides that have been purified, partially or substantially, from a recombinant host cell or from a native source. For example, a recombinantly produced version of a compound can be substantially purified by the one-step method described in Smith et al. (1988) Gene 67:3140. The terms isolated and purified are sometimes used interchangeably.

Thus, by "isolated" is meant that the nucleic acid is free of the coding sequences of those genes that, in a naturally-occurring genome immediately flank the gene encoding the nucleic acid of interest. Isolated DNA may be single-stranded or double-stranded, and may be genomic DNA, cDNA, recombinant hybrid DNA, or synthetic DNA. It may be identical to a native DNA sequence, or may differ from such sequence by the deletion, addition, or substitution of one or more nucleotides.

"Isolated" or "purified" as those terms are used to refer to preparations made from biological cells or hosts means any cell extract containing the indicated DNA or protein including a crude extract of the DNA or protein of interest. For example, in the case of a protein, a purified preparation can be obtained following an individual technique or a series of preparative or biochemical techniques and the DNA or protein of interest can be present at various degrees of purity in these preparations. Particularly for proteins, the procedures may include for example, but are not limited to, ammonium sulfate fractionation, gel filtration, ion exchange change chromatography, affinity chromatography, density gradient centrifugation, electrofocusing, chromatofocusing, and electrophoresis.

A preparation of DNA or protein that is "substantially pure" or "isolated" should be understood to mean a preparation free from naturally occurring materials with which such DNA or protein is normally associated in nature. "Essentially pure" should be understood to mean a "highly" purified preparation that contains at least 95% of the DNA or protein of interest.

A cell extract that contains the DNA or protein of interest should be understood to mean a homogenate preparation or cell-free preparation obtained from cells that express the protein or contain the DNA of interest. The term "cell extract" is intended to include culture media, especially spent culture media from which the cells have been removed.

As used herein, "modulate" refers to the suppression, enhancement or induction of a function. For example, zinc finger-nucleic acid binding domains and variants thereof may modulate a promoter sequence by binding to a motif within the promoter, thereby enhancing or suppressing transcription of a gene operatively linked to the promoter cellular nucleotide sequence. Alternatively, modulation may include inhibition of transcription of a gene where the zinc finger-nucleotide binding polypeptide variant binds to the structural gene and blocks DNA dependent RNA polymerase from reading through the gene, thus inhibiting transcription of the gene. The structural gene may be a normal cellular gene or an oncogene, for example. Alternatively, modulation may include inhibition of translation of a transcript.

As used herein, the term "inhibit" refers to the suppression of the level of activation of transcription of a structural gene operably linked to a promoter. For example, for the methods herein the gene includes a zinc finger-nucleotide binding motif.

As used herein, the term "transcriptional regulatory region" refers to a region that drives gene expression in the target cell. Transcriptional regulatory regions suitable for use herein include but are not limited to the human cytomegalovirus (CMV) immediate-early enhancer/promoter, the SV40 early enhancer/promoter, the JC polyoma virus promoter, the albumin promoter, PGK and the α-actin promoter coupled to the CMV enhancer. Other transcriptional regulatory regions are also known in the art.

As used herein, a promoter region of a gene includes the regulatory element or elements that typically lie 5' to a structural gene; multiple regulatory elements can be present, separated by intervening nucleotide sequences. If a gene is to be activated, proteins known as transcription factors attach to the promoter region of the gene. This assembly resembles an "on switch" by enabling an enzyme to transcribe a second genetic segment from DNA into RNA. In most cases the resulting RNA molecule serves as a template for synthesis of a specific protein; sometimes RNA itself is the final product. The promoter region may be a normal cellular promoter or, for example, an onco-promoter. An onco-promoter is generally a virus-derived promoter. Viral promoters to which zinc finger binding polypeptides may be targeted include, but are not limited to, retroviral long terminal repeats (LTRs), and Lentivirus promoters, such as promoters from human T-cell lymphotrophic virus (HTLV) 1 and 2 and human immunodeficiency virus (HIV) 1 or 2.

As used herein, the term "effective amount" includes that amount that results in the deactivation of a previously activated promoter or that amount that results in the inactivation of a promoter containing a zinc finger-nucleotide binding motif, or that amount that blocks transcription of a structural gene or translation of RNA. The amount of zinc finger derived-nucleotide binding polypeptide required is that amount necessary to either displace a native zinc finger-nucleotide binding protein in an existing protein/promoter complex, or that amount necessary to compete with the native zinc finger-nucleotide binding protein to form a complex with the promoter itself. Similarly, the amount required to block a structural gene or RNA is that amount which binds to and blocks RNA polymerase from reading through on the gene or that amount which inhibits translation, respectively. Preferably, the method is performed intracellularly. By functionally inactivating a promoter or structural gene, transcription or translation is suppressed. Delivery of an effective amount of the inhibitory protein for binding to or "contacting" the cellular nucleotide sequence containing the zinc finger-nucleotide binding protein motif, can be accomplished by one of the mechanisms described herein, such as by retroviral vectors or liposomes, or other methods well known in the art.

As used herein, the term "truncated" refers to a zinc finger-nucleotide binding polypeptide derivative that contains less than the full number of zinc fingers found in the native zinc finger binding protein or that has been deleted of non-desired sequences. For example, truncation of the zinc finger-nucleotide binding protein TFIIIA, which naturally contains nine zinc fingers, might result in a polypeptide with only zinc fingers one through three. The term "expansion" refers to a zinc finger polypeptide to which additional zinc finger modules have been added. For example, TFIIIA can be expanded to 12 fingers by adding 3 zinc finger domains. In addition, a truncated zinc finger-nucleotide binding polypeptide may include zinc finger modules from more than one wild type polypeptide, thus resulting in a "hybrid" zinc finger-nucleotide binding polypeptide.

As used herein, the term "mutagenized" refers to a zinc finger derived-nucleotide binding polypeptide that has been obtained by performing any of the known methods for accomplishing random or site-directed mutagenesis of the DNA encoding the protein. For instance, in TFIIIA, mutagenesis can be performed to replace nonconserved residues in one or more of the repeats of the consensus sequence. Truncated or expanded zinc finger-nucleotide binding proteins can also be mutagenized.

As used herein, a polypeptide "variant" or "derivative" refers to a polypeptide that is a mutagenized form of a polypeptide or one produced through recombination but that still retains a desired activity, such as the ability to bind to a ligand or a nucleic acid molecule or to modulate transcription.

As used herein, a zinc finger-nucleotide binding polypeptide "variant" or "derivative" refers to a polypeptide that is a mutagenized form of a zinc finger protein or one produced through recombination. A variant may be a hybrid that contains zinc finger domain(s) from one protein linked to zinc finger domain(s) of a second protein, for example. The domains may be wild type or mutagenized. A "variant" or "derivative" can include a truncated form of a wild type zinc finger protein, which contains fewer than the original number of fingers in the wild type protein. Examples of zinc finger-nucleotide binding polypeptides from which a derivative or variant may be produced include SP1C, TFIIIA, and Zif268. Similar terms are used to refer to "variant" or "derivative" nuclear hormone receptors and "variant" or "derivative" transcription effector domains.

As used herein a "zinc finger-nucleotide binding target or motif" refers to any two or three-dimensional feature of a nucleotide segment to which a zinc finger-nucleotide binding derivative polypeptide binds with specificity. Included within this definition are nucleotide sequences, generally of five nucleotides or less, as well as the three dimensional aspects of the DNA double helix, such as, but are not limited to, the major and minor grooves and the face of the helix. The motif is typically any sequence of suitable length to which the zinc finger polypeptide can bind. For example, a three finger polypeptide binds to a motif typically having about 9 to about 14 base pairs. Preferably, the recognition sequence is at least about 16 base pairs to ensure specificity within the genome. Therefore, zinc finger-nucleotide binding polypeptides of any specificity are provided. The zinc finger binding motif can be any sequence designed empirically or to which the zinc finger protein binds. The motif may be found in any DNA or RNA sequence, including regulatory sequences, exons, introns, or any non-coding sequence.

As used herein, the terms "pharmaceutically acceptable", "physiologically tolerable" and grammatical variations thereof, as they refer to compositions, carriers, diluents and reagents, are used interchangeably and represent that the materials are capable of administration to or upon a human without the production of undesirable physiological effects such as nausea, dizziness, gastric upset and the like which would be to a degree that would prohibit administration of the composition.

As used herein, the term "vector" refers to a nucleic acid molecule capable of transporting between different genetic environments another nucleic acid to which it has been operatively linked. Preferred vectors are those capable of autonomous replication and expression of structural gene products present in the DNA segments to which they are operatively linked. Vectors, therefore, preferably contain the replicons and selectable markers described earlier. Vectors include, but are not necessarily limited to, expression vectors.

As used herein with regard to nucleic acid molecules, including DNA fragments, the phrase "operatively linked" means the sequences or segments have been covalently joined, preferably by conventional phosphodiester bonds, into one strand of DNA, whether in single or double-stranded form such that operatively linked portions function as intended. The choice of vector to which transcription unit or a cassette provided herein is operatively linked depends directly, as is well known in the art, on the functional properties desired, e.g., vector replication and protein expression, and the host cell to be transformed, these being limitations inherent in the art of constructing recombinant DNA molecules.

As used herein, administration of a therapeutic composition can be effected by any means, and includes, but is not limited to, oral, subcutaneous, intravenous, intramuscular, intrasternal, infusion techniques, intraperitoneal administration and parenteral administration.

I. The Invention

The present invention provides zinc finger-nucleotide binding polypeptides, compositions containing one or more such polypeptides, polynucleotides that encode such polypeptides and compositions, expression vectors containing such polynucleotides, cells transformed with such polynucleotides or expression vectors and the use of the polypeptides, compositions, polynucleotides and expression vectors for modulating nucleotide structure and/or function.

II. Polypeptides

The present invention provides an isolated and purified zinc finger nucleotide binding polypeptide. The polypeptide contains a nucleotide binding region of from 5 to 10 amino acid residues and, preferably about 7 amino acid residues. The nucleotide binding region binds preferentially to a target nucleotide of the formula CNN, where N is A, C, G or T. Preferably, the target nucleotide has the formula CAA, CAC, CAG, CAT, CCA, CCC, CCG, CCT, CGA, CGC, CGG, CGT, CTA, CTC, CTG or CTT.

A polypeptide of this invention is a non-naturally occurring variant. As used herein, the term "non-naturally occurring" means, for example, one or more of the following: (a) a polypeptide comprised of a non-naturally occurring amino acid sequence; (b) a polypeptide having a non-naturally occurring secondary structure not associated with the polypeptide as it occurs in nature; (c) a polypeptide which includes one or more amino acids not normally associated with the species of organism in which that polypeptide occurs in nature; (d) a polypeptide which includes a stereoisomer of one or more of the amino acids comprising the polypeptide, which stereoisomer is not associated with the polypeptide as it occurs in nature; (e) a polypeptide which includes one or more chemical moieties other than one of the natural amino acids; or (f) an isolated portion of a naturally occurring amino acid sequence (e.g., a truncated sequence). A polypeptide of this invention exists in an isolated form and purified to be substantially free of contaminating substances. The polypeptide can be isolated and purified from natural sources; alternatively, the polypeptide can be made de novo using techniques well known, in the art such as genetic engineering or solid-phase peptide synthesis. A zinc finger-nucleotide binding polypeptide refers to a polypeptide that is, preferably, a mutagenized form of a zinc finger protein or one produced through recombination. A polypeptide may be a hybrid which contains zinc finger domain(s) from one protein linked to zinc finger domain(s) of a second protein, for example. The domains may be wild type or mutagenized. A polypeptide can include a truncated form of a wild type zinc finger protein. Examples of zinc finger proteins from which a polypeptide can be produced include SP1C, TFIIIA and Zif268, as well as C7 (a derivative of Zif268) and other zinc finger proteins known in the art. These zinc finger proteins from which other zinc finger proteins are derived are referred to herein as "backbones."

A zinc finger-nucleotide binding polypeptide of this invention comprises a unique heptamer (contiguous sequence of 7 amino acid residues) within the α-helicat domain of the polypeptide, which heptameric sequence determines binding specificity to a target nucleotide. That heptameric sequence can be located anywhere within the α-helical domain but it is preferred that the heptamer extend from position -1 to position 6 as the residues are conventionally numbered in the art. A polypeptide of this invention can include any β-sheet and framework sequences known in the art to function as part of a zinc finger protein. A large number of zinc finger-nucleotide binding polypeptides were made and tested for binding specificity against target nucleotides containing a CNN triplet.

The zinc finger-nucleotide binding polypeptide derivative can be derived or produced from a wild type zinc finger protein by truncation or expansion, or as a variant of the wild type-derived polypeptide by a process of site directed mutagenesis, or by a combination of the procedures. In addition, a truncated zinc finger-nucleotide binding polypeptide may include zinc finger modules from more than one wild type polypeptide, thus resulting in a "hybrid" zinc finger-nucleotide binding polypeptide.

The term "mutagenized" refers to a zinc finger derived-nucleotide binding polypeptide that has been obtained by performing any of the known methods for accomplishing random or site-directed mutagenesis of the DNA encoding the protein. For instance, in TFIIIA, mutagenesis can be performed to replace nonconserved residues in one or more of the repeats of the consensus sequence. Truncated zinc finger-nucleotide binding proteins can also be mutagenized. Examples of known zinc finger-nucleotide binding polypeptides that can be truncated, expanded, and/or mutagenized according to the present invention in order to inhibit the function of a nucleotide sequence containing a zinc finger-nucleotide binding motif include SP1C, TFIIIA, and Zif268. Those of skill in the art know other zinc finger-nucleotide binding proteins.

In one embodiment, a polypeptide of the invention contains a binding region that has an amino acid sequence with the same nucleotide binding characteristics as any of SEQ ID NOs: 1-28, 35-45, 48, 54, 57-82, 85-130, and 134-157. A detailed description of how those binding characteristics were determined can be found hereinafter in the Examples. Such a polypeptide competes for binding to a nucleotide target with any of SEQ ID NOs: 1-28, 35-45, 48, 54, 57-82, 85-130, and 134-157. That is, a preferred polypeptide contains a binding region that will displace, in a competitive manner, the binding of any of SEQ IDS NOs: 1-28, 35-45, 48, 54, 57-82, 85-130, and 134-157. Means for determining competitive binding are well known in the art. Preferably, the binding region has the amino acid sequence of any of SEQ ID NOs: 1-28, 35-45, 48, 54, 57-82, 85-130, and 134-157. More preferably, the binding region has the amino acid sequence of any of SEQ ID NOs: 54, 57-82, 85-130, and 134-157. Still more preferably, the binding region has the amino acid sequence of any of SEQ ID NOs: 2, 14, 18, 63, 64, 65, 66, 67, 68, 69, 70, 71, 72, 73, 75, 77, 78, 79, 80, 81, 82, 134, and 153. Even more preferably, the binding region has the amino acid sequence of any of SEQ ID NOs: 2, 14, 18, 63, 66, 71, 72, 73, 75, 77, 78, 79, 80, 81, 82, 134, and 153. Still even more preferably, the binding region has the amino acid sequence of any of SEQ ID NOs: 2, 14, 18, 63, 66, 71, 72, 73, 75, 77, 78, 79, 80, 81, and 82.

Typically, the binding region has seven amino acid residues and has α-helical structure.

Also within the scope of the present invention are polypeptides that differ from the polypeptides disclosed above by no more than two conservative amino acid substitutions or no more than 2 conservative amino acid substitutions) that have a binding affinity for the desired subsite or target region of at least 80% as great as the polypeptide before the substitutions are made. In terms of dissociation constants, this is equivalent to a dissociation constant no greater than 125% of that of the polypeptide before the substitutions are made. In this context, the term "conservative amino acid substitution" is defined as one of the following substitutions: Ala/Gly or Ser; Arg/Lys; Asn/Gln or His; Asp/Glu; Cys/Ser; Gin/Asn; Gly/Asp; Gly/Ala or Pro; His/Asn or Gln; Ile/Leu or Val; Leu/Ile or Val; Lys/Arg or Gln or Glu; Met/Leu or Tyr or Ile; Phe/Met or Leu or Tyr; Ser/Thr; Thr/Ser; Trp/Tyr; Tyr/Trp or Phe; Val/Ile or Leu. Preferably, the polypeptide differs from the polypeptides described above by no more than one conservative amino acid substitution.

In addition, the polypeptides of the present invention can be incorporated within longer polypeptides. Some examples of this are described below, when the polypeptides are used to create artificial transcription factors. In general, though the polypeptides can be incorporated into longer fusion proteins and retain their specific DNA binding activity. These fusion proteins can include various additional domains as is known in the art, such as purification tags, enzyme domains, or other domains, without significantly altering the specific DNA-binding activity of the zinc finger polypeptides. In one example, the polypeptides can be incorporated into two halves of a split enzyme like a β-lactamase to allow the sequences to be sensed in cells or *in vivo*. Binding of two halves of such a split enzyme then allows for assembly of the split enzyme (J.M. Spotts et al. "Time-Lapse Imaging of a Dynamic Phosphorylation Protein-Protein Interaction in Mammalian Cells," Proc. Natl. Acad. Sci. USA 99: 15142-15147 (2002)). In another example, multiple zinc finger domains according to the present invention can be tandemly linked to form polypeptides that have specific binding affinity for longer DNA sequences. This is described further below.

A polypeptide of this invention can be made using a variety of standard techniques well known in the art. As disclosed in detail hereinafter in the Examples, phage display libraries of zinc finger proteins were created and selected under conditions that favored enrichment of sequence specific proteins. Zinc finger domains recognizing a number of sequences required refinement by site-directed mutagenesis that was guided by both phage selection data and structural information.

Accordingly, a zinc finger nucleotide binding polypeptide according to the present invention can be a derivative or variant of a naturally-occurring zinc finger protein. The naturally-occurring zinc finger protein can be, for example, and not by way of limitation, SP1C, TFIIIA or Zif268. Other naturally-occurring zinc finger proteins, typically of the Cys₂-His₂ class, can be used to form zinc finger nucleotide binding polypeptides according to the present invention. This naturally-occurring zinc finger protein is referred to herein as a "backbone." The recitation of a naturally-occurring zinc finger protein as a backbone does not preclude the mutagenesis of other residues within the naturally-occurring zinc finger protein. This can be done to increase binding affinity, binding specificity, protein stability, or for other reasons.

Previously we reported the characterization of 16 zinc finger domains specifically recognizing each of the 5'-(GNN)-3' type of DNA sequences, that were isolated by phage display selections based on C7, a variant of the mouse transcription factor Zif268 and refined by site-directed mutagenesis [Segal et al., (1999) Proc Natl Acad Sci USA 96(6), 2758-2763; Dreier et al., (2000) J. Mol. Biol. 303, 489-502; and U.S. Pat. No. 6,140,081, the disclosure of which is incorporated herein by reference]. In general, the specific DNA recognition of zinc finger domains of the Cys₂-His₂ type is mediated by the amino acid residues -1, 3, and 6 of each α-helix, although not in every case are all three residues contacting a DNA base. One dominant cross-subsite interaction has been observed from position 2 of the recognition helix. Asp² has been shown to stabilize the binding of zinc finger domains by directly contacting the complementary adenine or cytosine of the 5' thymine or guanine, respectively, of the following 3 bp subsite. These non-modular interactions have been described as target site overlap. In addition, other interactions of amino acids with nucleotides outside the 3 bp subsites creating extended binding sites have been reported [Pavletich et al., (1991) Science 252(5007), 809-817; Elrod-Erickson et al., (1996) Structure 4(10), 1171-1180; Isalan et al., (1997) Proc Natl Acad Sci USA 94(11), 5617-5621].

Selection of the previously reported phage display library for zinc finger domains binding to 5' nucleotides other than guanine or thymine met with no success, due to the cross-subsite interaction from aspartate in position 2 of the finger-3 recognition helix RSD-E-LKR (SEQ ID NO:26) (FIG. 1). To extend the availability of zinc finger domains for the construction of artificial transcription factors, domains specifically recognizing the 5'-(ANN)-3' type of DNA sequences were selected (U.S. Patent Application Ser. No. 09/791,106, filed Feb. 21,2001, the disclosure of which is incorporated herein by reference). Other groups have described a sequential selection method which led to the characterization of domains recognizing four 5'-(ANN)-3' subsites, 5'-(AAA)-3', 5'-(AAG)-3', 5'-(ACA)-3', and 5'-(ATA)-3' [Greisman et al., (1997) Science 275(5300), 657-661; Wolfe et al., (1999) J Mol Biol 285(5), 1917-1934]. The present disclosure uses an approach to select zinc finger domains recognizing CNN sites by eliminating the target site overlap. First, finger 3 of C7 (RSD-E-RKR) (SEQ ID NO: 27) binding to the subsite 5'-(GCG)-3' was exchanged with a domain which did not contain aspartate in position 2 (FIG. 1). The helix TSG-N-LVR (SEQ ID NO: 28), previously characterized in finger 2 position to bind with high specificity to the triplet 5'-(GAT)-3', seemed a good candidate. This 3-finger protein (C7.GAT; FIG. 1A, lower panel), containing finger 1 and 2 of C7 and the 5'-(GAT)-3' recognition helix in finger-3 position, was analyzed for DNA-binding specificity on targets with different finger-2 subsites by multi-target ELISA in comparison with the original C7 protein (C7.GCG; FIG. 1B). Both proteins bound to the 5'-(TGG)-3' subsite (note that C7.GCG binds also to 5'-(GGG)-3' due to the 5' specification of thymine or guanine by Asp² of finger 3 which has been reported earlier. The recognition of the 5' nucleotide of the finger-2 subsite was evaluated using a mixture of all 16 5'-(XNN)-3' target sites (X represents adenine, guanine, cytosine or thymine). Indeed, while the original C7.GCG protein specified a guanine or thymine in the 5' position of finger 2, C7.GAT did not specify a base, indicating that the cross-subsite interaction to the adenine complementary to the 5' thymine was abolished. A similar effect has previously been reported for variants of Zif268 where Asp² was replaced by Ala² by site-directed mutagenesis [Isalan et al., (1997) Proc Natl Acad Sci USA 94(11), 5617-5621; Dreier et al., (2000) J. Mol. Biol. 303, 489-502]. The affinity of C7.GAT, measured by gel mobility shift analysis, was found to be relatively low, about 400 nM compared to 0.5 nM for C7.GCG [Segal et al., (1999) Proc Natl Acad Sci USA 96(6), 2758-2763], which may in part be due to the lack of the Asp² in finger 3.

Based on the 3-finger protein C7.GAT, a library was constructed in the phage display vector pComb3H [Barbas et al., (1991) Proc. Natl. Acad. Sci. USA 88, 7978-7982; Rader et al., (1997) Curr. Opin. Biotechnol. 8(4), 503-508]. Randomization involved positions -1, 1, 2, 3, 5, and 6 of the α-helix of finger 2 using a VNS codon doping strategy (V=adenine, cytosine or guanine, N=adenine, cytosine, guanine or thymine, S=cytosine or guanine). This allowed 24 possibilities for each randomized amino acid position, whereas the aromatic amino acids Trp, Phe, and Tyr, as well as stop codons, were excluded in this strategy. Because Leu is predominately found in position 4 of the recognition helices of zinc finger domains of the type Cys₂-His₂ this position was not randomized. After transformation of the library into ER2537 cells (New England Biolabs) the library contained 1.5 × 10⁹ members. This exceeded the necessary library size by 60-fold and was sufficient to contain all amino acid combinations.

Six rounds of selection of zinc finger-displaying phage were performed binding to each of the sixteen 5'-GAT-CNN-GCG-3' (SEQ ID NO: 29) biotinylated hairpin target oligonucleotides, respectively, in the presence of non-biotinylated competitor DNA. Stringency of the selection was increased in each round by decreasing the amount of biotinylated target oligonucleotide and increasing amounts of the competitor oligonucleotide mixtures. In the sixth round the target concentration was usually 18 nM, 5'-(ANN)-3', 5'-(GNN)-3', and 5'-(TNN)-3' competitor mixtures were in 5-fold excess for each oligonucleotide pool, respectively, and the specific 5'-(CNN)-3' mixture (excluding the target sequence) in 10-fold excess. Phage binding to the biotinylated target oligonucleotide was recovered by capture to streptavidin-coated magnetic beads. Clones were usually analyzed after the sixth round of selection.

These results provide a number of guidelines for the determination of sequences within the present invention to one of ordinary skill in the art. These guidelines are: (1) It is preferred that Gln be at position -1 when the 5'-nucleotide is adenine. (2) It is preferred that Gln, Asn, or Ser be at position -1 when the subsite is 5'-CAA-3'. (3) It is preferred that Ser be at position -1 when the subsite is 5'-CCA-3'. (4) It is generally preferred to have Arg, Asn, Gln, His Ser, Thr or Ile at position -1 when the target subsite has a 3'-guanine, with Arg particularly preferred. (5) It is generally preferred to have Asp at position 2 for binding to to 5'-CNG-3' subsites. (6) For binding to the subsite 5'-CNT-3', Arg, Asn, Gln, His, Ser, Thr, Ala, and Cys are generally preferred at position -1. (7) For subsites containing a 3'-cytosine, Gln, Asn, Ser, Gly, His, or Asp are typically preferred in position -1. (8) For the recognition of 5'-CAN-3', His, Asn, Gly, Val, Pro, Ile, and Lys are typically preferred in position 3; Asn is strongly preferred. (9) Thr or Asp are particularly preferred in position 3 of the helix that recognized 5'-GCN-3' subsites, but His, Lys, Arg, and Asn can also be accommodated. (10) For the target site 5'-CCC-3', position 3 is preferably Asn or His. (11) For the target site 5'-CCG-3', position 3 is preferably either Thr or His. (12) For the target site 5'-GGN-3', His is preferred at position 3. (13) For target sites 5'-CG(G/T)-3', Ser, Asp, Thr, Asn, Gln and Gly are preferred at position 3; His is also possible. (14) For the target site 5'-CGC-3' Trp and Thr are typically preferred at position 3; His is also possible. (15) In general, a middle guanine in 5'-CGN-3' can be recognized by His. (16) For the target sites 5'-CTN-3', position 3 is preferably either Ser or Ala, except for 5'-CTC-3' and 5'-CTT-3' where His is preferred. (17) At position 6, Glu, Asp, Asn, Ile, Ala, Ser, or Val is preferred; Glu is particularly preferred; the neighboring amino acids can influence this interaction. (18) Positions 1,2, and 5 can vary widely. (19) For recognition of a 3' adenine, Gln is typically preferred at position -1. (20) For recognition of a 3' guanine, Arg is typically preferred at position -1. (21) For recognition of a 3' thymine, Ser, Thr, or His is typically preferred at position -1. (22) For recognition of a 5' cytosine, the motif Leu-Thr-Glu in positions 4,5, and 6 is typically preferred. These are only guidelines, and the secondary or tertiary structure of a protein or polypeptide incorporating a zinc finger moiety according to the present invention can lead to different amino acids being preferred for recognition of particular subsites or particular nucleotides at a defined position of such subsites. Additionally, the conformation of a particular zinc finger moiety within a protein having a plurality of zinc finger moieties can affect the binding.

Additionally, proteins or polypeptides incorporating zinc fingers can be molecularly modeled, as detailed below in Example 13. One suitable computer program for molecular modeling is Insight II. Molecular modeling can be used to generate other zinc finger moieties based on variations of zinc finger moieties described herein and that are within the scope of the invention. When modeling establishes that such variations have a hydrogen-bonding pattern that is substantially similar to that of a zinc finger moiety within the scope of the invention and that has been used as the basis for modeling, such variations are also within the scope of the invention. As used herein, the term "substantially similar" with respect to hydrogen bonding pattern means that the same number of hydrogen bonds are present, that the bond angle of each hydrogen bond varies by no more than about 10 degrees, and that the bond length of each hydrogen bond varies by no more than about 0.2 A.

III. Polypeptide Compositions

In another aspect, the present invention provides a polypeptide composition that comprises a plurality of zinc finger-nucleotide binding polypeptides according to the present invention as described above operatively linked in such a manner to specifically bind a nucleotide target motif defined as 5'-(CNN)ₙ-3', where n is an integer greater than 1. The target motif can be located within any longer nucleotide sequence (e.g., from 3 to 13 or more TNN, GNN, ANN or NNN sequences). Preferably, n is an integer from 2 to about 12, and more preferably from 2 to 6. The individual polypeptides are preferably linked with oligopeptide linkers. Such linkers preferably resemble a linker found in naturally occurring zinc finger proteins. A preferred linker for use in the present invention is the amino acid residue sequence TGEKP (SEQ ID NO: 30). Another preferred linker for use in the present invention is the amino acid residue sequence TGGGGSGGGGTGEKP (SEQ ID NO: 133). This longer linker can be used when it is desired to have the two halves of a longer plurality of zinc finger binding polypeptides operate in a substantially independent manner. Other linkers such as glycine or serine repeats are well known in the art to link peptides (e.g., single chain antibody domains) and can be used in a composition of this invention. Still other linkers are known in the art and can be used, such as an AAARA linker (SEQ ID NO: 158), as described in S. Alwin et al., "Custom Zinc-Finger Nucleases for Use in Human Cells," Mol. Ther. 12: 610-617 (2005), incorporated herein by this reference. The use of a linker is not required for all purposes and can optionally be omitted. Preferably binding of polypeptide compositions according to the present invention to their specific nucleic acid sequences occurs with a K_{D} of from 10 µM to 1 µM, from 10 pM to 100 nM, from 100 pM to 10 nM and, more preferably with a K_{D} of from 1 nM to 10 nM.

Polypeptide compositions according to the present invention can be a derivative or variant of a naturally-occurring zinc finger protein. The naturally-occurring zinc finger protein can be, but is not limited to, SP1C, TFIIIA or Zif268, as described above. The naturally-occurring zinc finger protein used is referred to herein as a "backbone," as described above.

A polypeptide or polypeptide composition of this invention can be operatively linked to one or more functional polypeptides. Such functional polypeptides can be the complete sequence of proteins with a defined function, or can be derived from single or multiple domains that occur within a protein with a defined function. Such functional polypeptides are well known in the art and can be a transcription regulating factor such as a repressor or activation domain or a peptide having other functions. Exemplary and preferred such functional polypeptides are nucleases, methylases, nuclear localization domains, and restriction enzymes such as endo- or exonucleases (See, e.g. Chandrasegaran and Smith, Biol. Chem., 380:841-848, 1999).

An exemplary repression domain polypeptide is the ERF repressor domain (ERD) (Sgouras, D. N., Athanasiou, M. A., Beal, G. J., Jr., Fisher, R. J., Blair, D. G. & Mavrothalassitis, G. J. (1995) EMBO J. 14, 4781-4793), defined by amino acids 473 to 530 of the ets2 repressor factor (ERF). This domain mediates the antagonistic effect of ERF on the activity of transcription factors of the ets family. A synthetic repressor is constructed by fusion of this domain to the N- or C-terminus of the zinc finger protein. A second repressor protein is prepared using the Kruppel-associated box (KRAB) domain (Margolin, J. F., Friedman, J. R., Meyer, W., K.-H., Vissing, H., Thiesen, H.-J. & Rauscher III, F. J. (1994) Proc. Natl. Acad. Sci. USA 91, 4509-4513). This repressor domain is commonly found at the N-terminus of zinc finger proteins and presumably exerts its repressive activity on TATA-dependent transcription in a distance-and orientation-independent manner (Pengue, G. & Lania, L. (1996) Proc. Natl. Acad. Sci. USA 93, 1015-1020), by interacting with the RING finger protein KAP-1 (Friedman, J. R., Fredericks, W. J., Jensen, D. E., Speicher, D. W., Huang, X.-P., Neilson, E. G. & Rauscher III, F. J. (1996) Genes & Dev. 10,2067-2078). We utilized the KRAB domain found between amino acids 1 and 97 of the zinc finger protein KOX1 (Margolin, J. F., Friedman, J. R., Meyer, W., K.-H., Vissing, H., Thiesen, H.-J. & Rauscher III, F. J. (1994) Proc. Natl. Acad. Sci. USA 91, 4509-4513). In this case an N-terminal fusion with a zinc-finger polypeptide is constructed. Finally, to explore the utility of histone deacetylation for repression, amino acids 1 to 36 of the Mad mSIN3 interaction domain (SID) are fused to the N-terminus of the zinc finger protein (Ayer, D. E., Laherty, C. D., Lawrence, Q. A., Armstrong, A. P. & Eisenman, R. N. (1996) Mol. Cell. Biol. 16,5772-5781). This small domain is found at the N-terminus of the transcription factor Mad and is responsible for mediating its transcriptional repression by interacting with mSIN3, which in turn interacts the co-repressor N-COR and with the histone deacetylase mRPD1 (Heinzel, T., Lavinsky, R. M., Mullen, T.-M., Soderstrom, M., Laherty, C. D., Torchia, J., Yang, W.-M., Brard, G., & Ngo, S. D. (1997) Nature 387,43-46). To examine gene-specific activation, transcriptional activators are generated by fusing the zinc finger polypeptide to amino acids 413 to 489 of the herpes simplex virus VP16 protein (Sadowski, I., Ma, J., Triezenberg, S. & Ptashne, M. (1988) Nature 335, 563-564), or to an artificial tetrameric repeat of VP16's minimal activation domain (Seipel, K., Georgiev, O. & Schaffler, W. (1992) EMBO J. 11,4961-4968), termed VP64.

A polypeptide composition of this invention, as set forth above, can be operatively linked to one or more transcription modulating or regulating factors. Modulating factors such as transcription activators or transcription suppressors or repressors are well known in the art. Means for operatively linking polypeptides to such factors are also well known in the art. Exemplary and preferred such factors and their use to modulate gene expression are discussed in detail hereinafter.

In order to test the concept of using zinc finger proteins as gene-specific transcriptional regulators, six-finger proteins are fused to a number of effector domains. Transcriptional repressors are generated by attaching either of three human-derived repressor domains to the zinc finger protein. The first repressor protein is prepared using the ERF repressor domain (ERD) (Sgouras, D. N., Athanasiou, M. A., Beal, G. J., Jr., Fisher, R. J., Blair, D. G. & Mavrothalassitis, G. J. (1995) EMBO J. 14, 4781-4793), defined by amino acids 473 to 530 of the ets2 repressor factor (ERF). This domain mediates the antagonistic effect of ERF on the activity of transcription factors of the ets family. A synthetic repressor is constructed by fusion of this domain to the C-terminus of the zinc finger protein. The second repressor protein is prepared using the Kruppel-associated box (KRAB) domain (Margolin, J. F., Friedman, J. R., Meyer, W., K.-H., Vissing, H., Thiesen, H.-J. & Rauscher III F. J. (1994) Proc. Natl. Acad. Sci. USA 91, 4509-4513). This repressor domain is commonly found at the N-terminus of zinc finger proteins and presumably exerts its repressive activity on TATA-dependent transcription in a distance- and orientation-independent manner (Pengue, G. & Lania, L. (1996) Proc. Natl. Acad. Sci. USA 93,1015-1020), by interacting with the RING finger protein KAP-1 (Friedman, J. R., Fredericks, W. J., Jensen, D. E., Speicher, D. W., Huang, X.-P., Neilson, E. G. & Rauscher III, F. J. (1996) Genes & Dev. 10, 2067-2078). We utilize the KRAB domain found between amino acids 1 and 97 of the zinc finger protein KOX1 (Margolin, J. F., Friedman, J. R., Meyer, W., K.-H., Vissing, H., Thiesen, H.-J. & Rauscher III, F. J. (1994) Proc. Natl. Acad. Sci. USA 91, 4509-4513). In this case an N-terminal fusion with the six-finger protein is constructed. Finally, to explore the utility of histone deacetylation for repression, amino acids 1 to 36 of the Mad mSIN3 interaction domain (SID) are fused to the N-terminus of a zinc finger protein (Ayer, D. E., Laherty, C. D., Lawrence, Q. A., Armstrong, A. P. & Eisenman, R. N. (1996) Mol. Cell. Biol. 16, 5772-5781). This small domain is found at the N-terminus of the transcription factor Mad and is responsible for mediating its transcriptional repression by interacting with mSIN3, which in turn interacts the co-repressor N-CoR and with the histone deacetylase mRPD1 (Heinzel, T., Lavinsky, R. M., Mullen, T.-M., Soderstrom, M., Laherty, C. D., Torchia, J., Yang, W.-M., Brard, G., & Ngo, S. D. (1997) Nature 387, 43-46). Additional examples of six-finger zinc finger proteins fused to effector domains to generate artificial transcription factors are described in P. Blancafort et al., "Genetic Reprogramming of Tumor Cells by Zinc Finger Transcription Factors," Proc. Natl. Acad. Sci. USA 102: 11716-11721 (2005), incorporated herein by this reference, which described the selection and characterization of a six-zinc-finger artificial transcription factor containing the VP64 activator domain, which is capable of inducing drug resistance, cytoskeleton remodeling, matrix-dependent cell migration, and tumor cell invasion *in vitro.* Also, synthetic six- and twelve-finger synthetic zinc finger protein artificial transcription factors were shown to alter the levels of ErbB2 and ErbB3 receptors in an epidermoid squamous cell carcinoma line (C.V. Lund et al., "Zinc Finger Transcription Factors Designed for Bispecific Coregulation of ErbB2 and ErbB3 Receptors: Insights into ErbB Receptor Biology," Mol. Cell. Biol. 25: 9082-9091 (2005)), incorporated herein by this reference.

To examine gene-specific activation, transcriptional activators are generated by fusing the zinc finger protein to amino acids 413 to 489 of the herpes simplex virus VP 16 protein (Sadowski, I., Ma, J., Triezenberg, S. & Ptashne, M. (1988) Nature 335, 563-564), or to an artificial tetrameric repeat of VP16's minimal activation domain, DALDDFDLDML (SEQ ID NO:36) (Seipel, K., Georgiev, O. & Schaffner, W. (1992) EMBO J. 11, 49614968), termed VP64.

Reporter constructs containing fragments of the erbB-2 promoter coupled to a luciferase reporter gene are generated to test the specific activities of our designed transcriptional regulators. The target reporter plasmid contains nucleotides -758 to -1 with respect to the ATG initiation codon. Promoter fragments display similar activities when transfected transiently into HeLa cells, in agreement with previous observations (Hudson, L. G., Ertl, A. P. & Gill, G. N. (1990) J. Biol. Chem. 265, 4389-4393). To test the effect of zinc finger-repressor domain fusion constructs on erbB-2 promoter activity, HeLa cells are transiently co-transfected with zinc finger expression vectors and the luciferase reporter constructs. Significant repression is observed with each construct. The utility of gene-specific polydactyl proteins to mediate activation of transcription is investigated using the same two reporter constructs.

The data herein show that zinc finger proteins capable of binding novel 9- and 18-bp DNA target sites can be rapidly prepared using pre-defined domains recognizing 5'-(CNN)-3' sites. This information is sufficient for the preparation of 166 or 17 million novel six-finger proteins each capable of binding 18 bp of DNA sequence. This rapid methodology for the construction of novel zinc finger proteins has advantages over the sequential generation and selection of zinc finger domains proposed by others (Greisman, H. A. & Pabo, C. O. (1997) Science 275, 657-661) and takes advantage of structural information that suggests that the potential for the target overlap problem as defined above might be avoided in proteins targeting 5'-(CNN)-3' sites. Using the complex and well studied erbB-2 promoter and live human cells, the data demonstrate that these proteins, when provided with the appropriate effector domain, can be used to provoke or activate expression and to produce graded levels of repression down to the level of the background in these experiments.

A polypeptide composition according to the present invention, as defined above, can also be incorporated into a fusion protein molecule that possesses other activities, such as an enzymatic activity. Among the types of enzymatic activities that can be incorporated into the fusion protein molecule is an integrase. An example is described in W. Tan et al., "Human Immunodeficiency Virus Type 1 Incorporated with Fusion Proteins Consisting of Integrase and the Designed Polydactyl Zinc Finger Protein E2C Can Bias Integration of Viral DNA into a Predetermined Chromosomal Region in Human Cells," J. Virol. 80: 1939-1948 (2006), incorporated herein by this reference. Similarly, fusion proteins according to the present invention can also include nuclease activity, such as described in S. Alwin et al., "Custom Zinc-Finger Nucleases for Use in Human Cells," Mol. Ther. 12: 610-617 (2005), incorporated herein by this reference, which describes the formation of nucleases that catalyze targeted DNA double-strand breaks that stimulate homologous recombination in mammalian cells, including human cells. These fusion proteins incorporate *Fok*l catalytic domains. Such fusion proteins could be used for gene repair of human stem cells; corrected cells could be expanded *ex vivo* and then reintroduced into patients.

Still other fusion proteins are possible, including fusion proteins including therein domains with two or more activities in addition to the zinc finger nucleotide binding activity characteristic of the polypeptides or polypeptide compositions according to the present invention. Such fusion proteins are also within the scope of the present invention.

### IV. Isolated Heptapeptides

Another aspect of the present invention is an isolated heptapeptide having an α-helical structure and that binds preferentially to a target nucleotides of the formula CNN, where N is A, C, G or T. Preferred target nucleotides are as described above.

Preferably, the heptapeptide has the amino acid sequence of any of SEQ ID NOs: 1-28, 35-45, 48, 54, 57-82, 85-130, and 134-157. More preferably, the heptapeptide has the amino acid sequence of any of SEQ ID NOs: 54, 57-82, 85-130, and 134-157. Still more preferably, the heptapeptide has the amino acid sequence of any of SEQ ID NOs: 2, 14, 18, 63, 64, 65, 66, 67, 68, 69, 70, 71, 72, 73, 75, 77, 78, 79, 80, 81, 82, 134, and 153. Even more preferably, the heptapeptide has the amino acid sequence of any of SEQ ID NOs: 2, 14, 18, 63, 66, 71, 72, 73, 75, 77, 78, 79, 80, 81, 82, 134, and 153. Still even more preferably, the heptapeptide has the amino acid sequence of any of SEQ ID NOs: 2, 14, 18, 63, 66, 71, 72, 73, 75, 77, 78, 79, 80, 81, and 82.

Alternatively, the heptapeptide has an amino acid sequence selected from the group consisting of:
(a) the amino acid sequence of any of SEQ ID NOs: 2, 14, 18, 63, 64, 65, 66, 67, 68, 69, 70, 71, 72, 73, 75, 77, 78, 79, 80, 81, 82, 134, and 153; and
(b) an amino acid sequence differing from the amino acid sequence of any of SEQ ID NOs: 2, 14, 18, 63, 64, 65, 66, 67, 68, 69, 70, 71, 72, 73, 75, 77, 78, 79, 80, 81, 82, 134, and 153 by no more than two conservative amino acid substitutions, wherein the dissociation constant is no greater than 125% of that of the polypeptide before the substitutions are made, and wherein a conservative amino acid substitution is one of the following substitutions: Ala/Gly or Ser; Arg/Lys; Asn/Gln or His; Asp/Glu; Cys/Ser; Gln/Asn; Gly/Asp; Gly/Ala or Pro; His/Asn or Gln; Ile/Leu or Val; Leu/Ile or Val; Lys/Arg or Gin or Glu; Met/Leu or Tyr or Ile; Phe/Met or Leu or Tyr; Ser/Thr; Thr/Ser; Trp/Tyr; Tyr/Trp or Phe; Val/Ile or Leu. Preferably, the amino acid sequence differs from the amino acid sequence of any of SEQ ID NOs: 2, 14, 18, 63, 64, 65, 66, 67, 68, 69, 70, 71, 72, 73, 75, 77, 78, 79, 80, 81, 82, 134, and 153 by no more than one conservative amino acid substitution.

V. Polynucleotides, Expression Vectors, and Transformed Cells

The invention includes a nucleotide sequence encoding a zinc finger nucleotide binding polypeptide, including polypeptides, polypeptide compositions, and isolated heptapeptides as described above. DNA sequences encoding the zinc finger-nucleotide binding polypeptides of the invention, including native, truncated, and extended polypeptides, can be obtained by several methods. For example, the DNA can be isolated using hybridization procedures that are well known in the art. These include, but are not limited to: (1) hybridization of probes to genomic or cDNA libraries to detect shared nucleotide sequences; (2) antibody screening of expression libraries to detect shared structural features; and (3) synthesis by the polymerase chain reaction (PCR). RNA sequences of the invention can be obtained by methods known in the art (See, for example, Current Protocols in Molecular Biology, Ausubel, et al., Eds., 1989).

The development of specific DNA sequences encoding zinc finger-nucleotide binding polypeptides of the invention can be obtained by: (1) isolation of a double-stranded DNA sequence from the genomic DNA; (2) chemical manufacture of a DNA sequence to provide the necessary codons for the polypeptide of interest; and (3) in vitro synthesis of a double-stranded DNA sequence by reverse transcription of mRNA isolated from a eukaryotic donor cell. In the latter case, a double-stranded DNA complement of mRNA is eventually formed which is generally referred to as cDNA. Of these three methods for developing specific DNA sequences for use in recombinant procedures, the isolation of genomic DNA is the least common. This is especially true when it is desirable to obtain the microbial expression of mammalian polypeptides due to the presence of introns. For obtaining zinc finger derived-DNA binding polypeptides, the synthesis of DNA sequences is frequently the method of choice when the entire sequence of amino acid residues of the desired polypeptide product is known. When the entire sequence of amino acid residues of the desired polypeptide is not known, the direct synthesis of DNA sequences is not possible and the method of choice is the formation of cDNA sequences. Among the standard procedures for isolating cDNA sequences of interest is the formation of plasmid-carrying cDNA libraries which are derived from reverse transcription of mRNA which is abundant in donor cells that have a high level of genetic expression. When used in combination with polymerase chain reaction technology, even rare expression products can be clones. In those cases where significant portions of the amino acid sequence of the polypeptide are known, the production of labeled single or double-stranded DNA or RNA probe sequences duplicating a sequence putatively present in the target cDNA may be employed in DNA/DNA hybridization procedures which are carried out on cloned copies of the cDNA which have been denatured into a single-stranded form (Jay, et al., Nucleic Acid Research 11:2325, 1983).

With respect to nucleotide sequences that are within the scope of the invention, all nucleotide sequences encoding the polypeptides that are embodiments of the invention as described are included in nucleotide sequences that are within the scope of the invention. This further includes all nucleotide sequences that encode polypeptides according to the invention that incorporate conservative amino acid substitutions as defined above. This further includes nucleotide sequences that encode larger proteins incorporating the zinc finger domains, including fusion proteins, and proteins that incorporate transcription modulators operatively linked to zinc finger domains. For these purposes, "identity" is defined according to the Needleman-Wunsch algorithm (S.B. Needleman & C.D. Wunsch, "A General Method Applicable to the Search for Similarities in the Amino Acid Sequence of Two Proteins," J. Mol. Biol. 48: 443-453 (1970)).

Nucleotide sequences encompassed by the present invention can also be incorporated into a vector, including, but not limited to, an expression vector, and used to transfect or transform suitable host cells, as is well known in the art.
The vectors incorporating the nucleotide sequences that are encompassed by the present invention are also within the scope of the invention. Host cells that are transformed or transfected with the vector or with polynucleotides or nucleotide sequences of the present invention are also within the scope of the invention. The host cells can be prokaryotic or eukaryotic; if eukaryotic, the host cells can be mammalian cells, insect cells, or yeast cells. If prokaryotic, the host cells are typically bacterial cells.

Transformation of a host cell with recombinant DNA may be carried out by conventional techniques as are well known to those skilled in the art. Where the host is prokaryotic, such as E. coli, competent cells which are capable of DNA uptake can be prepared from cells harvested after exponential growth phase and subsequently treated by the CaCl₂ method by procedures well known in the art. Alternatively, MgCl₂ or RbCl can be used. Transformation can also be performed after forming a protoplast of the host cell or by electroporation.

When the host is a eukaryote, such methods of transfection of DNA as calcium phosphate co-precipitates, conventional mechanical procedures such as microinjection, electroporation, insertion of a plasmid encased in liposomes, or virus vectors may be used.

A variety of host-expression vector systems may be utilized to express the zinc finger derived-nucleotide binding coding sequence. These include but are not limited to microorganisms such as bacteria transformed with recombinant bacteriophage DNA, plasmid DNA or cosmid DNA expression vectors containing a zinc finger derived-nucleotide binding polypeptide coding sequence; yeast transformed with recombinant yeast expression vectors containing the zinc finger-nucleotide binding coding sequence; plant cell systems infected with recombinant virus expression vectors (e.g., cauliflower mosaic virus, CaMV; tobacco mosaic virus, TMV) or transformed with recombinant plasmid expression vectors (e.g., Ti plasmid) containing a zinc finger derived-DNA binding coding sequence; insect cell systems infected with recombinant virus expression vectors (e.g., baculovirus) containing a zinc finger-nucleotide binding coding sequence; or animal cell systems infected with recombinant virus expression vectors (e.g., retroviruses, adenovirus, vaccinia virus) containing a zinc finger derived-nucleotide binding coding sequence, or transformed animal cell systems engineered for stable expression. In such cases where glycosylation may be important, expression systems that provide for translational and post-translational modifications may be used; e.g., mammalian, insect, yeast or plant expression systems.

Depending on the host/vector system utilized, any of a number of suitable transcription and translation elements, including constitutive and inducible promoters, transcription enhancer elements, transcription terminators, etc. may be used in the expression vector (see e.g., Bitter, et al., Methods in Enzymology, 153:516-544, 1987). For example, when cloning in bacterial systems, inducible promoters such as pL of bacteriophage λ, plac, ptrp, ptac (ptrp-lac hybrid promoter) and the like may be used. When cloning in mammalian cell systems, promoters derived from the genome of mammalian cells (e.g., metallothionein promoter) or from mammalian viruses (e.g., the retrovirus long terminal repeat; the adenovirus late promoter; the vaccinia virus 7.5K promoter) may be used. Promoters produced by recombinant DNA or synthetic techniques may also be used to provide for transcription of the inserted zinc finger-nucleotide binding polypeptide coding sequence.

In bacterial systems a number of expression vectors may be advantageously selected depending upon the use intended for the zinc finger derived nucleotide-binding polypeptide expressed. For example, when large quantities are to be produced, vectors which direct the expression of high levels of fusion protein products that are readily purified may be desirable. Those which are engineered to contain a cleavage site to aid in recovering the protein are preferred. Such vectors include but are not limited to the *Escherichia coli* expression vector pUR278 (Ruther, et al., EMBO J., 2:1791, 1983), in which the zinc finger-nucleotide binding protein coding sequence may be ligated into the vector in frame with the lac Z coding region so that a hybrid zinc finger-lac Z protein is produced; plN vectors (Inouye & Inouye, Nucleic Acids Res. 13:3101-3109, 1985; Van Heeke & Schuster, J. Biol. Chem. 264:5503-5509, 1989); and the like.

In yeast, a number of vectors containing constitutive or inducible promoters may be used. For a review see, Current Protocols in Molecular Biology, Vol. 2,1988, Ed. Ausubel, et al., Greene Publish. Assoc. & Wiley Interscience, Ch. 13; Grant, et al., 1987, Expression and Secretion Vectors for Yeast, in Methods in Enzymology, Eds. Wu & Grossman, 31987, Acad. Press, N.Y., Vol. 153, pp.516-544; Glover, 1986, DNA Cloning, Vol. 11, IRL Press, Wash., D.C., Ch. 3; and Bitter, 1987, Heterologous Gene Expression in Yeast, Methods in Enzymology, Eds. Berger & Kimmel, Acad. Press, N.Y., Vol. 152, pp. 673-684; and The Molecular Biology of the Yeast Saccharomyces, 1982, Eds. Strathem et al., Cold Spring Harbor Press, Vols. I and II. A constitutive yeast promoter such as ADH or LEU2 or an inducible promoter such as GAL may be used (Cloning in Yeast, Ch. 3, R. Rothstein In: DNA Cloning Vol. 11, A Practical Approach, Ed. DM Glover, 1986, IRL Press, Wash., D.C.). Alternatively, vectors may be used which promote integration of foreign DNA sequences into the yeast chromosome.

In cases where plant expression vectors are used, the expression of a zinc finger-nucleotide binding polypeptide coding sequence may be driven by any of a number of promoters. For example, viral promoters such as the 35S RNA and 19S RNA promoters of CaMV (Brisson, et al., Nature, 310:511-514, 1984), or the coat protein promoter to TMV (Takamatsu, et al., EMBO J., 6:307-311, 1987) may be used; alternatively, plant promoters such as the small subunit of RUBISCO (Coruzzi, et al., EMBO J. 3:1671-1680, 1984; Broglie, et al., Science 224:838-843, 1984); or heat shock promoters, e.g., soybean hsp17.5-E or hsp17.3-B (Gurley, et al., Mol. Cell. Biol., 6:559-565, 1986) may be used. These constructs can be introduced into plant cells using Ti plasmids, Ri plasmids, plant virus vectors, direct DNA transformation, microinjection, electroporation, etc. For reviews of such techniques see, for example, Weissbach & Weissbach, Methods for Plant Molecular Biology, Academic Press, NY, Section VIII, pp. 421-463, 1988; and Grierson & Corey, Plant Molecular Biology, 2d Ed., Blackie, London, Ch. 7-9, 1988.

An alternative expression system that can be used to express a protein of the invention is an insect system. In one such system, *Autographa californica* nuclear polyhedrosis virus (AcNPV) is used as a vector to express foreign genes. The virus grows in *Spodoptera frugiperda* cells. The zinc finger-nucleotide binding polypeptide coding sequence may be cloned into non-essential regions (in *Spodoptera frugiperda* for example the polyhedrin gene) of the virus and placed under control of an AcNPV promoter (for example the polyhedrin promoter). Successful insertion of the zinc finger-nucleotide binding polypeptide coding sequence will result in inactivation of the polyhedrin gene and production of nonoccluded recombinant virus (i.e., virus lacking the proteinaceous coat coded for by the polyhedrin gene). These recombinant viruses are then used to infect cells in which the inserted gene is expressed. (E.g., see Smith, et al., J. Biol. 46:584, 1983; Smith, U.S. Pat. No. 4,215,051).

Eukaryotic systems, and preferably mammalian expression systems, allow for proper post-translational modifications of expressed mammalian proteins to occur. Therefore, eukaryotic cells, such as mammalian cells that possess the cellular machinery for proper processing of the primary transcript, glycosylation, phosphorylation, and, advantageously secretion of the gene product, are the preferred host cells for the expression of a zinc finger derived-nucleotide binding polypeptide. Such host cell lines may include but are not limited to CHO, VERO, BHK, HeLa, COS, MDCK, -293, and W138.

Mammalian cell systems that utilize recombinant viruses or viral elements to direct expression may be engineered. For example, when using adenovirus expression vectors, the coding sequence of a zinc finger derived polypeptide may be ligated to an adenovirus transcription/translation control complex, e.g., the late promoter and tripartite leader sequence. This chimeric gene may then be inserted into the adenovirus genome by in vitro or in vivo recombination. Insertion in a non-essential region of the viral genome (e.g., region E1 or E3) will result in a recombinant virus that is viable and capable of expressing the zinc finger polypeptide in infected hosts (e.g., see Logan & Shenk, Proc. Natl. Acad. Sci. USA 81:3655-3659, 1984). Alternatively, the vaccinia virus 7.5K promoter may be used. (e.g., see, Mackett, et al., Proc. Natl. Acad. Sci. USA, 79:7415-7419, 1982; Mackett, et al., J. Virol. 49:857-864, 1984; Panicali, et al., Proc. Natl. Acad. Sci. USA, 79:4927-4931, 1982). Of particular interest are vectors based on bovine papilloma virus which have the ability to replicate as extrachromosomal elements (Sarver, et al., Mol. Cell. Biol. 1:486, 1981). Shortly after entry of this DNA into mouse cells, the plasmid replicates to about 100 to 200 copies per cell. Transcription of the inserted cDNA does not require integration of the plasmid into the host's chromosome, thereby yielding a high level of expression. These vectors can be used for stable expression by including a selectable marker in the plasmid, such as the neo gene. Alternatively, the retroviral genome can be modified for use as a vector capable of introducing and directing the expression of the zinc finger-nucleotide binding protein gene in host cells (Cone & Mulligan, Proc. Natl. Acad. Sci. USA 81:6349-6353,1984). High level expression may also be achieved using inducible promoters, including, but not limited to, the metallothionein IIA promoter and heat shock promoters.

For long-term, high-yield production of recombinant proteins, stable expression is preferred. Rather than using expression vectors which contain viral origins of replication, host cells can be transformed with the a cDNA controlled by appropriate expression control elements (e.g., promoter, enhancer, sequences, transcription terminators, polyadenylation sites, etc.), and a selectable marker. The selectable marker in the recombinant plasmid confers resistance to the selection and allows cells to stably integrate the plasmid into their chromosomes and grow to form foci which in turn can be cloned and expanded into cell lines. For example, following the introduction of foreign DNA, engineered cells may be allowed to grow for 1-2 days in an enriched media, and then are switched to a selective media. A number of selection systems may be used, including but not limited to the herpes simplex virus thymidine kinase (Wigler, et al., Cell 11:223, 1977), hypoxanthine-guanine phosphoribosyltransferase (Szybalska & Szybalski, Proc. Natl. Acad. Sci. USA, 48:2026,1962), and adenine phosphoribosyltransferase (Lowy, et al., Cell, 22:817, 1980) genes, which can be employed in tk.sup.-, hgprt.sup.- or aprt.sup.- cells respectively. Also, antimetabolite resistance-conferring genes can be used as the basis of selection; for example, the genes for dhfr, which confers resistance to methotrexate (Wigler, et al., Natl. Acad. Sci. USA,77:3567, 1980; O'Hare, et al., Proc. Natl. Acad. Sci. USA, 78:1527, 1981); gpt, which confers resistance to mycophenolic acid (Mulligan & Berg, Proc. Natl. Acad. Sci. USA, 78:2072, 1981; neo, which confers resistance to the aminoglycoside G418 (Colberre-Garapin, et al., J. Mol. Biol., 150:1, 1981); and hygro, which confers resistance to hygromycin (Santerre, et al., Gene, 30:147, 1984). Recently, additional selectable genes have been described, namely trpB, which allows cells to utilize indole in place of tryptophan; hisD, which allows cells to utilize histinol in place of histidine (Hartman & Mulligan, Proc. Natl. Acad. Sci. USA, 85:804, 1988); and ODC (ornithine decarboxylase) which confers resistance to the ornithine decarboxylase inhibitor, 2-(difluoromethyl)-DL-omithine, DFMO (McConlogue L., In: Current Communications in Molecular Biology, Cold Spring Harbor Laboratory ed., 1987).

Isolation and purification of microbially expressed protein, or fragments thereof provided by the invention, may be carried out by conventional means including preparative chromatography and immunological separations involving monoclonal or polyclonal antibodies. Antibodies provided in the present invention are immunoreactive with the zinc finger-nucleotide binding protein of the invention. Antibody which consists essentially of pooled monoclonal antibodies with different epitopic specificities, as well as distinct monoclonal antibody preparations are provided. Monoclonal antibodies are made from antigen containing fragments of the protein by methods well known in the art (Kohler, et al., Nature, 256:495, 1975; Current Protocols in Molecular Biology, Ausubel, et al., ed., 1989).

VI. Pharmaceutical Compositions

In another aspect, the present invention provides a pharmaceutical composition comprising:
(1) a therapeutically effective amount of a polypeptide, polypeptide composition, or isolated heptapeptide according to the present invention as described above; and
(2) a pharmaceutically acceptable carrier.
Alternatively, the present invention also provides:
(1) a therapeutically effective amount of a nucleotide sequence that encodes a polypeptide, polypeptide composition, or isolated heptapeptide according to the present invention as described above; and
(2) a pharmaceutically acceptable carrier.

As used herein, in this description of a pharmacological composition according to the present invention that includes a therapeutic amount of a nucleotide sequence, the nucleotide sequence can be further incorporated in a vector as described above.

The preparation of a pharmacological composition that contains active ingredients dissolved or dispersed therein is well understood in the art. Typically such compositions are prepared as sterile injectables either as liquid solutions or suspensions, aqueous or non-aqueous, however, solid forms suitable for solution, or suspensions, in liquid prior to use can also be prepared. The preparation can also be emulsified. The active ingredient can be mixed with excipients that are pharmaceutically acceptable and compatible with the active ingredient and in amounts suitable for use in the therapeutic methods described herein. Suitable excipients are, for example, water, saline, dextrose, glycerol, ethanol or the like and combinations thereof. In addition, if desired, the composition can contain minor amounts of auxiliary substances such as wetting or emulsifying agents, as well as pH buffering agents and the like which enhance the effectiveness of the active ingredient. Still other ingredients that are conventional in the pharmaceutical art, such as chelating agents, preservatives, antibacterial agents, antioxidants, coloring agents, flavoring agents, and others, can be employed depending on the characteristics of the composition and the intended route of administration for the composition.

The pharmaceutical composition of the present invention can include pharmaceutically acceptable salts of the components therein. Pharmaceutically acceptable salts include the acid addition salts (formed with the free amino groups of the polypeptide) that are formed with inorganic acids such as, for example, hydrochloric or phosphoric acids, or such organic acids as acetic, tartaric, mandelic and the like. Salts formed with the free carboxyl groups can also be derived from inorganic bases such as, for example, sodium, potassium, ammonium, calcium or ferric hydroxides, and such organic bases as isopropylamine, trimethylamine, 2-ethylaminoethanol, histidine, procaine and the like. Physiologically acceptable carriers are well known in the art. Exemplary of liquid carriers are sterile aqueous solutions that contain no materials in addition to the active ingredients and water, or contain a buffer such as sodium phosphate at physiological pH value, physiological saline or both, such as phosphate-buffered saline. Still further, aqueous carriers can contain more than one buffer salt, as well as salts such as sodium and potassium chlorides, dextrose, propylene glycol, polyethylene glycol and other solutes. Liquid compositions can also contain liquid phases in addition to and to the exclusion of water. Exemplary of such additional liquid phases are glycerin, vegetable oils such as cottonseed oil, organic esters such as ethyl oleate, and water-oil emulsions.

VI. Uses

In one embodiment, a method of the invention includes a process for modulating (inhibiting or suppressing) expression of a nucleotide sequence that contains a CNN target sequence. The method includes the step of contacting the nucleotide with an effective amount of a zinc finger-nucleotide binding polypeptide of this invention that binds to the motif. In the case where the nucleotide sequence is a promoter, the method includes inhibiting the transcriptional transactivation of a promoter containing a zinc finger-DNA binding motif. The term "inhibiting" refers to the suppression of the level of activation of transcription of a structural gene operably linked to a promoter, containing a zinc finger-nucleotide binding motif, for example.
In addition, the zinc finger-nucleotide binding polypeptide can bind a target within a structural gene or within an RNA sequence.

The term "effective amount" includes that amount which results in the deactivation of a previously activated promoter or that amount which results in the inactivation of a promoter containing a target nucleotide, or that amount which blocks transcription of a structural gene or translation of RNA. The amount of zinc finger derived-nucleotide binding polypeptide required is that amount necessary to either displace a native zinc finger-nucleotide binding protein in an existing protein/promoter complex, or that amount necessary to compete with the native zinc finger-nucleotide binding protein to form a complex with the promoter itself.
Similarly, the amount required to block a structural gene or RNA is that amount which binds to and blocks RNA polymerase from reading through on the gene or that amount which inhibits translation, respectively. Preferably, the method is performed intracellularly. By functionally inactivating a promoter or structural gene, transcription or translation is suppressed. Delivery of an effective amount of the inhibitory protein for binding to or "contacting" the cellular nucleotide sequence containing the target sequence can be accomplished by one of the mechanisms described herein, such as by retroviral vectors or liposomes, or other methods well known in the art. The term "modulating" refers to the suppression, enhancement or induction of a function. For example, the zinc finger-nucleotide binding polypeptide of the invention can modulate a promoter sequence by binding to a target sequence within the promoter, thereby enhancing or suppressing transcription of a gene operatively linked to the promoter nucleotide sequence. Alternatively, modulation may include inhibition of transcription of a gene where the zinc finger-nucleotide binding polypeptide binds to the structural gene and blocks DNA dependent RNA polymerase from reading through the gene, thus inhibiting transcription of the gene. The structural gene may be a normal cellular gene or an oncogene, for example. Alternatively, modulation may include inhibition of translation of a transcript.

The promoter region of a gene includes the regulatory elements that typically lie 5' to a structural gene; multiple regulatory elements can be present, separated by intervening nucleotide sequences. If a gene is to be activated, proteins known as transcription factors attach to the promoter region of the gene. This assembly resembles an "on switch" by enabling an enzyme to transcribe a second genetic segment from DNA to RNA. In most cases the resulting RNA molecule serves as a template for synthesis of a specific protein; sometimes RNA itself is the final product.

The promoter region may be a normal cellular promoter or, for example, an onco-promoter. An onco-promoter is generally a virus-derived promoter. For example, the long terminal repeat (LTR) of retroviruses is a promoter region that may be a target for a zinc finger binding polypeptide variant of the invention. Promoters from members of the Lentivirus group, which include such pathogens as human T-cell lymphotrophic virus (HTLV) 1 and 2, or human immunodeficiency virus (HIV) 1 or 2, are examples of viral promoter regions which may be targeted for transcriptional modulation by a zinc finger binding polypeptide of the invention.

A target CNN nucleotide sequence can be located in a transcribed region of a gene or in an expressed sequence tag. A gene containing a target sequence can be a plant gene, an animal gene or a viral gene. The gene can be a eukaryotic gene or prokaryotic gene such as a bacterial gene. The animal gene can be a mammalian gene including a human gene. In a preferred embodiment, a method of modulating nucleotide expression is accomplished by transforming a cell that contains a target nucleotide sequence with a polynucleotide that encodes a polypeptide or composition of this invention. Preferably, the encoding polynucleotide is contained in an expression vector suitable for use in a target cell. Suitable expression vectors are well known in the art.

The CNN target can exist in any combination with other target triplet sequences. That is, a particular CNN target can exist as part of an extended CNN sequence (e.g., [CNN]₂₋₁₂) or as part of any other extended sequence such as (GNN)₁₋₁₂, (ANN)₁₋₁₂, (TNN)₁₋₁₂ or (NNN)₁₋₁₂.

The Examples that follow illustrate preferred embodiments of the present invention and are not limiting of the specification and claims in any way. These Examples, particularly Example 13, are further described in B. Dreier et al., "Development of Zinc Finger Domains for Recognition of the 5'-CNN-3' Family DNA Sequences and Their Use in the Construction of Artificial Transcription Factors," J. Biol. Chem. 280: 35588-35597 (2005), incorporated herein by this reference.

### EXAMPLE 1

### Construction of Zinc Finger Library and Selection via Phage Display

Construction of the zinc finger library was based on the earlier described C7 protein ([Wu et al., (1995) PNAS 92,344-348]; FIG. 1A, upper panel). Finger 3 recognizing the 5'-(GCG)-3' subsite was replaced by a domain binding to a 5'-(GAT)-3' subsite [Segal et al., (1999) Proc Natl Acad Sci USA 96(6), 2758-2763] via a overlap PCR strategy using a primer coding for finger 3 (5'-GAGGAAGTTTGCCACCAGTGGCAACCTG GTGAGGCATACCAAAATC-3') (SEQ ID NO:31) and a pMal-specific primer (5'-GTAAAACGACGGCCAG TGCCAAGC-3') (SEQ ID NO:32). Randomization the zinc finger library by PCR overlap extension was essentially as described [Wu et al., (1995) PNAS 92, 344-348; Segal et al., (1999) Proc Natl Acad Sci USA 96(6), 2758-2763]. The library was ligated into the phagemid vector pComb3H [Rader et al., (1997) Curr. Opin. Biotechnol. 8(4), 503-508]. Growth and precipitation of phage were performed as previously described [Barbas et al., (1991) Methods: Companion Methods Enzymol. 2(2), 119-124; Barbas et al., (1991) Proc. Natl. Acad. Sci. USA 88, 7978-7982; Segal et al., (1999) Proc Natl Acad Sci USA 96(6), 2758-2763]. Binding reactions were performed in a volume of 500 µl zinc buffer A (ZBA: 10 mM Tris, pH 7.5/90 mM KCl/lm M MgCl₂/90 mM ZnCl₂)/0.2% BSA/5 mM DTT/1% Blotto (Biorad)/20 mg double-stranded, sheared herring sperm DNA containing 100 µl precipitated phage (10¹³ colony-forming units). Phage were allowed to bind to non-biotinylated competitor oligonucleotides for 1 hr at 4° C before the biotinylated target oligonucleotide was added. Binding continued overnight at 4° C. After incubation with 50 µl streptavidin coated magnetic beads (Dynal; blocked with 5% Blotto in ZBA) for 1 hr, beads were washed ten times with 500 µl ZBA/2% Tween 20/5 mM DTT, and once with buffer containing no Tween. Elution of bound phage was performed by incubation in 25 µl trypsin (10 µg/µl) in TBS (Tris-buffered saline) for 30 min at room temperature. Hairpin competitor oligonucleotides had the sequence 5'-GGCCGCN'N'N'ATCGAGTTTTCTCGATNN NGCGGCC-3' (SEQ ID NO:33) (target oligonucleotides were biotinylated), where NNN represents the finger-2 subsite oligonucleotides, N'N'N' its complementary bases. Target oligonucleotides were usually added at 72 nM in the first three rounds of selection, then decreased to 36 nM and 18 nM in the sixth and last round. As competitor a 5'-(TGG)-3' finger-2 subsite oligonucleotide was used to compete with the parental clone. An equimolar mixture of 15 finger-2 5'-(CNN)-3' subsites, except for the target site, respectively, and competitor mixtures of each finger-2 subsites of the type 5'-(ANN)-3', 5'-(GNN)-3', and 5'-(TNN)-3' were added in increasing amounts with each successive round of selection. Usually no specific 5'-(CNN)-3' competitor mix was added in the first round.

### EXAMPLE 2

Multitarget Specificity Assay and Gel Mobility Shift Analysis--The zinc finger-coding sequence was subcloned from pComb3H into a modified bacterial expression vector pMal-c2 (New England Biolabs). After transformation into XL1-Blue (Stratagene) the zinc finger-maltose-binding protein (MBP) fusions were expressed after addition of 1 mM isopropyl-β-D-thiogalactoside (IPTG). Freeze/thaw extracts of these bacterial cultures were applied in 1:2 dilutions to 96-well plates coated with streptavidin (Pierce), and were tested for DNA-binding specificity against each of the sixteen 5'-(GAT CNN GCG)-3' (SEQ ID NO:34) target sites, respectively. ELISA (enzyme-linked immunosorbent assay) was performed essentially as described [Segal et al., (1999) Proc Natl Acad Sci USA 96(6), 2758-2763; Dreier et al., (2000) J. Mol. Biol. 303, 489-502]. After incubation with a mouse anti-MBP (maltose-binding protein) antibody (Sigma, 1:1000), a goat anti-mouse antibody coupled with alkaline phosphatase (Sigma, 1:1000) was applied. Detection followed by addition of alkaline phosphatase substrate (Sigma), and the OD₄₀₅ was determined with SOFTMAX2.35 (Molecular Devices).

Gelshift analysis was performed with purified protein (Protein Fusion and Purification System, New England Biolabs) essentially as described.

### EXAMPLE 3

### Site-Directed Mutagenesis of Finger-2

Finger-2 mutants were constructed by PCR as described [Segal et al., (1999) Proc Natl Acad Sci USA 96(6), 2758-2763; Dreier et al., (2000) J. Mol. Biol. 303, 489-502]. As PCR template the library clone containing 5'-TGG-3' finger 2 and 5'-GAT-3' finger 3 was used. PCR products containing a mutagenized finger 2 and 5'-GAT-3' finger 3 were subcloned via Nsil and Spel restriction sites in frame with finger 1 of C7 into a modified pMal-c2 vector (New England Biolabs). Three-finger proteins were constructed by finger-2 stitchery using the SP1C framework as described [Beerli et al., (1998) Proc Natl Acad Sci USA 95(25), 14628-14633]. The proteins generated in this work contained helices recognizing 5'-GNN-3' DNA sequences [Segal et al., (1999) Proc Natl Acad Sci USA 96(6), 2758-2763], as well as 5'-ANN-3' and 5'-TAG-3' helices described here. Six finger proteins were assembled via compatible Xmal and BsrFi restriction sites. Analysis of DNA-binding properties were performed from IPTG-induced freeze/thaw bacterial extracts.

### EXAMPLE 4

### General Methods

Transfection and Luciferase Assays

HeLa cells were used at a confluency of 40-60%. Cells were transfected with 160 ng reporter plasmid (pGL3-promoter constructs) and 40 ng of effector plasmid (zinc finger-effector domain fusions in pcDNA3) in 24 well plates. Cell extracts were prepared 48 hrs after transfection and measured with luciferase assay reagent (Promega) in a MicroLumat LB96P luminometer (EG & Berthold, Gaithersburg, Md.).

Retroviral Gene Targeting and Flow Cytometric Analysis

These assays were performed as described [Beerli et al., (2000) Proc Natl Acad Sci U S A 97(4), 1495-1500; Beerli et al., (2000) J. Biol. Chem. 275(42), 32617-32627]. As primary antibody an ErbB-1-specific mAb EGFR (Santa Cruz), ErbB-2-specific mAb FSP77 (gift from Nancy E. Hynes; Harwerth et al., 1992) and an ErbB-3-specific mAb SGP1 (Oncogene Research Products) were used. Fluorescently labeled donkey F(ab')₂ anti-mouse IgG was used as secondary antibody (Jackson Immuno-Research).

### EXAMPLE 5

### Bacterial Extracts of pMal-Fusion Proteins for ELISA Assays

The selected zinc finger proteins were cloned into the pMal vector (New England Biolabs) for expression. The constructs were transferred into the E. coli strain XL1-Blue by electroporation and streaked on LB plates containing 50 µg/ml carbenecillin. Four single colonies of each mutant were inoculated into 3 ml of SB media containing 50 µg/ml carbenecillin and 1% glucose. Cultures were grown overnight at 37° C. 1.2 ml of the cultures were transformed into 20 ml of fresh SB media containing 50 µg/ml Carbenecillin, 0.2% glucose, 90 µg/ml ZnCl₂ and grown at 37° C for another 2 hours. IPTG was added to a final concentration of 0.3 mM. incubation was continued for 2 hours. The cultures were centrifuged at 4° C for 5 minutes at 3500 rpm in a Beckman GPR centrifuge. Bacterial pellets were resuspended in 1.2 ml of Zinc Buffer A containing 5 mM fresh DTT. Protein extracts were isolated by freeze/thaw procedure using dry ice/ethanol and warm water. This procedure was repeated 6 times. Samples were centrifuged at 4° C for 5 minutes in an Eppendorf centrifuge. The supernatant was transferred to a clean 1.5 ml centrifuge tube and used for the ELISA assays.

ELISA assays--Finger-2 variants of C7.GAT were subcloned into bacterial expression vector as fusion with maltose-binding protein (MBP) and proteins were expressed by induction with 1 mM IPTG (proteins (p) are given the name of the finger-2 subsite against which they were selected). Proteins were tested by enzyme-linked immunosorbant assay (ELISA) against each of the 16 finger-2 subsites of the type 5'-(GAT CNN GCG)-3' (SEQ ID NO:34) to investigate their DNA-binding specificity.

In addition, the 5'-nucleotide recognition was analyzed by exposing zinc finger proteins to the specific target oligonucleotide and three subsites which differed only in the 5'-nucleotide of the middle triplet. For example, pCAA was tested on 5'-AAA-3' ,5'-CAA-3', 5'-GAA-3', and 5'-TAA-3' subsites. Many of the tested 3-finger proteins showed exquisite DNA-binding specificity for the finger-2 subsite against which they were selected (Table 1).

**TABLE 1**

| TARGET | ZINC FINGER HEPTAMER | |
|---|---|---|
| CAA | SEQ ID NO: 1 | QRHNLTE |
| | SEQ ID NO: 2 | QSGNLTE |
| CAC | SEQ ID NO: 3 | NLQHLGE |
| CAG | SEQ ID NO: 4 | RADNLTE |
| | SEQ ID NO: 5 | RADNLAI |
| | SEQ ID NO: 14 | RSDHLTE |
| | SEQ ID NO: 16 | RSDHLTD |
| | SEQ ID NO: 8 | RNDTLTE |
| CAT | SEQ ID NO: 1 | QRHNLTE |
| | SEQ ID NO: 6 | NTTHLEH |
| | SEQ ID NO: 24 | TKQTLTE |
| | SEQ ID NO: 3 | NLQHLGE |
| CCA | SEQ ID NO: 6 | NTTHLEH |
| | SEQ ID NO: 25 | QSGDLTE |
| CCC | SEQ ID NO: 7 | SKKHLAE |
| CCG | SEQ ID NO: 8 | RNDTLTE |
| | SEQ ID NO: 9 | RNDTLQA |
| CCT | SEQ ID NO: 6 | NTTHLEH |
| CGA | SEQ ID NO: 10 | QSGHLTE |
| | SEQ ID NO: 11 | QLAHLKE |
| | SEQ ID NO: 12 | QRAHLTE |
| | SEQ ID NO: 17 | RSDHLTN |
| CGC | SEQ ID NO: 13 | HTGHLLE |
| CGG | SEQ ID NO: 14 | RSDHLTE |
| | SEQ ID NO: 15 | RSDKLTE |
| | SEQ ID NO: 16 | RSDHLTD |
| | SEQ ID NO: 17 | RSDHLTN |
| | SEQ ID NO: 8 | RNDTLTE |
| CGT | SEQ ID NO: 18 | SRRTCRA |
| | SEQ ID NO: 19 | QLRHLRE |
| | SEQ ID NO: 7 | SKKHLAE |
| CTA | SEQ ID NO: 20 | QRHSLTE |
| CTC | SEQ ID NO: 21 | QLAHLKR |
| | SEQ ID NO: 22 | NLQHLGE |
| CTG | SEQ ID NO: 23 | RNDALTE |
| | SEQ ID NO: 5 | RADNLAI |
| | SEQ ID NO: 8 | RNDTLTE |
| | SEQ ID NO: 14 | RSDHLTE |
| | SEQ ID NO: 9 | RNDTLQA |
| CTT | SEQ ID NO: 6 | NTTHLEH |

### EXAMPLE 6

### Gel Mobility Shift Assays

Zinc finger polypeptides linked to transcription regulating factors are purified to >90% homogeneity using the Protein Fusion and Purification System (New England Biolabs), except that ZBA/5 mM DTT is used as the column buffer. Protein purity and concentration are determined from Coomassie blue-stained 15% SDS-PAGE gels by comparison to BSA standards. Target oligonucleotides are labeled at their 5' or 3' ends with [³²P] and gel purified. Eleven 3-fold serial dilutions of protein are incubated in 20 µl binding reactions (1 x Binding Buffer/10% glycerol/>>1 pM target oligonucleotide) for three hours at room temperature, then resolved on a 5% polyacrylamide gel in 0.5 x TBE buffer. Quantitation of dried gels is performed using a Phosphorimager and ImageQuant software (Molecular Dynamics), and the K_{D} was determined by Scatchard analysis.

### EXAMPLE 7

### Construction of Zinc Finger-Effector Domain Fusion Proteins

For the construction of zinc finger-effector domain fusion proteins, DNAs encoding amino acids 473 to 530 of the ets repressor factor (ERF) repressor domain (ERD) (Sgouras, D. N., Athanasiou, M. A., Beal, G. J., Jr., Fisher, R. J., Blair, D. G. & Mavrothalassitis, G. J. (1995) EMBO J. 14, 4781-4793), amino acids 1 to 97 of the KRAB domain of KOX1 (Margolin, J. F., Friedman, J. R., Meyer, W., K.-H., Vissing, H., Thiesen, H.-J. & Rauscher III, F. J. (1994) Proc. Natl. Acad. Sci. USA 91, 4509-4513), or amino acids 1 to 36 of the Mad mSIN3 interaction domain (SID) (Ayer, D. E., Laherty, C. D., Lawrence, Q. A., Armstrong, A. P. & Eisenman, R. N. (1996) Mol. Cell. Biol. 16, 5772-5781) are assembled from overlapping oligonucleotides using Taq DNA polymerase. The coding region for amino acids 413 to 489 of the VP16 transcriptional activation domain (Sadowski, L, Ma, J., Triezenberg, S. & Ptashne, M. (1988) Nature 335, 563-564) is PCR amplified from pcDNA3/C₇-C₇-VP16 (10). The VP64 DNA, encoding a tetrameric repeat of VP16's minimal activation domain, comprising amino acids 437 to 447 (Seipel, K., Georgiev, O. & Schaffner, W. (1992) EMBO J. 11, 4961-4968), is generated from two pairs of complementary oligonucleotides. The resulting fragments are fused to zinc finger coding regions by standard cloning procedures, such that each resulting construct contained an internal SV40 nuclear localization signal, as well as a C-terminal HA decapeptide tag. Fusion constructs are cloned in the eukaryotic expression vector pcDNA3 (Invitrogen).

### EXAMPLE 8

### Construction of Luciferase Reporter Plasmids

An erbB-2 promoter fragment comprising nucleotides -758 to -1, relative to the ATG initiation codon, is PCR amplified from human bone marrow genomic DNA with the TaqExpand DNA polymerase mix (Boehringer Mannheim) and cloned into pGL3basic (Promega), upstream of the firefly luciferase gene. A human erbB-2 promoter fragment encompassing nucleotides -1571 to -24, is excised from pSVOALD5'/erbB-2(N--N) (Hudson, L. G., Ertl, A. P. & Gill, G. N. (1990) J. Biol. Chem. 265, 4389-4393) by Hind3 digestion and subcloned into pGL3basic, upstream of the firefly luciferase gene.

### EXAMPLE 9

### Luciferase Assays

For all transfections, HeLa cells are used at a confluency of 40-60%. Typically, cells are transfected with 400 ng reporter plasmid (pGL3-promoter constructs or, as negative control, pGL3basic), 50 ng effector plasmid (zinc finger constructs in pcDNA3 or, as negative control, empty pcDNA3), and 200 ng internal standard plasmid (phrAct-bGal) in a well of a 6 well dish using the lipofectamine reagent (Gibco BRL). Cell extracts are prepared approximately 48 hours after transfection. Luciferase activity is measured with luciferase assay reagent (Promega), βGal activity with Galacto-Light (Tropix), in a MicroLumat LB 96P luminometer (EG&G Berthold). Luciferase activity is normalized on βGal activity.

### EXAMPLE 10

### Regulation of the erbB-2 Gene in Hela Cells

The erbB-2 gene is targeted for imposed regulation. To regulate the native erbB-2 gene, a synthetic repressor protein and a transactivator protein are utilized (R. R. Beerli, D. J. Segal, B. Dreier, C. F. Barbas, III, Proc. Natl. Acad. Sci. USA 95, 14628 (1998)). This DNA-binding protein is constructed from 6 pre-defined and modular zinc finger domains (D. J. Segal, B. Dreier, R. R. Beerli, C. F. Barbas, III, Proc. Natl. Acad. Sci. USA 96, 2758 (1999)). The repressor protein contains the Kox-1 KRAB domain (J. F. Margolin et al., Proc. Natl. Acad. Sci. USA 91, 4509 (1994)), whereas the transactivator VP64 contains a tetrameric repeat of the minimal activation domain (K. Seipel, O. Georgiev, W. Schaffner, EMBO J. 11,4961 (1992)) derived from the herpes simplex virus protein VP16.

A derivative of the human cervical carcinoma cell line HeLa, HeLa/tet-off, is utilized (M. Gossen and H. Bujard, Proc. Natl. Acad. Sci. USA 89, 5547 (1992)). Since HeLa cells are of epithelial origin they express ErbB-2 and are well suited for studies of erbB-2 gene targeting. HeLa/tet-off cells produce the tetracycline-controlled transactivator, allowing induction of a gene of interest under the control of a tetracycline response element (TRE) by removal of tetracycline or its derivative doxycycline (Dox) from the growth medium. We use this system to place our transcription factors under chemical control. Thus, repressor and activator plasmids are constructed and subcloned into pRevTRE (Clontech) using BamHI and Clal restriction sites, and into PMX-IRES-GFP [X. Liu et al., Proc. Natl. Acad. Sci. USA 94, 10669 (1997)] using BamHl and Notl restriction sites. Fidelity of the PCR amplification are confirmed by sequencing, transfected into HeLa/tet-off cells, and 20 stable clones each are isolated and analyzed for Dox-dependent target gene regulation. The constructs are transfected into the HeLa/tet-off cell line (M. Gossen and H. Bujard, Proc. Natl. Acad. Sci. USA 89, 5547 (1992)) using Lipofectamine Plus reagent (Gibco BRL). After two weeks of selection in hygromycin-containing medium, in the presence of 2 mg/ml Dox, stable clones are isolated and analyzed for Dox-dependent regulation of ErbB-2 expression. Western blots, immunoprecipitations, Northern blots, and flow cytometric analyses are carried out essentially as described [D. Graus-Porta, R. R. Beerli, N. E. Hynes, Mol. Cell. Biol. 15, 1182 (1995)]. As a read-out of erbB-2 promoter activity, ErbB-2 protein levels are initially analyzed by Western blotting. A significant fraction of these clones will show regulation of ErbB-2 expression upon removal of Dox for 4 days, i.e., downregulation of ErbB-2 in repressor clones and upregulation in activator clones. ErbB-2 protein levels are correlated with altered levels of their specific mRNA, indicating that regulation of ErbB-2 expression is a result of repression or activation of transcription.

### EXAMPLE 11

### Introduction of the Coding Regions of the E2S-KRAB. E2S-VP64. E3F-KRAB and E3F-VP64 Proteins Into the Retroviral Vector pM-IRES-GFP.

In order to express the E2S-KRAB, E2S-VP64, E3F-KRAB and E3F-VP64 proteins (See Table 2, below) in several cell lines, their coding regions were introduced into the retroviral vector pMX-IRES-GFP.

The sequences of these constructs were selected to bind to specific regions of the ErbB-2 or ErbB-3 promoters (See Table 2). The coding regions were PCR amplified from pcDNA3-based expression plasmids (R. R. Beerli, D. J. Segal, B. Dreier, C. F. Barbas, III, Proc. Natl. Acad. Sci. USA 95, 14628 (1998)) and subcloned into pRevTRE (Clontech) using BamHI and ClaI restriction sites, and into pMX-IRES-GFP [X. Liu et al., Proc. Natl. Acad. Sci. USA 94, 10669 (1997)] using BamHl and Notl restriction sites. Fidelity of the PCR amplification was confirmed by sequencing. This vector expresses a single bicistronic message for the translation of the zinc finger protein and, from an internal ribosome-entry site (IRES), the green fluorescent protein (GFP). Since both coding regions share the same mRNA, their expression is physically linked to one another and GFP expression is an indicator of zinc finger expression. Virus prepared from these plasmids was then used to infect the human carcinoma cell line A431.

**TABLE 2**

| | | | | | | |
|---|---|---|---|---|---|---|
| DNA Target e2t 5'→3' | CAA | CGA | AGT | CTG | CGA | GTC |
| Zinc Finger Sequence E2T | QRHNLTE | QLAHLlKE | HRTTLTN | RNDALTE | QRAHLER | DPGALVR |
| SEQ ID NO: | 1 | 11 | 35 | 23 | 36 | 37 |
| DNA Target e2s 5'→3' | CGG | GGG | GCT | CCC | CTG | GTT |
| Zinc Finger Sequence E2S | RSDHLTE | RSDKLVR | TSGELYR | SKKRLAE | RNDALTE | TSGSLVR |
| SEQ ID NO: | 14 | 38 | 39 | 7 | 23 | 39 |
| DNA Target e3f 5'→3' | AGG | GGC | CCC | CGG | GCC | GGA |
| Zinc Finger Sequence E3F | RSDHLTN | DPGULVR | SKKHLAE | RSDHLTE | DCRDLAR | QRAHLER |
| SEQ ID NO: | 40 | 41 | 7 | 14 | 42 | 36 |

### EXAMPLE 12

### Regulation of ErbB-2 and ErbB-3 Gene Expression

Plasmids from Example 11 were transiently transfected into the amphotropic packaging cell line Phoenix Ampho using Lipofectamine Plus (Gibco BRL) and, two days later, culture supernatants were used for infection of target cells in the presence of 8 mg/ml polybrene. Three days after infection, cells were harvested for analysis. Three days after infection, ErbB-2 and ErbB-3 expression was measured by flow cytometry. The results show that E2S-KRAB and E2S-VP64 compositions inhibited and enhanced ErbB-2 gene expression, respectively. The data also show that E3F-KRAB and E3F-VP64 compositions inhibited and enhanced ErbB-2 gene expression, respectively.

The human erbB-2 and erbB-3 genes were chosen as model targets for the development of zinc finger-based transcriptional switches. Members of the ErbB receptor family play important roles in the development of human malignancies. In particular, erbB-2 is overexpressed as a result of gene amplification and/or transcriptional deregulation in a high percentage of human adenocarcinomas arising at numerous sites, including breast, ovary, lung, stomach, and salivary gland (Hynes, N. E. & Stem, D. F. (1994) Biochim. Biophys. Acta 1198, 165-184). Increased expression of ErbB-2 leads to constitutive activation of its intrinsic tyrosine kinase, and has been shown to cause the transformation of cultured cells. Numerous clinical studies have shown that patients bearing tumors with elevated ErbB-2 expression levels have a poorer prognosis (Hynes, N. E. & Stem, D. F. (1994) Biochim. Biophys. Acta 1198, 165-184). In addition to its involvement in human cancer, erbB-2 plays important biological roles, both in the adult and during embryonic development of mammals (Hynes, N. E. & Stem, D. F. (1994) Biochim. Biophys. Acta 1198, 165-184, Altiok, N., Bessereau, J.-L. & Changeux, J.-P. (1995) EMBO J. 14, 4258-4266, Lee, K.-F., Simon, H., Chen, H., Bates, B., Hung, M.-C. & Hauser, C. (1995) Nature 378, 394-398).

The erbB-2 promoter therefore represents an interesting test case for the development of artificial transcriptional regulators. This promoter has been characterized in detail and has been shown to be relatively complex, containing both a TATA-dependent and a TATA-independent transcriptional initiation site (Ishii, S., Imamoto, F., Yamanashi, Y., Toyoshima, K. & Yamamoto, T. (1987) Proc. Natl. Acad. Sci. USA 84, 43744378). Whereas early studies showed that polydactyl proteins could act as transcriptional regulators that specifically activate or repress transcription, these proteins bound upstream of an artificial promoter to six tandem repeats of the proteins binding site (Liu, Q., Segal, D. J., Ghiara, J. B. & Barbas, C. F. (1997) Proc. Natl. Acad. Sci. USA 94, 5525-5530). Furthermore, this study utilized polydactyl proteins that were not modified in their binding specificity. Herein, we tested the efficacy of polydactyl proteins assembled from predefined building blocks to bind a single site in the native erbB-2 and erbB-3 promoter.

For generating polydactyl proteins with desired DNA-binding specificity, the present studies have focused on the assembly of predefined zinc finger domains, which contrasts the sequential selection strategy proposed by Greisman and Pabo (Greisman, H. A. & Pabo, C. O. (1997) Science 275, 657-661). Such a strategy would require the sequential generation and selection of six zinc finger libraries for each required protein, making this experimental approach inaccessible to most laboratories and extremely time-consuming to all. Further, since it is difficult to apply specific negative selection against binding alternative sequences in this strategy, proteins may result that are relatively unspecific as was recently reported (Kim, J.-S. & Pabo, C. O. (1997) J. Biol. Chem. 272, 29795-29800).

The general utility of two different strategies for generating three-finger proteins recognizing 18 bp of DNA sequence was investigated. Each strategy was based on the modular nature of the zinc finger domain, and takes advantage of a family of zinc finger domains recognizing triplets of the 5'-(NNN)-3'. Three six-finger proteins recognizing half-sites of erbB-2 or erbB-3 target sites were generated in the first strategy by fusing the pre-defined finger 2 (F2) domain variants together using a PCR assembly strategy.

The affinity of each of the proteins for its target was determined by electrophoretic mobility-shift assays. These studies demonstrated that the zinc finger peptides have affinities comparable to Zif268 and other natural transcription factors.

The affinity of each protein for the DNA target site is determined by gel-shift analysis.

### EXAMPLE 13

### Development of Additional Zinc Finger Domains Specifically Binding (CNN) Sequences

The ability to rapidly prepare proteins with predefined specificities for DNA sequences could enable a wide range of technologies that might be used for example to direct the expression of genes or to physically modify genes and genomes. In order to develop a universal system for gene regulation, much effort has been applied to the development of artificial transcription factors based on polydactyl zinc finger proteins. (1-3). Such a system might have considerable impact on biology and biotechnology and offer a new approach for treatment of diseases based on directed gene regulation. It has now been shown that gene expression can be specifically altered using artificial transcription factors based on polydactyl zinc finger proteins that bind to 18 base pair (bp) target sites (1,2). Targeting of sites as small as 9 bp can also provide some degree of regulatory specificity presumably through the aid of chromatin occlusion (4-6). In addition to transcriptional regulation, novel zinc finger DNA-binding specificities are showing tremendous promise in directing homologous recombination through their fusion with the Fok I nuclease domain (7,8).

Zinc finger domains of the type Cys₂-His₂ are a unique and promising class of proteins for the recognition of extended DNA sequences due to their modular nature. Each domain consists of approximately 30 amino acids folded into a ββα structure stabilized by hydrophobic interactions and chelation of a zinc ion by the conserved Cys₂-His₂ residues (9,10). To date, the best-characterized protein of this family of zinc finger proteins is the mouse transcription factor Zif268. Each of the three zinc finger domains of Zif268 binds to a 3 bp subsite by insertion of the α-recognition helix into the major groove of the DNA double helix (11,12). To facilitate the rapid construction of DNA-binding proteins and to study protein-DNA interactions, we have previously created domains that bind to the 5'-GNN-3' and 5'-ANN-3' family of DNA sequences (13-15). We demonstrated that these domains function as modular recognition units that can be assembled into polydactyl zinc finger proteins that specifically recognize from 9 to 18 bp target sites. Significantly, an 18 bp site is long enough to potentially be unique within the human, or any other genome and transcriptional specificity of such proteins has been demonstrated in transgenic plants and human cells using array analysis (16,17). In addition to constitutive regulation, fusion of ligand-binding domains from nuclear hormone receptors with specific binding domains provides inducible gene regulation with this class of transcription factors (18). To provide for ultimate freedom in DNA targeting it is important to identify the 64 DNA-binding domains required to target each possible 3-bp subsite.

Due to the limited structural data on zinc finger/DNA interactions (19-24) de novo design of zinc proteins that bind with a high degree of specificity to novel sequences has been of limited success (25). Significantly, for the study reported here there is no structural information available on the interaction of natural zinc finger domains with 5'-CNN-3' subsites. Finger 4 of YY1 which contains a DNA recognition helix with the sequence QST-N-LKS (SEQ ID NO: 54) (sequence is given starting from the first residue (-1) proximal to the N-terminus of the α-helix of the protein) that binds to the DNA subsite 5'-CAA-3' in the context of the full-length protein does not directly interact with the 5' cytosine and does not bind this site specifically (22). Crystallographic data and mutagenesis studies concerning the mode of interaction of zinc finger domains of the Cys₂-His₂ family has guided us in the construction of phage display libraries for selection of domains that recognize many DNA subsites (15). The analysis of the Zif268/DNA complex suggests that DNA binding is predominantly achieved by the interaction of amino acid residues of the α-helix in positions -1, 3, and 6 with the 3', middle, and 5' nucleotides of a 3 bp DNA subsite, respectively (11,12). Positions 1, 2, and 5 of the α-helix make direct or water-mediated contacts with the phosphate backbone of the DNA and are important contributors to the ultimate specificity of the protein. Leucine is typically found in position 4 and packs into the hydrophobic core of the domain. Position 2 of the αhelix interacts with other helix residues and, in addition, can make contact with a nucleotide outside the 3 bp subsite resulting in target site overlap (13,15,26-28).

Phage display libraries based on Zif268 were suitable for the selection of domains that bound to 5'-GNN-3' motifs. Due to target site overlap issues wherein some zinc finger domains interact with extended recognition contacts, the selection of 5'-ANN-3' required refinement of the phage display library. This was achieved by replacement of finger-3 of Zif268 containing an Asp at position 2 of the α-helix with a domain lacking residues mediating inter-domain recognition. From this library, we selected domains for the 5'-ANN-3' subsites and further refined or designed novel domains through site-directed mutagenesis (15). Other groups have applied different selection strategies towards the development of zinc finger domains with altered DNA-binding specificity (1-3).

Here we report a selection approach based on the modularity of zinc finger domains to extend the existing set of predefined domains to domains specific to 5'-CNN-3' target sequences. From phage display libraries, eight zinc finger domains specifically recognizing 5'-CNN-3' target sites were selected. Improvement of the DNA-binding specificity for four domains was achieved by site-directed mutagenesis. For six of the 5'-CNN-3' target sites, specific domains were generated by *de novo* design. Resulting proteins were analyzed for DNA-binding specificity. Furthermore, we demonstrate that these domains can be used as modules for the construction of artificial transcription factors with DNA-binding specificity for an 18 bp target site. When fused to the VP64 activation domain or the KRAB repression domain, the 6-finger protein E2S targeted against the 5' UTR of the human *ERBB-2* gene was capable of altering the expression of the endogenous gene. Likewise, a transcriptional activator known as gg1-VP64 was constructed and shown to regulate endogenous expression of human γ-globin. The results reported here provide new insight into zinc finger/DNA recognition of 5'-CNN-3' subsites and significantly extends the repertoire of DNA sequences that can be targeted with designed transcription factors and nucleases.

### Materials and Methods

### Construction of zinc finger library and selection via phage display

Construction of the zinc finger library was based on the earlier described C7 protein (29). Finger-3 recognizing the 5'-GCG-3' subsite was replaced by a domain known to bind to a 5'-GAT-3' subsite (13) via a overlap PCR strategy using a primer coding for finger-3 (5'-GAGGAAGTTTGCCACCAGTGGCAACCTGGTGAGGCATA CCAAAATC-3') (SEQ ID NO: 31) and a pMal-specific primer (5'-GTAAAACGACGGCCAGTGCCAAGC-3') (SEQ ID NO: 32) as previously described (15). Randomization of the zinc finger library by PCR overlap extension was essentially as described (13,29). The library was ligated into the phagemid vector pComb3H (30). Growth and precipitation of phage were performed as previously described (13,31,32). Binding reactions were performed in a volume of 500 µL zinc buffer A (ZBA: 10 mM Tris, pH 7.5/90 mM KCl/1 mM MgCl₂/90 µM ZnCl₂)/0.2% BSA/5 mM DTT/1% Blotto (Biorad)/20 □g double-stranded, sheared herring sperm DNA containing 100 µL precipitated phage (10¹³ colony-forming units). Phage was allowed to bind to non-biotinylated competitor oligonucleotides for 1 hr at 4°C before the biotinylated target oligonucleotide was added. Binding continued 3 hrs or overnight at 4°C. After incubation with 50 µL streptavidin coated magnetic beads (Dynal; blocked with 5% Blotto in ZBA) for 1 hr, beads were washed ten times with 500 µL ZBA/2% Tween 20/5 mM DTT, and once with buffer containing no Tween. Elution of bound phage was performed by incubation in 25 µL trypsin (10 µg/µL) in TBS (50 mM Tris, 150 mM NaCl, pH 7.5) for 30 min at room temperature.

Target and competitor oligonucleotides were designed to form hairpins and had the sequence 5'-GGCCGCN'N'N'ATCGAGTTTTCTCGATNNNGCGGCC-3' (SEQ ID NO: 33) (target oligonucleotides were biotinylated at the 5' end), where NNN represents the finger-2 subsite and N'N'N' represents complementary bases. Target oligonucleotides were usually added at 72 nM in the first three rounds of selection, then decreased to 36 nM and 18 nM in the fifth and sixth rounds. The wild-type competitor hairpin in the initial round of selection had the sequence at NNN equal to 5'-TGG-3' at a concentration of 108 nM and was increased in each round to up to 460 nM in the sixth round. For successive rounds of selection, increasing amounts of an equimolar mixture of 15 hairpin oligonucleotides with all finger-2 5'-CNN-3' subsites, except for the target sequence, were usually added in the first round at a 5 molar excess of target. Mixtures of each finger-2 subsite sequence of the type 5'-ANN-3', 5'-GNN-3', and 5'-TNN-3' were usually added at 1.25 molar excess of target in the first round and was increased to a 10- or even 40-fold excess of the target sequence depending on the experiment. In addition, competitors with NNN of 5'-CGA-3', 5'-CAG-3', 5'-CGG-3' and/or 5'-CTG-3' (if these were not the target sites) were included at concentrations up to 180 nM to enforce selection for specific recognition of the particular target site.

### Multitarget specificity ELISA

The zinc finger-coding sequence was subcloned from pComb3H (30,31) into a modified bacterial expression vector pMal-c2 (New England Biolabs). The zinc finger-maltose-binding protein (MBP) fusions were transformed into XL1-Blue (Stratagene) and expressed after addition of 1 nM isopropyl β-D-thiogalactoside (IPTG). Freeze/thaw extracts of these bacterial cultures or purified proteins were applied in 1:2 dilutions to 96-well plates coated with streptavidin (Pierce) and were tested for DNA-binding specificity against each of the sixteen 5'-GAT CNN GCG-3' (SEQ ID NO: 29) target sites using the hairpin oligonucleotide described above. ELISA (enzyme-linked immunosorbent assay) was performed essentially as described (13,14). After incubation with a mouse anti-MBP (maltose-binding protein) antibody (Sigma, 1:1000), a goat anti-mouse antibody coupled with alkaline phosphatase (Sigma, 1:1000) was applied. Alkaline phosphatase substrate (Sigma) was added and the optical density at 405 nm (OD₄₀₅) was determined with SOFTMAX2.35 (Molecular Devices).

### Gel mobility shift and DNase I footprint analysis

The coding sequence of pE2S was subcloned into a modified pMAL-c2 (New England Biolabs) bacterial expression vector and transformed into *E*. *coli* strain XL-1 Blue (Stratagene). Protein was purified using the Protein Fusion and Purification System (New England Biolabs) with Zinc Buffer A/5 mM DTT as the column buffer. Protein purity was evaluated by Coomassie blue-stained 4-12% Novex gels. Concentration was determined by Bradford assay with bovine serum albumin (BSA) standards. Purified protein was used to perform *DNase I* footprints and gel mobility shift assays to determine the DNA-binding site and affinity.

For *DNase I* footprints a DNA fragment of the human *ERBB-2* promoter was generated by PCR using 5'-³²P-labeled E2SF (5'-GGC TGC TTG AGG AAG TAT AAG AAT GAA GTT GTG AAG C-3') (SEQ ID NO:55) and pGLP2 (5'-CTT TAT GTT TTT GGC GTC TTC CA-3') (SEQ ID NO: 56), Invitrogen) primers from a genomic fragment inserted into pGL3 (33). This DNA fragment contained 267 bp and included region -209 to +3 of the *ERBB-2* promoter. The reaction buffer contained 10 mM Tris-HCl, 10 mM KCl, 10 mM MgCl₂, 5 mM CaCl₂, 10 □M ZnCl₂, pH 7.0. Binding reactions contained 15 kcpm ³²P-end-labeled *ERBB-2* promoter fragment and 5 mM DTT and the protein concentration was varied from 0.1 to 100 nM. Reactions were incubated at 4° C for 12 to 18 hrs. Digestion of DNA was performed using DNase I (Roche Diagnostics) as has been described (34). Samples were separated on a 6% acrylamide/8M urea gel, exposed on phosphoimager plates, and recorded by a Phosphorlmager SI (Molecular Dynamics). Analysis was performed using ImageQuant (Molecular Dynamics) and KaleidaGraph software (Synergy, Reading, PA) to give K_{d} values.

Gel mobility shift analysis was performed with purified protein essentially as described (13).

### Site-directed mutagenesis of finger-2

Finger-2 mutants were constructed by PCR as described (13,14). The library clone containing 5'-TGG-3' finger-2 and 5'-GAT-3' finger-3 was used as PCR template. PCR products containing a mutagenized finger-2 and 5'-GAT-3' finger-3 were subcloned via Nsi I and Spe I restriction sites in frame with finger-1 of C7 into a modified pMal-c2 vector (New England Biolabs).

### Construction of polydactyl zinc finger proteins

Three-finger proteins were constructed by finger-2 stitchery using the SP1C framework as described (33). Six finger proteins were assembled via compatible Xma I and BsrF I restriction sites. Analysis of DNA-binding properties was performed from IPTG-induced freeze/thaw bacterial extracts. For the analysis of capability of these proteins to regulate gene expression they were fused to the activation domain VP64 (the tetrameric repeat of herpes simplex virus' VP16 minimal activation domain) or repression domain KRAB of Kox-1 as described earlier (33,35) and subcloned into the retroviral pMX-IRES-GFP vector (35,36); IRES, internal ribosome-entry site; GFP, green fluorescent protein).

### Retroviral gene targeting and flow cytometric analysis

These assays were performed essentially as previously described (35). For production of recombinant retrovirus, 3.5 x 10⁶ 293GagPol cells were cotransfected with 3.75 µg pMX-IRES encoding each of the zinc finger proteins fused to a regulatory domain and 1.25 µg of pMDG-VSV plasmid using Lipofectamine PLUS reagent (Invitrogen). Viral supernatant was collected two days post-transfection and used to infect 1 x 10⁵ A431 cells. Two days post-infection A431 cells were stained with an ERBB2-specific antibody (5 µg/mL) and analyzed by flow cytometry. As primary antibody, the ERBB-2-specific mAb FSP77 (gift from Nancy E. Hynes; (37) was used. Phycoerythrin labeled donkey F(ab')2 anti-mouse IgG was used as secondary antibody (Jackson Immuno-Research).

### Computer modeling

Computer models were generated using Insight II (Molecular Simulations, Inc.). Models were based on the coordinates of the co-crystal structures of Zif268-DNA (PDB accession 1AAY). The structures were not energy minimized and are presented only to suggest possible interactions. Hydrogen bonds were considered plausible when the distance between the heavy atoms was 3 (± 0.3) Å and the angle formed by the heavy atoms and hydrogen was 120° or greater.

### RESULTS

### Library construction and selection

Previously we reported the isolation by phage display selection and characterization of 16 zinc finger domains that each specifically recognize a 5'-GNN-3' type DNA sequence. The library used for the selection was based on C7, a high-affinity variant of the mouse transcription factor Zif268 (29). Finger-1 of Zif268 has amino acid sequence RSD-E-LTR (SEQ ID NO: 15) (positions -1 through 6) and C7 has the sequence KSA-D-LKR (SEQ ID NO: 44). Finger-2 of both proteins has the sequence RSD-H-LTT (SEQ ID NO: 45) (positions -1 through 6) and finger-3 has the sequence RAD-E-RKR (positions -1 through 6) (SEQ ID NO: 57). Both proteins bind to the DNA target site 5'-GCG-TGG-GCG-3' (SEQ ID NO: 46). Attempts to select zinc finger domains from this library that bound to 5'-A/CNN-3' subsites met with no success due to the dominant cross-subsite interaction of an Asp in position 2 of the recognition helix with either adenine or cytosine on the opposite strand of the following 3 bp subsite (11,12). This interdomain contact has been referred to as target site overlap (13,26-28).

In order to select for zinc finger domains that bound to 5'-CNN-3' subsites, we constructed a phage display library by finger-swapping, as reported earlier (15). Finger-3 of C7 (RSD-E-RKR, positions -1 through 6) (SEQ ID NO: 27) with specificity for the subsite 5'-GCG-3' was exchanged with a domain previously characterized to bind the 5'-GAT-3' subsite (13) generating the 3-finger protein C7.GAT. This recognition helix (TSG-N-LVR) (SEQ ID NO: 58) did not contain Asp at position 2 allowing the selection of zinc finger domains that bound to 5'-ANN-3' or 5'-CNN-3' DNA subsites. Since we had previously argued that recognition of the two target sites, 5'-ACG-3' and 5'-ACT-3', may require aromatic amino acid residues an additional phage display library was constructed using a NNK codon doping strategy (N = adenine, cytosine, guanine, or thymine; K = guanine or thymine). This library included aromatic amino acid residues, but no stop codons. Randomization involved positions -1, 1, 2, 3, 5, and 6 of the α-helix of finger-2 with 32 possibilities for each amino acid position. The library contained 2.4 x 10⁹ members, ensuring representation of almost all amino acid combinations.

Selection of zinc finger-displaying phage was performed using biotinylated hairpin oligonucleotides containing the desired nine bp-binding site. Usually six rounds of panning with each of the sixteen 5'-GAT-CNN-GCG-3' (SEQ ID NO: 29) target oligonucleotides were carried out in the presence of non-biotinylated competitor DNA. Stringency of the selection was increased in each round by decreasing the amount of biotinylated target oligonucleotide and increasing the amounts of the competitor oligonucleotide mixtures. In the sixth round the target concentration was typically 18 nM. The competitor mixtures for 5'-ANN-3', 5'-GNN-3', and 5'-TNN-3' finger-2 subsites were in 5-fold excess for each oligonucleotide pool and the specific 5'-CNN-3' mixture (excluding the target sequence) was present in 10-fold excess of the target. Several specific competitors were added in up to 20-fold excess depending on the target site. The competitor 5'-TGG-3' was used to reduce selection of the wild type protein. The competitors 5'-CAG-3', 5'-CGG-3' and 5'-CGA-3' were added to reduce selection of proteins binding nonspecifically to all 5'-CNN-3' target sites (see below). Phage that bound to the biotinylated target oligonucleotide was recovered by capture with streptavidin-coated magnetic beads.

Clones were sequenced directly after the sixth round of selection or after subcloning into a modified *E. coli* expression vector pMal-c2. The amino acid sequences of selected finger-2 helices were determined by sequence analysis (Fig. 2). For Figure 2, for each target site several clones were usually sequenced after the sixth round of panning. The amino acid sequence for each clone was determined. The finger-2 subsites are shown on the left side and the position within the recognition helix above each set. Amino acids likely to make direct contact with DNA are indicated by bold-face type. Boxed sequences represent clones that bound with the best affinity and specificity profiles. DNA-binding specificity of these three-finger proteins is shown in Fig. 2 upper panel. Numbers at the right of each helix determined represent the number of occurrences of the particular sequence. The * indicates clones identified after subcloning into a modified pMal-c2 expression vector. The # indicates clones selected from the NNK library. Proteins were generally cloned and analyzed in the Zif268 backbone. Some proteins were cloned in the Sp-1 backbone as indicated by the addition of SP1. Proteins were selected that bound with reasonable affinity and specificity to eight of the 16 5'-CNN-3' target sites (Figs. 2 and 3). Attempts to select zinc fingers for the other seven subsites by panning using the C7.GAT NNK library or by addition of specific competitors were not successful.

The amino acids composing the helical recognition domain of finger-2 selected by phage display for the 5'-CNN-3' target sites are shown in Fig. 2. Those amino acid sequences with good specificity are indicated by boxes in Fig. 2. Over 50% of selected helices contained a His at position 3. The helices selected for target sites 5'-CNA-3' and 5'-CNG-3' generally showed good conservation in position -1, consistent with previously observed amino acid residues in these positions (13-15). Position -1 was Gin when the 3' nucleotide was adenine (5'-CGA-3' and 5'-CTA-3'). For 5'-CAA-3', Gln, Asn, or Ser were preferred in position -1, while for 5'-CCA-3' Ser was selected. The interaction of Ser at position -1 with a 3' adenine had previously been observed for the domain binding 5'-ACA-3' QPA-D-LTN) (SEQ ID NO: 59) (15). Panning against finger-2 target sites containing a 3' guanine strongly selected an Arg at position -1, but Asn, Gln, His, Ser, Thr, and Ile were also observed. The domains binding to 5'-CNG-3' subsites often contained Asp at position 2; Asp likely stabilizes the interaction of the 3-finger protein by contacting the cytosine base paired to the 5' guanine of finger-1. For the target sites 5'-CNT-3' Arg, Asn, Gln, His, Ser, Thr, Ala, and Cys were found in position -1 of the recognition helix. For finger-2 subsites containing a 3' cytosine, Gln, Asn, Ser, Gly, His, or Asp were selected in position -1.

For the recognition of 5'-CAN-3', His, Asn, Gly, Val, Pro, Ile, and Lys were selected in position 3 of the recognition helix. Previously, Thr or Asp were found in position 3 of the helix that recognized 5'-GCN-3' (13,15). Here, we selected His, Lys, Arg, Asn, as well as Asp and Thr. For the target site 5'-CCC-3', position 3 was Asn or His. For 5'-CCG-3', position 3 was either Thr or His (Fig. 2). For 5'-GGN-3', His was strongly selected at position 3 consistent with previously observed results (13-15). For the target sites 5'-CGG-3' and 5'-CGT-3', Ser, Asp, Thr, Asn, Gln and Gly were selected at position 3. For 5'-CGC-3' Trp and Thr were selected in some cases. For helices binding to 5'-CTN-3', position 3 was either Ser or Ala. The exceptions were 5'-CTC-3' and 5'-CTT-3' where His was selected. Positions 1, 2, and 5 of the α-recognition helix were variable. This is not surprising because these residues are usually not involved in direct base contacts with DNA (11,12). Position 4 was not randomized, but a spontaneous mutation generated a helix with a change from Leu to Cys at position 4 that bound to 5'-CGT-3' (Fig. 2). This type of spontaneous mutation was observed in rare instances in selection for proteins that bound to 5'-ATC-3' and 5'-GCC-3' target sites (13,15).

Little was known about the recognition of a 5' cytosine by Cys₂₋His₂ zinc finger domains. The recognition of a 5' guanine has been well characterized and is achieved by either Arg or Lys in position 6 of the helix (12,13,19-24). Selection site-directed mutagenesis also suggested that recognition can be achieved by the amino acid in position 6; for recognition of a 5' adenine, the amino acid can be Asn, Ala, Val, Asp, Arg, or Glu (15). By analogy, one could assume that the recognition of 5' cytosine is accomplished by the amino acid residue in position 6 of the α-recognition helix. Phage display selection of domains binding the 5'-CNN-3' finger-2 subsites resulted in the selection of Glu, Asn, Ile, Asp, Ala, Ser, and Val at position 6. Strikingly, Glu was present at position 6 in 65% (82 of 127) of the sequenced proteins (Fig. 2).

### DNA-binding specificity of selected domains recognizing 5'-CNN-3' target sites

To evaluate the DNA-binding specificity of the zinc finger domains selected by phage display, each was tested using multi-target specificity ELISA (Fig. 3) against all 16 sites of the type 5'-GAT-CNN-GCG-3' (SEQ ID NO; 29) (Fig. 3, black bars). In addition, the 5' specificity was analyzed by evaluating the binding to an equimolar mixture of the 16 oligonucleotides for each of the 5'-ANN-3', 5'-CNN-3', 5'-GNN-3', and 5'-TNN-3' target sites (Fig. 3, white bars). In Figure 3, at the top of each graph is the oligonucleotide sequence recognized (pNNN) and the amino acid sequence of the finger-2 domain (positions -1 to 6 with respect to the helix start) of the 3-finger protein analyzed. Black bars represent binding to target oligonucleotides with different finger-2 subsites: 5'-CAA-3', 5'-CAC-3', 5'-CAG-3', 5'-CAT-3', 5'-CCA-3', 5'-CCC-3', 5'-CCG-3', 5'-CCT-3', 5'-CGA-3', 5'-CGC-3', 5'-CGG-3', 5'-CGT-3', 5'-CTA-3', 5'-CTC-3', 5'-CTC-3', 5'-CTG-3'. White bars represent binding to a mixture of the 16 oligonucleotides of each of the subsites 5'-ANN-3', 5'-CNN-3', 5'-GNN-3', and 5'-TNN-3' to evaluate the 5' recognition. The height of each bar represents the relative affinity of the protein for each target, averaged over two independent experiments and normalized to the highest signal among the black or white bars. Error bars represent the deviation from the average. Upper panel: Proteins analyzed correspond to the boxed helix sequences from Fig. 1 obtained from panning. Lower panel: Proteins analyzed were derived by site-directed mutagenesis (pm). Proteins were generally cloned and analyzed in the Zif268 backbone. Some proteins were cloned in the Sp-1 backbone as indicated by the addition of SP1. Results for clones derived from panning are summarized in Fig. 3, upper panel. In general, the selected helices showed exquisite DNA-binding specificity to a 5' cytosine when position 6 of the recognition helix was Glu. The only exception was for target site 5'-CGG-3' (protein sequence RSD-H-LTE) (SEQ ID NO: 14), where binding was tighter to the oligonucleotides with a 5' adenine (Fig. 3g). Clones containing IIe (RAD-H-LAI; Fig. 3a) (SEQ ID NO: 60) or Asp (RSD-H-LTD) (SEQ ID NO: 61); not shown) at position 6, selected for binding to 5'-CAG-3' and 5'-CCG-3', respectively, preferentially bound a 5' guanine. Two of the helices had Ala at position 6. The zinc finger with sequence RND-T-LQA (SEQ ID NO: 62), selected for binding to 5'-CCG-3', showed preferential binding to a 5' guanine (Fig. 3d), while the protein with sequence SRR-T-CRA (SEQ ID NO: 18), selected for binding to 5'-CGT-3', bound with high specificity to a 5' cytosine (Fig. 3h). Previously, an Ala at position 6 had been found to mediate recognition of a 5' adenine (15). These results may indicate that recognition of the 5' nucleotide of the triplet is not only dependent of the amino acid residue in position 6 of the α-helix, but also on the neighboring amino acids, possibly through coordinated interactions.

Recognition of a middle adenine (5'-CAN-3') was observed when the helix contained Asn at position 3, as in binding of 5'-CAG-3' by RAD-N-LAI (SEQ ID NO: 5) (Fig. 3a), consistent with previously reported results (13-15). A middle cytosine (5'-CCN-3') was specifically recognized when position 3 of the helix was His, as in the case of SKK-H-LAE (SEQ ID NO: 63) (Fig. 3b). Asn was also fairly specific for recognition of a middle cytosine (SVR-N-LRE) (SEQ ID NO: 64) (Fig. 3c). Also consistent with previous observations was recognition of 5'-CCG-3' by RND-T-LQA (SEQ ID NO: 65) (Fig. 3d) with Thr at position 3 (13-15). A middle guanine, 5'-CGN-3', was recognized by a His at position 3 as previously reported (13-15). While HTG-H-LLE (SEQ ID NO: 66) (Fig. 3f) showed excellent DNA-binding specificity for 5'-CGC-3', other proteins (QLA-H-LKE (SEQ ID NO: 11) (Fig. 3e), RSD-H-LTE (SEQ ID NO: 67) (Fig. 3g), and QLR-H-LRE (SEQ ID NO: 68) (Fig. 3i) that recognized 5'-CGN-3' have some cross-reactivity to other sites. QLA-H-LKE (SEQ ID NO: 11) (Fig. 3e), is representative of a low affinity and specificity clone. The helix SRR-T-CRA (SEQ ID NO: 69) (Fig. 2h) exhibited specific binding to a middle guanine in 5'-CGT-3' despite the Thr at position 3. A middle thymine was recognized specifically by the helices QRH-S-LTE (SEQ ID NO: 70) (Fig. 3j) and RND-A-LTE (SEQ ID NO: 71) (Fig. 3k) containing either Ser or Ala at position 3, consistent with previous findings (13-15).

Recognition of a 3' adenine was observed for the helices containing Gln at position -1 (QLA-H-LKE, (SEQ ID NO: 11) (Fig. 3e); QRH-S-LTE, (SEQ ID NO: 70) (Fig. 3j) as reported earlier (13-15). For the recognition of a 3' cytosine, Ser and His were observed at position -1 (HTG-H-LLE (SEQ ID NO: 66) (Fig. 2f); SKK-H-LAE (SEQ ID NO: 63) (Fig. 3b); and SVR-N-LRE, (SEQ ID NO: 64) (Fig. 3c). Recognition of 3' guanine was achieved by an Arg at position -1 (Fig. 3a, 3d, 3g, and 3k). This data is consistent with previous reports (13-15). Recognition of a 3' thymine was reported to be mediated by Ser, Thr, or His in position -1 (13-15). In this study, helices that recognize 3' thymine had Ser (SRR-T-CRA (SEQ ID NO: 69) (Fig. 3h) or Gln (QLR-H-LRE (SEQ ID NO: 68) (Fig. 2i) in position -1.

In summary, phage display selection yielded domains for eight 5'-CNN-3' target sites, including 5'-CAG-3', 5'-CCC-3', 5'-CCG-3', 5'-CGC-3', 5'-CGG-3', 5'-CGT-3', 5'-CTA-3', and 5'-CTG-3'. For the other target sites no domains with reasonable specificity resulted from panning. For 5'-CAG-3' (Fig. 2a) and 5'-CCG-3' (Fig. 2d) improvement of specificity in the binding of the 5' cytosine was necessary. The helix binding to 5'-CGA-3' (Fig. 2e) showed little specificity and required improvement by site-directed mutagenesis. For domains binding the 5'-CNG-3' type of DNA sequences specificity for the middle nucleotide was usually insufficient. This phenomenon we have previously described also for domains binding to 5'-GNG-3' and 5'-ANG-3' type of DNA sequences (13-15).

### Improvement of the specificity of domains binding to the 5'-CNN-3' family of sequences by site-directed mutagenesis and de novo design

Phage display selections did not generate zinc finger domains that bound specifically to 5'-CAA-3', 5'-CAC-3', 5'-CAT-3', 5'-CCA-3', 5'-CCT-3', 5'-CGA-3', 5'-CTC-3', or 5'-CTT-3'. In some cases selected helices bound their cognate target site, but with some cross-reactivity to other sites (for example 5'-CAG-3', Fig. 3a; 5'-CCG-3', Fig. 3d; 5'-CGA-3', Fig. 3e; 5'-CGG-3', Fig. 3g). Site-directed mutagenesis was used to improve DNA-binding specificity. Results are shown in Fig. 3, lower panel. The DNA-binding motif Leu-Thr-Glu in positions 4, 5, and 6 of the α-helix was found in numerous sequences selected during panning to mediate 5' cytosine recognition. The helix selected to recognize 5'-CAG-3' (RAD-N-LAI (SEQ ID NO: 60) (Fig. 3a) was changed to this consensus sequence (RAD-N-LTE) (SEQ ID NO: 72). DNA-binding specificity was greatly improved (Fig. 3n) not only for 5' cytosine, but also for the middle adenine. Use of the Leu-Thr-Glu motif also improved binding to 5'-CCG-3' (compare RND-T-LQA (SEQ ID NO: 62) (Fig. 3d) to RND-T-LTE (SEQ ID NO: 73) (Fig. 3q). In an attempt to improve 5' cytosine recognition in the target site 5'-CGG-3', His at position 3 was replaced with a Lys (RSD-H-LTE (SEQ ID NO: 14) (Fig. 3g) vs. RSD-K-LTE (SEQ ID NO: 74) (Fig. 3t). This did not improve 5' cytosine recognition, but did result in exclusive recognition of the middle guanine. The amino acid sequence selected for recognition of 5'-CGA-3' was changed from QIA-H-LKE (SEQ ID NO: 11) (Fig. 3e) to QSG-H-LTE (SEQ ID NO: 75) based on a sequence from Segal et al., 1999 (QSG-D-LRR) (SEQ ID NO: 76). This improved DNA-binding specificity and affinity but the net specificity remained lower than most other domains (Fig. 3s).

Rational design was also applied to generate a domain for recognition of 5'-CAA-3'. The finger-2 helix QSG-N-LTE (SEQ ID NO: 2) (derived from a zinc finger that recognized 5'-GCA-3', QSG-D-LRR (SEQ ID NO: 76) (13) bound its target site with good specificity (Fig. 3l). The helix SKK-A-LTE (SECT ID NO: 77) bound preferentially to its target site 5'-CAC-3' with excellent 5' cytosine recognition (Fig. 3m), but weak middle base specificity. For the recognition of 5'-CAT-3', the helix TSG-N-LTE (SEQ ID NO: 78) was generated based on a helix that recognized 5'-GAT-3' (TSG⁶LVR) (SEQ ID NO: 28) (13). Multitarget ELISA showed that this helix bound preferentially to its target site with excellent recognition of the 5' cytosine (Fig. 3o). Changing the 5'-CTA-3' helix of QRH-S-LTE (Fig. 3j) (SEQ ID NO: 70) to QNS-T-LTE (SEQ ID NO: 79) (Fig. 3u) reduced the nonspecific binding to other targets. With the exception of 5'-CCC-3' (Fig. 2b), specific helices that targeted sites containing only pyrimidine nucleotides were not generated from phage display. The helix TSH-S-LTE (SEQ ID NO: 80) was designed to bind the subsite 5'-CTC-3', but surprisingly bound preferentially to 5'-CCA-3' with excellent 5' cytosine recognition (Fig. 3p). This data then allowed us to design a highly specific recognition domain for 5'-CCT-3', TKN-S-LTE (SEQ ID NO: 81) (Fig. 3r) and the helix TTG-A-LTE (SEQ ID NO: 82) for 5'-CTT-3' (Fig. 3v). For the subsite 5'-CTC-3' no specific binder could be identified following testing of multiple helices.

### Generation of polydactyl zinc finger proteins containing 5'-CNN' domains

We have previously demonstrated that exogenous and endogenous genes can be regulated with 6-finger proteins containing zinc finger domains specifically recognizing 5'-(GNN)₆-3' DNA sequences (33,35,36). In addition, we showed that 6-finger proteins containing varying numbers of domains recognizing 5'-GNN-3', 5'-ANN-3', and 5'-TNN-3' target sites were capable of endogenous gene regulation (15). To investigate whether the domains described here that recognize the 5'-CNN-3' family of DNA sequences are suitable for the construction of artificial transcription factors, the 6-finger protein pE2S was constructed. We chose the human *ERBB-2* gene as model system since we have previously reported specific gene regulation with the 6-finger proteins E2C and E2X targeted against the 5' UTR of the *ERBB-2* gene (15,35). The 6-finger pE2S was designed to bind the target site 5'-CGG-GGG-GCT-CCC-CTG-GTT-3' (SEQ ID NO: 83) at position -137 to -154 within the 5' UTR (38). This target site contains recognition sites for three 5'-GNN-3' domains previously identified (13) and three 5'-CNN-3' domains described here (indicated by underlining).

Two 3 finger-coding regions were generated using a rapid PCR overlap extension method and the Sp1C framework (33). These 3-finger proteins were then fused to create a 6-finger protein that was cloned into the bacterial expression vector pMal-c2. This 6-finger protein was expressed in *E. coli* as MPB fusion proteins and was purified. The affinity of purified pE2S was measured using an electrophoretic mobility shift assay (EMSA) and a dissociation constant of 3.25 nM was determined. Characterization of the binding specificity of the six finger protein E2S for its DNA target site within a 267 bp fragment of the *ERBB-2* promoter containing the pE2S binding site was determined using DNase I footprinting (Fig. 4). E2S protein was titrated over a range of 100 nM to 0.1 nM to provide a dissociation constant. In Figure 4, the six-finger protein pE2S was constructed using the SP1C framework. After bacterial expression and purification pE2S was incubated with a 267 bp radioactively-labeled DNA fragment containing region -209 to +3 of the *ERBB-2* promoter. After DNase I digestion, samples were separated on a 6% acrylamide/8 M urea gel. Control: DNA fragment without digestion; G+A ladder: sequencing ladder of the 267 bp DNA fragment; DNase I: digestion of the labeled fragment without E2S protein; Lanes 4-13 contained 100 nM, 50 nM, 20 nM, 10 nM, 5 nM, 2 nM, 1 nM, 0.5 nM, 0.2 nM and 0.1 nM E2S protein. The location of the E2S binding site is indicated. The average of three independent experiments produced a K_{d} value of 14 ± 4 nM, consistent with affinity data derived from EMSA. DNA binding of pE2S was observed precisely at the 18 bp target site 5'-CGG-GGG-GCT-CCC-CTG-GTT-3' (SEQ ID NO: 83). This result shows that the 5'-CNN-3' domains characterized here also promote highly specific binding in the context of a 6-finger protein.

To investigate the potential of the 6-finger protein pE2S to alter expression of the endogenous *ERBB-2* gene, their coding regions were subcloned into the retroviral vector pMX-IRES and fused to either the VP64 activation domain or the KRAB repression domain of Kox-1 (33,39). The human carcinoma cell line A431 was infected with the recombinant retrovirus. Two days after infection, cells were analyzed by flow cytometry using an ErbB-2-specific antibody (Fig. 5). In Figure 5, A431 cells were transduced with retrovirus encoding transcription factors consisting of either pE2S fused to the activation domain VP64 or pE2S fused to the repressor domain KRAB. Three days later, intact cells were stained with ErbB2-specific mAb FSP77 in combination with phycoerythrin-labeled secondary antibody and analyzed by flow cytometry. Thin line, staining with secondary antibody alone; stippled line, specific staining of mock-transduced cells; solid lines, specific staining of cells transduced to express the transcription factors E2S-VP64 (A) or E2S-KRAB (B). Strong up-regulation of the endogenous *ERBB-2* gene was observed with pE2S fused to the VP64 activation domain (Fig. 5A) and down-regulation was observed when pE2S fused to the KRAB repression domain was expressed (Fig. 5B). The result for pE2S clearly demonstrates that the 5'-CNN-3' domains described here can serve as modular building blocks for the construction of designed transcriptional regulators.

Likewise, we have recently reported the construction of the γ-globin targeting protein gg1 that binds the sequence 5'-GTC AAG GCA AGG CTG GCC-3' (SEQ ID NO: 84) with a 0.7 nM dissociation constant (40). This protein was shown by DNase I footprinting and chromatin immunoprecipitation to bind the targeted sequence *in vitro* and *in vivo* and transcription factors based on this protein were robust regulators of the endogenous gene. These two cases indicate that the domains selected here are readily combined with the variety of other domains we have reported.

### DISCUSSION

Zinc finger domains of the type Cys₂-His₂ have proven to be versatile tools for the targeted regulation of endogenous genes and more recently as nuclease targeting domains to facilitate gene repair through homologous recombination. Recognition of a 3 bp DNA sequence by a zinc finger is predominantly achieved by the interaction of amino acid residues of the α-helix in positions -1, 3, and 6 with the 3', middle, and 5' nucleotides, respectively, while amino acids at other positions within the helix are key to providing elements of fine specificity. Position 2 of the α-helix interacts with other helix residues and, in addition, can make contact with a nucleotide outside the 3 bp subsite (11,12,41). This target site overlap is a limitation of the modular approach to generation of transcription factors, but this influence seems to be restricted only to inter-domain interactions if position 2 of the α-recognition helix is Asp (41,42).

In the present study we describe the generation of zinc finger domains binding 5'-CNN-3' subsites by selection from a phage display library based on the 3 finger protein C7.GAT and refinement and or creation through rational design. This C7.GAT library was constructed with a finger-3 that did not contain an Asp at position 2 to enable the selection of zinc finger domains recognizing finger-2 subsites containing a 5' adenine or 5' cytosine. Phage display selections from this library for domains binding to the 5'-CNN-3' type of DNA sequences was not as successful as for selection of 5'-ANN-3' domains (15). Selections for target sites containing a 5' pyrimidine (cytosine or thymine) may be more difficult since pyrimidines are not as accessible to the side chains of the recognition helix and offer fewer opportunities for hydrogen bonding.

Zinc finger domains that specifically recognized eight of the 16 possible 5'-CNN-3' target sites were selected from phage display (Fig. 2). These included five domains with excellent DNA-binding specificity with target sites 5'-CCC-3', 5'-CGC-3', 5'-CGT-3', 5'-CTA-3', and 5'-CTG-3' (Fig. 3b, f, h, j, and k) and three domains with good specificity that bound 5'-CAG-3', 5'-CCG-3', and 5'-CGG-3' (Fig. 3a, d, and g). DNA-binding specificity for target sites 5'-CAG-3', 5'-CCG-3', 5'-CGA-3', and 5'-CTA-3' were improved by applying rational design to amino acid sequences obtained from phage display (Fig. 3n, q, s, and u). For the target sites 5'-CAA-3', 5'-CAC-3', 5'-CAT-3', 5'-CCA-3', 5'-CCT-3', and 5'-CTT-3' zinc finger domains with reasonable to excellent DNA-binding specificity were obtained through *de novo* design (Fig. 3l, m, o, p, r, and v). Despite extensive analysis, we could not identify a zinc finger domain to recognize 5'-CTC-3' with specificity. The most optimal zinc finger domains and their recognition sites are summarized in Table 3.

**TABLE 3**

| Target Site 5' →3' | Finger 2 Helix | Source | Reference to Figure |
|---|---|---|---|
| **CAA** | QSG-N-LTE (SEQ ID NO: 2) | M | 3l |
| **CAC** | SKK-A-LTE (SEQ ID NO: 77) | M | 3m |
| **CAG** | RAD-N-LTE (SEQ ID NO: 72) | M | 3n |
| **CAT** | TSG-N-LTE (SEQ ID NO: 78) | M | 3o |
| **CCA** | TSH-S-LTE (SEQ ID NO:80) | M | 3p |
| **CCC** | SKK-H-LAE (SEQ ID NO:63) | P | 3b |
| **CCG** | RND-T-LTE (SEQ ID NO:73) | M | 3q |
| **CCT** | TKN-S-LTE (SEQ ID NO:81) | M | 3r |
| **CGA** | QSG-H-LTE (SEQ ID NO:75) | M | 3i |
| **CGC** | HIG-H- LLE (SEQ ID NO:66) | P | 3f |
| **CGG** | RSD-H-LTE (SEQ ID NO:14) | P | 3g |
| **CGT** | SRR-T-CRA (SEQ ID NO: 18) | P | 3h |
| **CTA** | QNS-T-LTE (SEQ ID NO:79) | M | 3u |
| **CTC** | none | | |
| **CTG** | RND-A-LTE (SEQ ID NO: 71) | P | 3k |
| **CTT** | TTG-A-LTE (SEQ ID NO: 82) | M | 3v |

Nonspecific zinc fingers were also noted in our selections. The same three helices, QRH-H-LVE (SEQ ID NO: 85), SPG-H-LIE (SEQ ID NO: 86), and SCS-H-LAE (SECT ID NO: 87), were obtained in selections against a number of the target sites 5'-CAA-3', 5'-CAC-3', 5'-CAT-3', 5'-CCA-3', 5'-CCT-3', 5'-CTC-3', and 5'-CTT-3' (Fig. 2). For these helices no specific target sites were identified. The three helices appear to bind DNA with high affinity, but low specificity.

In general, the recognition of the middle and 3' nucleotide of the 5'-CNN-3' subsite by zinc finger domains was consistent with previous observations (13-15). Middle adenines (5'-CAN-3') are predominantly recognized by Asn at position 3, with the exception of 5'-CAC-3' (SKK-A-LTE (SEQ ID NO: 77) (Fig. 3m). Middle guanine was recognized by His at position 3, with the exception of 5'-CGT-3' (SRR-T-CRA (SEQ ID NO: 18) (Fig. 3h) and middle thymine was recognized by either Ser or Ala at position 3. Interestingly, the recognition of middle cytosine was not achieved by Thr, Asp, or Glu at position 3 as previously reported, but by Ser or His (the exception was 5'-CCG-3', RND-T-LTE (SEQ ID NO: 73) (Fig. 3q). The recognition of the 3' nucleotide of the target was mediated by the amino acid at position -1: 3' adenine was recognized by Gln (the exception was 5'-CCA-3', TSH-S-LTE (SEQ ID NO: 80) (Fig. 3p), 3' guanine by Arg, and 3' thymine by Thr or Ser. Unusual amino acid residues were found in position -1 for domains that recognized a 3' cytosine, e.g., Asn, Ser, Thr and His. Previously only Asp and Glu were found to mediate specific recognition of a 3' cytosine (13-15).

Many of the selected zinc finger domains had Glu in position 6 of the α-recognition helix (Fig. 2). Although it was tempting to assume that this Glu mediated recognition of the 5' cytosine, analysis of the DNA-binding specificity of the selected helices showed that recognition of the cytosine was more complex and dependent on other positions with the helix. Excellent discrimination of the 5' cytosine was observed for eight domains with Glu in position 6 for (Fig. 3b, c, e, f, h, i, j, and k) but RSD-H-LTE (SEQ ID NO: 14) (Fig. 3g) was an exception. The most frequent motif selected at positions 5 and 6 was TE. When this motif was used for the rational design of zinc finger domains, domains showed excellent discrimination of a 5' cytosine (Fig. 3l-v), with the exceptions of QSG-N-LTE (SEQ ID NO: 2) and RSD-K-LTE (SEQ ID NO: 74) (Fig. 3s, t).

Molecular modeling was performed to gain insights into how the changes in the zinc finger recognition helix produced the observed DNA-binding specificity. Of primary interest was how Glu at position 6 mediated recognition of 5' cytosine. The helix RSD-H-LTE (SEQ ID NO: 14), which recognized 5'-CGG-3' (Fig. 3g), was modeled (Fig. 6B) taking advantage of sequence similarity with finger-2 of the well-characterized Zif268 protein, RSD-H-LTT (SEQ ID NO: 45) (Fig. 6A). The structure of Zif268 bound to its operator DNA (11) revealed several amino acid:base interactions relevant to the current study. The finger-2 subsite in the Zif268 structure is 5'-TGG-3'. Recognition of 3' guanine was accomplished by two H-bonds from Arg at position -1. The Arg side chain was conformationally constrained by two buttressing H-bonds to the Asp in position 2 (Fig. 6A). Recognition of the middle guanine was accomplished by an H-bond from His at position 3. This His stacked against the 5' thymine, an interaction thought to limit the conformational flexibility of the His and thus enhance its specificity for middle guanine. The Thr at position 6 made no hydrogen bonds with the 5' thymine. The specificity for 5' thymine was achieved though a stacking interaction between the His at position 3 and the thymine and a hydrogen bond between the adenine on the opposite strand and an Asp at position 2 in neighboring finger-3 recognition helix (not shown in Fig. 6A). This target site overlap was discussed above. In Figure 6, select oxygen (red), nitrogen (blue), and phosphate (purple) atoms are colored for clarity. Green dotted lines indicate suggested hydrogen bonds. The sequence of each helix, the DNA subsite, and proposed interactions are summarized below each model. Green lines indicate hydrogen bonds. Arrows indicate hydrogen acceptors. A, finger-2 of Zif268; B, pCGG (Fig. 3g); C, pmCAG (Fig. 3n).

It seems reasonable, *a priori,* to expect that the finger-2 helix RSD-H-LTE (SEQ ID NO: 14) will bind 5'-CGG-3' in much the same way as the Zif268 finger-2 helix RSD-H-LTT (SEQ ID NO: 45) binds 5'-TGG-3'. Substituting the appropriate amino acids and bases onto the coordinates of the Zif268 produced the model for RSD-H-LTE (SEQ ID NO: 14) shown in Fig. 6B. This model places the ε oxygens of Glu at position 6 within H-bonding distance (3 ± 0.5Å) of N4 on the 5' cytosine, providing a reasonable explanation for specificity of Glu for this base. However, the multi-target ELISA data demonstrated that RSD-H-LTE (SEQ ID NO: 14) actually bound preferentially to a 5' adenine rather than a 5' cytosine (Fig. 3g). A possible explanation for the poor specificity of 5' cytosine by RSD-H-LTE (SEQ ID NO: 14) could be a stacking interaction between His at position 3 and the 5' cytosine, similar to the His-5' thymine interaction observed in finger-2 of Zif268. Such an interaction might position the 5' cytosine in a manner that does not favor a hydrogen bond with the position 6 Glu. The phage selection data also supports a potential interaction between His and 5' cytosine (Fig. 2). His appeared at position 3 in 56% of selected zinc finger domains targeting 5'-CGN-3', which might be expected due to its established role in the recognition of middle-guanine. However, it also appeared in 51% of cases overall (42 of 82 sequences), including most of the domains binding to 5'-CNA-3' (with the exception of 5'-CTA-3'), 5'-CNC-3', and 5'-CNT-3' (Fig. 2). Therefore, the presence of 5' cytosine in the target site might have biased the selection of His as the position 3 residue. Other positions in the recognition helix may also affect specificity for the 5' nucleotide. QSG-H-LTE (SEQ ID NO: 75) displayed poor 5' specificity (Fig. 3s), but SKK-H-LAE (SEQ ID NO: 63) and HTG-H-LLE (SEQ ID NO: 66) both strongly specified 5' cytosine (Fig. 3b and 3f). Unfortunately, any models based on such helices would be highly speculative due to the lack of sufficient structural data of related sequences.

A second interesting case to consider is that of RAD-N-LTE (SEQ ID NO: 72), which recognizes 5'-CAG-3' (Fig. 6C). The primary difference between this helix and RSD-H-LTE (SEQ ID NO: 14) is the Asn in position 3. This helix achieves excellent specificity for 5' cytosine (Fig. 3n). To investigate the basis for the 5' specificity, the appropriate amino acids and bases were substituted onto the coordinates of the structure of finger-2 of Zif268. These simple substitutions placed the δO and δN of the position 3 Asn well beyond hydrogen-bonding distance (≈4.5 Å) with N6 and N7 of the adenine. However, previous structural data as well as the excellent specificity of this domain for a middle adenine suggests that a hydrogen bond must be present. In principle, this could be accomplished either by repositioning of the adenine or by a rotation of α-recognition helix that would bring Asp closer to the DNA. Both conformational changes are observed in the structure of finger-2 of the protein Tramtrak, RKD-N-MTA (SEQ ID NO: 88), bound to 5'-AAG-3' (21). This conformational change would also be expected to bring the Gin at position 6 closer to 5' cytosine, potentially facilitating an interaction that would produce the observed excellent specificity for 5' cytosine. It should be noted that Ala at position 6 in finger-2 of the Tramtrak protein does not contact the 5' base of the target, and modeling RAD-N-LTE (SEQ ID NO: 72) on the coordinates of the Tramtrak helix provided little additional insight (data not shown).

Due to lack of existing structural data, the specificity of 5' cytosine by many of the zinc finger helices described in this study cannot easily be rationalized by computer modeling. In structures where recognition of 5' adenine, thymine, or cytosine can be rationalized, the specificity has been the result of a target site overlap (11,42). We expect no target site overlap interactions from the finger-3 helix used in this study as 5' cytosine recognition was variable despite the presence of a common finger-3 for all proteins in this study. It is clear that more structural studies will be required to understand the parameters involved in DNA base positioning and the docking orientation of the α-helix with respect to the DNA.

Although we do not yet have a detailed understanding of the molecular interactions that underlie the specificity of the zinc finger domains described here for their 5'-CNN-3' target sites, their use in artificial transcription factors has been demonstrated. We have constructed the 6-finger protein pE2S containing three 5'-CNN-3' recognition helices that targets the 18 bp sequence 5'-CGG-GGG-GCT-CCC-CTG-GTT-3' (SEQ ID NO: 83) within the 5' UTR of the *ERBB-*2 gene. Binding of pE2S was specific for this 18 bp recognition site as demonstrated by DNase I footprint analysis (Fig. 4). Furthermore, we have demonstrated that pE2S was capable of up- and down-regulation of the endogenous *ERBB*-2 when fused to either the activation domain VP64 or the repression domain KRAB, respectively (Fig. 5). Recently we also described a transcription factor designed to upregulate then γ-globin gene in a strategy aimed at treating sickle cell anemia (40). In this report the transcription factor gg1 was designed to bind the sequence 5'-GTC AAG GCA AGG CTG GCC-3' (SEQ ID NO: 84), incorporating the 5'-CTG-3' domain together with 5'-ANN-3' and 5'-GNN-3' domains. This transcription factor was also shown to bind its target sequence with high affinity and specificity *in vitro* and *in vivo* and regulate the endogenous gene.

In summary, the zinc finger domains described here that recognize 5'-CNN-3' DNA subsites are suitable for the rapid construction of artificial transcription factors. These fifteen 5'-CNN-3' zinc finger domains augment the sixteen 5'-GNN-3', fourteen 5'-ANN-3', and two 5'-TNN-3' domains we have previously developed and together provide for the rapid construction of over 10 billion proteins that bind 5'-[(G/A/C)NN]₆-3' sites. Sites of this type occur approximately once every 6 nucleotide in random sequence. Therefore, the predefined domains disclosed here significantly increase the number of DNA sequences that can be rapidly targeted by artificial transcription factors and nucleases.

References for Example 13

The references for Example 13 are provided and listed by number for convenience. These references are incorporated herein by this reference.

### REFERENCES

1. Blancafort, P., Segal, D. J., and Barbas, C. F., 3rd. (2004) Mol Pharmacol 66(6), 1361-1371
2. Beerli, R. R., and Barbas, C. F., 3rd. (2002) Nat Biotechnol 20(2), 135-141
3. Jantz, D., and Berg, J. M. (2004) Chem Rev. 104(2), 789-799
4. Zhang, L., Spratt, S. K., Liu, Q., Johnstone, B., Qi, H., Raschke, E. E., Jamieson, A. C., Rebar, E. J., Wolffe, A. P., and Case, C. C. (2000) J Biol Chem 275(43), 33850-33860
5. Liu, P. Q., Rebar, E. J., Zhang, L., Liu, Q., Jamieson, A. C., Liang, Y., Qi, H., Li, P. X., Chen, B., Mendel, M. C., Zhong, X., Lee, Y. L., Eisenberg, S. P., Spratt, S. K., Case, C. C., and Wolffe, A. P. (2001) J Biol Chem 276(14), 11323-11334
6. Blancafort, P., Magnenat, L., and Barbas, C. F., 3rd. (2003) Nat Biotechnol 21 (3), 269-274
7. Umov FD, M. J., Lee YL, Beausejour CM, Rock JM, Augustus S, Jamieson AC, Porteus MH, Gregory PD, Holmes MC. (2005) Nature 435(7042), 646-651
8. Bibikova, M., Beumer, K., Trautman, J. K., and Carroll, D. (2003) Science 300(5620), 764
9. Miller, J., McLachlan, A. D., and Klug, A. (1985) EMBO J. 4(6), 1609-1614
10. Lee, M. S., Gippert, G. P., Soman, K. V., Case, D. A., and Wright, P. E. (1989) Science (Washington, D. C., 1883-) 245(4918), 635-637
11. Pavletich, N. P., and Pabo, C. O. (1991) Science (Washington, D. C., 1883-) 252(5007), 809-817
12. Elrod-Erickson, M., Rould, M. A., Nekludova, L., and Pabo, C. O. (1996) Structure 4, 1171-1180
13. Segal, D. J., Dreier, B., Beerli, R. R., and Barbas, C. F., 3rd. (1999) Proc Natl Acad Sci USA 96(6), 2758-2763
14. Dreier, B., Segal, D. J., and Barbas, C. F., 3rd. (2000) J Mol Biol 303(4), 489-502
15. Dreier, B., Beerli, R. R., Segal, D. J., Flippin, J. D., and Barbas, C. F., 3rd. (2001) J Biol Chem 276(31), 29466-29478
16. Guan, X., Stege, J., Kim, M., Dahmani, Z., Fan, N., Heifetz, P., Barbas, C. F., 3rd, and Briggs, S. P. (2002) Proc Natl Acad Sci U S A 99(20), 13296-13301
17. Tan, S., Guschin, D., Davalos, A., Lee, Y. L., Snowden, A. W., Jouvenot, Y., Zhang, H. S., Howes, K., McNamara, A. R., Lai, A., Ullman, C., Reynolds, L., Moore, M., Isalan, M., Berg, L. P., Campos, B., Qi, H., Spratt, S. K., Case, C. C., Pabo, C. O., Campisi, J., and Gregory, P. D. (2003) Proc. Natl. Acad Sci., U S A. 100(21), 11997-12002
18. Beerli, R. R., Schopfer, U., Dreier, B., and Barbas, C. F., 3rd. (2000) J Biol Chem 275(42), 32617-32627
19. Pavletich, N. P., and Pabo, C. O. (1993) Science (Washington, D. C., 1883-) 261(5129), 1701-1707
20. Kim, C. A., and Berg, J. M. (1996) Nature Structural Biology 3, 940-945
21. Fairall, L., Schwabe, J. W. R., Chapman, L., Finch, J. T., and Rhodes, D. (1993) Nature (London) 366(6454), 483-487
22. Houbaviy, H. B., Usheva, A., Shenk, T., and Burley, S. K. (1996) Proc. Natl. Acad. Sci. U. S. A. 93(24), 13577-13582
23. Wuttke, D. S., Foster, M. P., Case, D. A., Gottesfeld, J. M., and Wright, P. E. (1997) J. Mol. Biol. 273(1),183-206
24. Nolte, R. T., Conlin, R. M., Harrison, S. C., and Brown, R. S. (1998) Proc. Natl. Acad. Sci. U. S. A. 95(6), 2938-2943
25. Havranek JJ, D. C., Baker D. (2004) J Mol Biol. 344(1), 59-70
26. Wolfe SA, G. H., Ramm EI, Pabo CO. (1999) J Mol Biol. 285(5), 1917-1934.
27. Isalan, M., Choo, Y., and Klug, A. (1997) Proc. Natl. Acad. Sci. U. S. A. 94(11),5617-5621
28. Pabo C.O., Nekludova, L.. (2000) J Mol Biol. 301(3), 597-624
29. Wu, H., Yang, W.-P., and Barbas, C. F., III. (1995) Proc. Natl. Acad. Sci. U. S. A. 92(2), 344-348
30. Rader, C., and Barbas, C. F., III. (1997) Curr. Opin. Biotechnol. 8(4), 503-508
31. Barbas, C. F., III, Kang, A. S., Lemer, R. A., and Benkovic, S. J. (1991) Proc. Natl. Acad. Sci. U. S. A. 88(18), 7978-7982
32. Barbas, C. F., III, and Lerner, R. A. (1991) Methods (San Diego) 2(2), 119-124
33. Beerli, R. R., Segal, D. J., Dreier, B., and Barbas, C. F., 3rd. (1998) Proc Natl Acad Sci U S A 95(25), 14628-14633
34. Trauger, J.W., Dervan, P.B. (2001) Methods Enzymol. 340, 450-466
35. Beerli, R. R., Dreier, B., and Barbas, C. F., 3rd. (2000) Proc Natl Acad Sci U S A 97(4), 1495-1500
36. Liu, Q., Segal, D. J., Ghiara, J. B., and Barbas, C. F., III. (1997) Proc. Natl. Acad. Sci. U. S. A. 94(11), 5525-5530
37. Harwerth IM, W. W., Marte BM, Hynes NE. (1992) J Biol Chem. 267(21), 15160-15167
38. Hudson, L. G., Ertl, A. P., and Gill, G. N. (1990) J. Biol. Chem. 265(8), 4389-4393
39. Margolin, J. F., Friedman, J. R., Meyer, W. K.-H., Vissing, H., Thiesen, H.-J., and Rauscher, F. J., III. (1994) Proc. Natl. Acad. Sci. U. S. A. 91(10), 4509-4513
40. Graslund, T., Li, X., Magnenat, L., Popkov, M., and Barbas, C. F., 3rd. (2005) J Biol Chem 280(5), 3707-3714
41. Segal, D. J., Beerli, R. R., Blancafort, P., Dreier, B., Effertz, K., Huber, A., Koksch, B., Lund, C. V., Magnenat, L., Valente, D., and Barbas, C. F., 3rd. (2003) Biochemistry 42(7), 2137-2148
42. Wolfe SA, N. L., Pabo CO. (2000) Annu Rev Biophys Biomol Struct. 29, 183-212.

### EXAMPLE 14

### Construction of Additional Zinc Fingers

Additional zinc fingers were constructed as in Example 13, using various backbones.

### 1. TSG-N-LTE (SEQ ID NO: 78)

A zinc finger using TSG-N-LTE (SEQ ID NO: 78) was constructed in a Zif268 backbone. The binding capacity was analyzed using crude lysate by ELISA as in Example 13. The zinc finger bound CAT and CTT with more affinity for CAT.

Additionally, another zinc finger using TSG-N-LTE (SEQ ID NO: 78) was constructed in a SP1C backbone. The binding capacity was analyzed using crude lysate by ELISA. The zinc finger bound CAT.

### 2. TKN-S-LTE (SEQ ID NO: 81)

A zinc finger using TKN-S-LTE (SEQ ID NO: 81) was constructed in a SP1C backbone. The binding capacity was analyzed using crude lysate by ELISA. The zinc finger bound CCT.

### 3. QNS-T-LTE (SEQ ID NO: 79)

A zinc finger using QNS-T-LTE (SEQ ID NO: 79) was constructed in a Zif268 backbone. The binding capacity was analyzed using crude lysate by ELISA. The zinc finger bound CTA with great specificity.

### 4. NLQ-H-LGE (SEQ ID NO: 3)

A zinc finger using NLQ-H-LGE (SEQ ID NO: 3) was constructed in a Zif268 backbone. The binding capacity was analyzed using crude lysate by ELISA. The zinc finger bound CAC and CAT, with some cross-reactivity.

### 5. QSG-N-LTE (SEQ ID NO: 2)

A zinc finger using QSG-N-LTE (SEQ ID NO: 2) was constructed in a Zif268 backbone. The binding capacity was analyzed using crude lysate by ELISA. The zinc finger bound CAC and CAT, with some cross-reactivity.

### 6. QRH-N-LTE (SEQ ID NO: 1)

A zinc finger using QRH-N-LTE (SEQ ID NO: 1) was constructed in a Zif268 backbone. The binding capacity was analyzed using crude lysate by ELISA. The zinc finger bound 14 of the possible CNN triplets, with little binding of CTG and CTT.

### 7. RND-T-LTE (SEQ ID NO: 8)

A zinc finger using RND-T-LTE (SEQ ID NO: 8) was constructed in a Zif268 backbone. The binding capacity was analyzed using crude lysate by ELISA. The zinc finger bound CAG, CCG, CGG, and CTG.

### 8. RND-T-LQA (SEQ ID NO: 62)

A zinc finger using RND-T-LQA (SEQ ID NO: 62) was constructed in a Zif268 backbone. The binding capacity was analyzed using crude lysate by ELISA. The zinc finger bound CCG with high specificity.

### 9. QLA-H-LKE (SEQ ID NO: 11)

A zinc finger using QLA-H-LKE (SEQ ID NO: 11) was constructed in a Zif268 backbone. The binding capacity was analyzed using crude lysate by ELISA. The zinc finger bound CAT and CGA, but cross-reacted with most of the other triplets except CTT.

### 10. QSG-H-LTE (SEQ ID NO: 10)

A zinc finger using QSG-H-LTE (SEQ ID NO: 10) was constructed in a Zif268 backbone. The binding capacity was analyzed using crude lysate by ELISA. The zinc finger bound CAG and CGA, but cross-reacted with most of the other triplets.

Another zinc finger using QSG-H-LTE (SEQ ID NO: 10) was constructed in a SP1C backbone. The binding capacity was analyzed using crude lysate by ELISA. The zinc finger bound most of the triplets except for CGT.

### 11. RAD-N-LTE (SEQ ID NO: 4)

A zinc finger using RAD-N-LTE (SEQ ID NO: 4) was constructed in a Zif268 backbone. The binding capacity was analyzed using crude lysate by ELISA. The zinc finger bound CAG with high specificity.

Another zinc finger using RAD-N-LTE (SEQ ID NO: 4) was constructed in a SP1 C backbone. The binding capacity was analyzed using crude lysate by ELISA. The zinc finger bound CAG, CGG, and CGG.

### 12. TSH-S-TLE (SEQ ID NO: 80)

A zinc finger using TSH-S-TLE (SEQ ID NO: 80) was constructed in a Zif268 backbone. The binding capacity was analyzed using crude lysate by ELISA. The zinc finger bound CTA with some cross-reactivity.

Another zinc finger using TSH-S-TLE (SEQ ID NO: 80) was constructed in a SP1C backbone. The binding capacity was analyzed using crude lysate by ELISA. The zinc finger bound CCA with considerable specificity.

### 13. NLQ-N-LGE (SEQ ID NO: 134)

A zinc finger using NLQ-N-LGE (SEQ ID NO: 134) was constructed in a SP1C backbone. The binding capacity was analyzed using crude lysate by ELISA. The zinc finger bound CCT with considerable specificity.

### 14. QSG-H-CRA (SEQ ID NO: 135)

A zinc finger using QSG-H-CRA (SEQ ID NO: 135) was constructed in a SP1C backbone. The binding capacity was analyzed using crude lysate by ELISA. The zinc finger bound CGA with considerable cross-reactivity; recognition of the 5' nucleotide was poor.

### 15. RSD-K-CRA (SEQ ID NO: 136)

A zinc finger using RSD-K-CRA (SEQ ID NO: 136) was constructed in a SP1C backbone. The binding capacity was analyzed using crude lysate by ELISA. The zinc finger bound CGG; recognition of the 5' nucleotide was poor.

### 16. HKN-A-CRA (SEQ ID NO: 137)

A zinc finger using HKN-A-CRA (SEQ ID NO: 137) was constructed in a SP1C backbone. The binding capacity was analyzed using crude lysate by ELISA. The zinc finger bound all of the 16 triplets.

### 17. TSG-A-LTE (SEQ ID NO: 138)

A zinc finger using TSG-A-LTE (SEQ ID NO: 138) was constructed in a SP1C backbone. The binding capacity was analyzed using partially purified lysate (1:8) by ELISA. The zinc finger bound CTT with some cross-reactivity.

### 18. TSG-T-LTE (SEQ ID NO: 139)

A zinc finger using TSG-T-LTE (SEQ ID NO: 139) was constructed in a SP1C backbone. The binding capacity was analyzed using partially purified lysate (1:8) by ELISA. The zinc finger bound CAC, CTC, and CTT.

### 19. TTG-A-LTE (SEQ ID NO: 82)

A zinc finger using TTG-A-LTE (SEQ ID NO: 82) was constructed in a SP1C backbone. The binding capacity was analyzed using partially purified lysate (1:8) by ELISA. The zinc finger bound CTT, with some cross-reactivity.

### 20. TTG-G-LTE (SEQ ID NO: 140)

A zinc finger using TTG-G-LTE (SEQ ID NO: 140) was constructed in a SP1C backbone. The binding capacity was analyzed using partially purified lysate (1:8) by ELISA. The zinc finger bound most of the triplets except CAC, CTC, and CTT.

### 21. DCR-T-LAE (SEQ ID NO: 141)

A zinc finger using DCR-T-LAE (SEQ ID NO: 141) was constructed in a SP1C backbone. The binding capacity was analyzed using partially purified lysate (1:8) by ELISA. The zinc finger bound CTT.

### 22. DCR-A-LTE (SEQ ID NO: 142)

A zinc finger using DCR-A-LTE (SEQ ID NO: 142) was constructed in a SP1C backbone. The binding capacity was analyzed using partially purified lysate (1:8) by ELISA. The zinc finger bound most of the triplets except CAC, CTC, and CTT.

### 23. DCR-T-LTE (SEQ ID NO: 143)

A zinc finger using DCR-T-LTE (SEQ ID NO: 143) was constructed in a SP1C backbone. The binding capacity was analyzed using partially purified lysate (1:8) by ELISA. the zinc finger bound most of the triplets except CAC, CTC, and CTT.

### 24. SCR-T-LAE (SEQ ID NO: 144)

A zinc finger using SCR-T-LAE (SEQ ID NO: 144) was constructed in a SP1C backbone. The binding capacity was analyzed using partially purified lysate (1:8) by ELISA. The zinc finger bound most of the triplets except CAC, CTC, and CTT.

### 25. SCR-A-LTE (SEQ ID NO: 145)

A zinc finger using SCR-A-LTE (SEQ ID NO: 145) was constructed in a SP1C backbone. The binding capacity was analyzed using partially purified lysate (1:8) by ELISA. The zinc finger bound most of the triplets except CAC, CTC, and CTT; however, there was some specificity for CCT and CGC.

### 26. SCR-T-LTE (SEQ ID NO: 146)

A zinc finger using SCR-T-LTE (SEQ ID NO: 146) was constructed in a SP1C backbone. The binding capacity was analyzed using partially purified lysate (1:8) by ELISA. The zinc finger bound CCT with some cross-reactivity.

### 27. DKK-A-LAE (SEQ ID NO: 147)

A zinc finger using DKK-A-LAE (SEQ ID NO: 147) was constructed in a SP1 C backbone. The binding capacity was analyzed using partially purified lysate (1:8) by ELISA. The zinc finger bound most of the triplets except CAC, CTC, and CTT.

### 28. DKK-T-LAE (SEa ID NO: 148)

A zinc finger using DKK-T-LAE (SEQ ID NO: 148) was constructed in a SP1C backbone. The binding capacity was analyzed using partially purified lysate (1:8) by ELISA. The zinc finger bound most of the triplets except CAC, CTC, and CTT.

### 29. DKK-A-LTE (SEQ ID NO: 149)

A zinc finger using DKK-A-LTE (SEQ ID NO: 149) was constructed in a SP1C backbone. The binding capacity was analyzed using partially purified lysate (1:8) by ELISA. The zinc finger bound most of the triplets except CAC, CTC, and CTT.

### 30. DKK-T-LTE (SEQ ID NO: 150)

A zinc finger using DKK-T-LTE (SEQ ID NO: 150) was constructed in a SP1 C backbone. The binding capacity was analyzed using partially purified lysate (1:8) by ELISA. The zinc finger bound CAC and CCC, with some cross-reactivity.

### 31. SKK-A-LAE (SEQ ID NO: 151)

A zinc finger using SKK-A-LAE (SEQ ID NO: 151) was constructed in a SP1C backbone. The binding capacity was analyzed using partially purified lysate (1:8) by ELISA. The zinc finger bound most of the triplets except CAC, CTC, and CTT.

### 32. SKK-T-LAE (SEQ ID NO: 152)

A zinc finger using SKK-T-LAE (SEQ ID NO: 152) was constructed in a SP1 C backbone. The binding capacity was analyzed using partially purified lysate (1:8) by ELISA. The zinc finger bound most of the triplets except CAC, CTC, and CTT.

### 33. SKK-A-LTE (SEQ ID NO: 77)

A zinc finger using SKK-A-LTE (SEQ ID NO: 77) was constructed in a SP1C backbone. The binding capacity was analyzed using partially purified lysate (1:8) by ELISA. The zinc finger bound CAC.

### 34. SKK-T-LTE (SEQ ID NO: 153)

A zinc finger using SKK-T-LTE (SEQ ID NO: 143) was constructed in a SP1C backbone. The binding capacity was analyzed using partially purified lysate (1:8) by ELISA. The zinc finger bound CAC.

### 35. HTG-A-LLE (SEQ ID NO: 154)

A zinc finger using HTG-A-LLE (SEQ ID NO: 154) was constructed in a SP1C backbone. The binding capacity was analyzed using partially purified lysate (1:8) by ELISA. The zinc finger bound most of the triplets except CAC, CTC, and CTT.

### 36. HTG-N-LLE (SEQ ID NO: 155)

A zinc finger using HTG-N-LLE (SEQ ID NO: 155) was constructed in a SP1 C backbone. The binding capacity was analyzed using partially purified lysate (1:8) by ELISA. The zinc finger bound most of the triplets except CAC, CTC, and CTT.

### 37. SKK-H-LAE (SEQ ID NO: 7)

A zinc finger using SKK-H-LAE (SEQ ID NO: 7) was constructed in a Zif268 backbone. The binding capacity was analyzed using crude lysate by ELISA. The zinc finger bound CCC.

### 38. DPG-N-LVR (SEQ ID NO: 156)

A zinc finger using DPG-N-LVR (SEQ ID NO: 156) was constructed in a SP1C backbone. The binding capacity was analyzed using partially purified lysate (1:8) by ELISA. The zinc finger bound GAC.

### 39. DSG-N-LVR (SEQ ID NO: 157)

A zinc finger using DSG-N-LVR (SEQ ID NO: 157) was constructed in a SP1C backbone. The binding capacity was analyzed using partially purified lysate (1:8) by ELISA. The zinc finger bound CAC and CAT; it was originally determined to have binding specificity for AAC.

**TABLE 4**

| **Summary of Protein and Nucleic Acid Sequences Recited** | |
|---|---|
| **Heptapeptide Zinc Finger Moieties** | |
| **Heptapeptide** | **SEQ ID NO** |
| QRH-N-LTE | 1 |
| QSG-N-LTE | 2 |
| NLQ-H-LGE | 3 |
| RAD-N-LTE | 4 |
| RAD-N-LAI | 5 |
| NTT-H-LEH | 6 |
| SKK-H-LAE | 7 |
| RND-T-LTE | 8 |
| RND-T-LNA | 9 |
| QSG-H-LTE | 10 |
| QLA-H-LKE | 11 |
| QRA-H-LTE | 12 |
| HTG-H-LLE | 13 |
| RSD-H-LTE | 14 |
| RSD-K-LTE | 15 |
| RSD-H-LTE | 16 |
| RSD-H-LTN | 17 |
| SRR-T-CRA | 18 |
| QLR-H-LRE | 19 |
| QRH-S-LTE | 20 |
| QLA-H-LKE | 21 |
| NLQ-H-LGE | 22 |
| RND-A-LTE | 23 |
| TKN-T-LTE | 24 |
| QSG-D-LTE | 25 |
| RSD-E-LKR | 26 |
| RSD-E-RKR | 27 |
| TSG-N-LVR | 28 |
| HRT-T-LTN | 35 |
| QRA-H-LER | 36 |
| DPG-A-LVR | 37 |
| RSD-K-LVR | 38 |
| TSG-E-LVR | 39 |
| RSD-H-LTN | 40 |
| DPG-H-LVR | 41 |
| DCR-D-LAR | 42 |
| KSA-D-LKR | 44 |
| RSD-H-LTT | 45 |
| TSG-S-LVR | 48 |
| QST-N-LKS | 54 |
| RAD-E-RKR | 57 |
| TSG-N-LVR | 58 |
| SPA-D-LTN | 59 |
| RAD-H-LAI | 60 |
| RSD-H-LTD | 61 |
| RND-T-LQA | 62 |
| SKK-H-LAE | 63 |
| SVR-N-LRE | 64 |
| RND-T-LQA | 65 |
| HTG-H-LLE | 66 |
| RSD-H-LTE | 67 |
| QLR-H-LRE | 68 |
| SRR-T-CRA | 69 |
| QRH-S-LTE | 70 |
| RND-A-LTE | 71 |
| RAD-N-LTE | 72 |
| RND-T-LTE | 73 |
| RSD-K-LTE | 74 |
| QSG-H-LTE | 75 |
| QSG-D-LRR | 76 |
| SKK-A-LTE | 77 |
| TSG-N-LTE | 78 |
| QNS-T-LTE | 79 |
| TSH-S-LTE | 80 |
| TKN-S-LTE | 81 |
| TTG-A-LTE | 82 |
| QRH-H-LVE | 85 |
| SPG-H-LIE | 86 |
| SCS-H-LAE | 87 |
| RKD-N-MTA | 88 |
| QLG-H-LIQ | 89 |
| NTK-Q-LRN | 90 |
| NTT-H-LEH | 91 |
| SCG-H-LTE | 92 |
| THR-D-LRQ | 93 |
| QRH-S-LTE | 94 |
| NCN-H-LAE | 95 |
| GPG-G-LIR | 96 |
| SPG-H-LVE | 97 |
| GLK-W-LVV | 98 |
| DLR-W-LVV | 99 |
| NCF-H-LTE | 100 |
| NVR-T-LDT | 101 |
| RLN-N-LLV | 102 |
| DRK-V-LAT | 103 |
| HVI-D-LDH | 104 |
| NTT-H-LEH | 105 |
| NVN-P-LPP | 106 |
| NHT-R-LAS | 107 |
| IPA-D-LKQ | 108 |
| RRR-G-WRK | 109 |
| ETR-T-LTA | 110 |
| HII-D-LDH | 111 |
| TRK-E-LRS | 112 |
| NHG-H-LTE | 113 |
| GRL-I-LLG | 114 |
| AMG-H-LVE | 115 |
| QWH-H-LTD | 116 |
| SCV-H-LAE | 117 |
| TCG-H-LVE | 118 |
| SCY-K-LRE | 119 |
| SCG-G-LTA | 120 |
| QCG-H-LVA | 121 |
| GCS-K-LRE | 122 |
| SCG-H-LQE | 123 |
| CCA-H-LSE | 124 |
| DHQ-D-LTI | 125 |
| DLR-S-CRS | 126 |
| QRR-H-LLS | 127 |
| LRR-Q-LAH | 128 |
| RMR-N-LQK | 129 |
| NLQ-H-LGE | 130 |
| NLQ-N-LGE | 134 |
| QSG-H-CRA | 135 |
| RSD-K-CRA | 136 |
| HKN-A-CRA | 137 |
| TSG-A-LTE | 138 |
| TSG-T-LTE | 139 |
| TTG-G-LTE | 140 |
| DCR-T-LAE | 141 |
| DCR-A-LTE | 142 |
| DCR-T-LTE | 143 |
| SCR-T-LAE | 144 |
| SCR-A-LTE | 145 |
| SCR-T-LTE | 146 |
| DKK-A-LAE | 147 |
| DKK-T-LAE | 148 |
| DKK-A-LTE | 149 |
| DKK-T-LTE | 150 |
| SKK-A-LAE | 151 |
| SKK-T-LAE | 152 |
| SKK-T-LTE | 153 |
| HTG-A-LLE | 154 |
| HTG-N-LLE | 155 |
| DPG-N-LVR | 156 |
| DSG-N-LVR | 157 |

| Other Protein Sequences | |
|---|---|
| TGEKP (Linker) | 30 |
| DALDDFDLDML (Synthetic Peptide) | 43 |
| TGGGGSGGGGTGEKP (Linker) | 133 |
| AAARA (Linker) | 158 |

| Nucleotide Sequences | |
|---|---|
| GATCNNGCG¹ | 29 |
| GAGGAAGTTT GCCACCAGTG GCAACCTGGT GAGGCATACC AAAATC | 31 |
| GTAAAACGAC GGCCAGTGCC AAGC | 32 |
| GGCCGCNNNA TCGAGTTTTC TCGANNNGC GGCC² | 33 |
| GATCNNGCG¹ | 34 |
| GCGTGGGCG | 46 |
| GATTGGGCG | 47 |
| CNNCNNCNNC NNCNNCNNCN NCNNCNNCNN CNNCNN³ | 49 |
| CNNCNNCNNC NNCNNCNN⁴ | 50 |
| GNNGNNGNNG NNGNNGNNGN NGNNGNNGNN GNNGNN⁵ | 51 |
| ANNANNANNA NNANNANNAN NANNANNANN ANNANN⁶ | 52 |
| TNNTNNTNNT NNTNNTNNTN NTNNTNNTNN TNNTNN⁷ | 53 |
| GGCTGCTTGA GGAAGTATAA AATGAAGTTG GAAG | 55 |
| CTTTATGTTT TTGGCGTCTT CCA | 56 |
| CGGGGGGCTC CCCTGGTT | 83 |
| GTCAAGGCAA GGCTGGCC | 84 |
| AACCAGGGGAGCCCCCCG | 131 |
| TTGGTCCCCTCGGGGGGC | 132 |

| | |
|---|---|
| Footnotes for Nucleic Acid Sequences ¹N=any of A, C, G, or T ²Bases 7-9 complementary to bases 26-28; bases 26-28 any of A, C, G, or T ³Encompasses 2 to 12 repeats of CNN nucleotide motif, where N is any of A, C, G, or T ⁴Encompasses 2 to 6 repeats of CNN nucleotide motif, where N is any of A, C, G, or T ⁵Encompasses 2 to 12 repeats of GNN nucleotide motif, where N is any of A, C, G, or T ⁶Encompasses 2 to 12 repeats of ANN nucleotide motif, where N is any of A, C, G, or T ⁷Encompasses 2 to 12 repeats of TNN nucleotide motif, where N is any of A, C, G, or T | |

### ADVANTAGES OF THE INVENTION

The present invention provides versatile binding proteins for nucleic acid sequences, particularly DNA sequences. These binding proteins can be coupled with transcription mdulators and can therefore be utilized for the upregulation or downregulation of particular genes in a specific manner. These binding proteins can, therefore, be used in gene therapy or protein therapy for the treatment of cancer, autoimmune diseases, metabolic disorders, developmental disorders, and other diseases or conditions associated with the dysregulation of gene expression.

The binding proteins for nucleic acid sequences, nucleic acid sequences encoding the binding proteins, vectors incorporating the nucleic acid sequences, and host cells transformed or transfected by the vectors possess industrial applicability for the preparation of medicaments for the treatment of diseases or conditions characterized by the dysregulation of transcription or the presence of abnormal patterns of transcription. These binding proteins, nucleic acid sequences, vectors, and host cells also possess industrial applicability for screening of proteins, nucleic acids, and cells for nucleic acid sequences to develop and isolate further zinc finger proteins.

With respect to ranges of values, the invention encompasses each intervening value between the upper and lower limits of the range to at least a tenth of the lower limit's unit, unless the context clearly indicates otherwise. Moreover, the invention encompasses any other stated intervening values and ranges including either or both of the upper and lower limits of the range, unless specifically excluded from the stated range.

Unless defined otherwise, the meanings of all technical and scientific terms used herein are those commonly understood by one of ordinary skill in the art to which this invention belongs. One of ordinary skill in the art will also appreciate that any methods and materials similar or equivalent to those described herein can also be used to practice or test this invention.

The publications and patents discussed herein are provided solely for their disclosure prior to the filing date of the present application. Nothing herein is to be construed as an admission that the present invention is not entitled to antedate such publication by virtue of prior invention. Further the dates of publication provided may be different from the actual publication dates which may need to be independently confirmed.

All the publications cited are incorporated herein by reference in their entireties, including all published patents, patent applications, literature references, as well as those publications that have been incorporated in those published documents. However, to the extent that any publication incorporated herein by reference refers to information to be published, applicants do not admit that any such information published after the filing date of this application to be prior art.

As used in this specification and in the appended claims, the singular forms include the plural forms. For example the terms "a," "an," and "the" include plural references unless the content clearly dictates otherwise. Additionally, the term "at least" preceding a series of elements is to be understood as referring to every element in the series. The inventions illustratively described herein can suitably be practiced in the absence of any element or elements, limitation or limitations, not specifically disclosed herein. Thus, for example, the terms "comprising," "including," "containing," etc. shall be read expansively and without limitation. Additionally, the terms and expressions employed herein have been used as terms of description and not of limitation, and there is no intention in the use of such terms and expressions of excluding any equivalents of the future shown and described or any portion thereof, and it is recognized that various modifications are possible within the scope of the invention claimed. Thus, it should be understood that although the present invention has been specifically disclosed by preferred embodiments and optional features, modification and variation of the inventions herein disclosed can be resorted by those skilled in the art, and that such modifications and variations are considered to be within the scope of the inventions disclosed herein. The inventions have been described broadly and generically herein. Each of the narrower species and subgeneric groupings falling within the scope of the generic disclosure also form part of these inventions. This includes the generic description of each invention with a proviso or negative limitation removing any subject matter from the genus, regardless of whether or not the excised materials specifically resided therein. In addition, where features or aspects of an invention are described in terms of the Markush group, those schooled in the art will recognize that the invention is also thereby described in terms of any individual member or subgroup of members of the Markush group. It is also to be understood that the above description is intended to be illustrative and not restrictive. Many embodiments will be apparent to those of in the art upon reviewing the above description. The scope of the invention should therefore, be determined not with reference to the above description, but should instead be determined with reference to the appended claims, along with the full scope of equivalents to which such claims are entitled. Those skilled in the art will recognize, or will be able to ascertain using no more than routine experimentation, many equivalents to the specific embodiments of the invention described. Such equivalents are intended to be encompassed by the following claims.

### EXAMPLE 11

### Introduction of the Coding Regions of the E2S-KRAB. E2S-VP64. E3F-KRAB and E3F-VP64 Proteins Into the Retroviral Vector pM-IRES-GFP.

In order to express the E2S-KRAB, E2S-VP64, E3F-KRAB and E3F-VP64 proteins (See Table 2, below) in several cell lines, their coding regions were introduced into the retroviral vector pMX-IRES-GFP.

The sequences of these constructs were selected to bind to specific regions of the ErbB-2 or ErbB-3 promoters (See Table 2). The coding regions were PCR amplified from pcDNA3-based expression plasmids (R. R. Beerli, D. J. Segal, B. Dreier, C. F. Barbas, III, Proc. Natl. Acad. Sci. USA 95, 14628 (1998)) and subcloned into pRevTRE (Clontech) using BamHI and Clal restriction sites, and into pMX-IRES-GFP [X. Liu et al., Proc. Natl. Acad. Sci. USA 94, 10669 (1997)] using BamHI and Notl restriction sites. Fidelity of the PCR amplification was confirmed by sequencing. This vector expresses a single bicistronic message for the translation of the zinc finger protein and, from an internal ribosome-entry site (IRES), the green fluorescent protein (GFP). Since both coding regions share the same mRNA, their expression is physically linked to one another and GFP expression is an indicator of zinc finger expression. Virus prepared from these plasmids was then used to infect the human carcinoma cell line A431.

**TABLE 2**

| DNA Target e2t 5'→3' | CAA | CGA | AGT | CTG | GGA | GTC |
|---|---|---|---|---|---|---|
| Zinc Finger Sequence E2T | QRHNLTE | QLAHLKE | HRTTLTN | RNDALTE | QRAHLER | DPGALVR |
| SEQ ID NO: | 1 | 11 | 35 | 23 | 36 | 37 |
| DNA Target e2s 5'→3' | CGG | GGG | GCT | CCC | CTG | GTT |
| Zinc Finger Sequence E2S | RSDHLTE | RSDKLVR | TSGELYR | SKKRLAE | RNDALTE | TSGSLVR |
| SEQ ID NO: | 14 | 38 | 39 | 7 | 23 | 39 |
| DNA Target e3f 5'→3' | AGG | GGC | CCC | CGG | GCC | GGA |
| Zinc Finger Sequence E3F | RSDHLTN | DPGULVR | SKKHLAE | RSDHLTE | DCRDLAR | QRAHLER |
| SEQ ID NO: | 40 | 41 | 7 | 14 | 42 | 36 |

## Claims

1. A non-naturally occurring zinc finger nucleotide binding polypeptide comprising a zinc finger nucleotide binding region, which binding region binds preferentially to a target nucleotide sequence of the formula CAN₁, wherein N₁ is C or T, CCN₂, wherein N₂ is A or T, or CTN₃, wherein N₃ is A or T.

2. The polypeptide of claim 1 wherein the target nucleotide sequence has the formula CAC or CAT.

3. The polypeptide of claim 1 wherein the target nucleotide sequence has the Formula CCA or CCT.

4. The polypeptide of claim 1 wherein the target nucleotide sequence has the formula CTA or CTT.

5. The polypeptide of claim 1 wherein the binding region has the amino acid sequence of any of SEQ ID NOs: 77-82, 134, 138, 141 or 146.

6. The polypeptide of claim 1 wherein the binding region has an amino acid sequence binding region differing from the amino acid sequence of any of SEQ ID NOs: 77-82, 138, 141 or 146 by no more than two conservative amino acid substitutions, wherein the a dissociation constant is no greater than 125% of that of the polypeptide before the substitutions are made, and wherein a conservative amino acid substitution is one of the following substitutions: Ala/Gly or Ser; Arg/Lys; Asn/Gln or His; Asp/Glu; Cys/Ser; Gln/Asn; Gly/Asp; Gly/Ala or Pro; His/Asn or Gln; Ile/Leu or Val; Leu/Ile or Val; Lys/Arg or Gln or Glu; Met/Leu or Tyr or Ile, Phe/Met or Leu or Tyr; Ser/Thr; Thr/Ser; Trp/Tyr; Tyr/Trp or Phe; or Val/Ile or Leu.

7. The polypeptide of claim I wherein the nucleotide binding region is linked to sequences for a naturally-occurring zinc finger protein selected from the group consisting of SP1C, TFIIIA, and Zif268.

8. A polypeptide composition comprising a plurality of the polypeptides of claim 1, wherein the polypeptides are operatively linked to each other.

9. The polypeptide composition of claim 8 wherein the polypeptides are operatively linked via a flexible peptide linker of from 5 to 15 amino acid residues.

10. The polypeptide composition of claim 8 wherein the linker has a sequence selected from the group consisting of SEQ ID NO: 30 and SEQ ID NO: 133.

11. The polypeptide composition of claim 8 wherein the composition comprises from 2 to 12 polypeptides.

12. The polypeptide composition of claim 11 wherein the composition comprises from 2 to 6 polypeptides.

13. The polypeptide composition of claim 9 wherein the nucleotide binding region is linked to sequences for a naturally-occurring zinc finger protein selected from the group consisting of SP1C, TFIIIA, and Zif268.

14. An isolated heptapeptide having an α-helical structure and that binds preferentially to a target nucleotide sequence of the formula CAN₁ wherein N₁ is C or T, CCN₂ wherein N₂ is A or T, or CTN₃ wherein N₃ is A or T.

15. The isolated heptapeptide of claim 14 wherein the target nucleotide sequence has the formula CAC, CAT, CCA, CCT or CTT.

16. The isolated heptapeptide of claim 14 wherein the heptapeptide has the amino acid sequence of any of SEQ ID NOs: 77-82, 134, 138, 141 or 146.

17. The isolated heptapeptide of claim 14 wherein the heptapeptide has an amino acid sequence differing from the amino acid sequence of any of SEQ ID NOs: 77-80, 82, 138, 141 or 146 by no more than two conservative amino acid substitutions, wherein the a dissociation constant is no greater than 125% of that of the polypeptide before the substitutions are made, and wherein a conservative amino acid substitution is one of the following substitutions: Ala/Gly or Ser; Arg/Lys; Asn/Gln or His; Asp/Glu; Cys/Ser; Gln/Asn; Gly/Asp; Gly/Ala or Pro; His/Asn or Glen; Ile/Leu or Val; Leu/Ile or Val; Lys/Arg or Gln or Glu; Met/Leu or Tyr or Ile; Phe/Met or Leu or Tyr; Ser/Thr; Thr/Ser; Trp/Tyr; Tyr/Tip or Phe; or Val/Ile or Leu.

18. The polypeptide of claim 1 or the polypeptide composition of claim 8 operatively linked to one or more transcription regulating factors.

19. The polypeptide of claim 18 wherein the transcription regulating factor is a repressor of transcription.

20. The polypeptide of claim 18 wherein the transcription regulating factor is an activator of transcription.

21. An isolated and purified polynucleotide that encodes the polypeptide of claim 1, one of the plurality of polypeptides in the composition of claim 8, or the heptapeptide of claim 14, or nucleic acid sequences that are at least 95% identical thereto which encode a polypeptide that possesses the specific nucleic acid binding activity of the polypeptide of claim 1.

22. A vector comprising the isolated and purified polynucleotide of claim 21.

23. A host cell transformed or transfected with the vector of claim 22 or the polynucleotide of claim 21.

24. The host cell of claim 23 that is eukaryotic.

25. The host cell of claim 23 that is prokaryotic.

26. A process of regulating expression of a nucleotide sequence that contains the sequence 5'-(CNN)ₙ-3', where n is 2 to 12, the process comprising exposing the nucleotide sequence to an effective amount of the polypeptide of claim 1 or the polypeptide composition of claim 8.

27. The process of claim 26 wherein the sequence 5'-(CNN)-3' is located within a 5'-(CNN)ₙ-3' sequence.

28. The process of claim 26 wherein the sequence 5'-(CNN)ₙ-3' is located in the transcribed region of the nucleotide sequence.

29. The process of claim 26 wherein the sequence 5'-(CNN)ₙ-3' is located in a promoter region of the nucleotide sequence.

30. The process of claim 26 wherein the sequence 5'-(CNN)ₙ-3' is located within an expressed sequence tag.

31. The process of claim 26 wherein at least one of the polypeptides in the composition is operatively linked to one or more transcription regulating factors.

32. The process of claim 31 wherein the transcription regulating factor is a repressor of transcription.

33. The process of claim 31 wherein the transcription regulating factor is an activator of transcription.

34. The process of claim 26 wherein the nucleotide sequence is a gene.

35. The process of claim 34 wherein the gene is a eukaryotic gene, a prokaryotic gene or a viral gene.

36. The process of claim 35 wherein the eukaryotic gene is a mammalian or a plant gene.

37. The process of claim 36 wherein the mammalian gene is a human gene.

38. The process of claim 35 wherein the prokaryotic gene is a bacterial gene.

39. A pharmaceutical composition comprising:
(a) a therapeutically effective amount of the polypeptide of claim 1, the polypeptide composition of claim 8 or the heptapeptide of claim 14; and
(b) a pharmaceutically acceptable carrier.

40. A pharmaceutical composition comprising:
(a) a therapeutically effective amount of the polynucleotide of claim 21; and
(b) a pharmaceutically acceptable carrier.


SEQUENCE LISTING

<110> THE SCRIPPS RESEARCH INSTITUTE

<120> ZINC FINGER BINDING DOMAINS FOR CNN

<130> 8098-012-WO

<140> PCT/US2006/029075
<141> 2006-07-26

<150> 60/707,839
<151> 2005-08-11

<160> 158

<170> PatentIn Ver. 3.3

<210> 1
<211> 7
<212> PRT
<213> Artificial Sequence

<220>
<223> Description of Artificial Sequence: Synthetic
      peptide

<400> 1
Gln Arg His Asn Leu Thr Glu
  1               5


<210> 2
<211> 7
<212> PRT
<213> Artificial Sequence

<220>
<223> Description of Artificial Sequence: Synthetic
      peptide

<400> 2
Gln Ser Gly Asn Leu Thr Glu
  1               5


<210> 3
<211> 7
<212> PRT
<213> Artificial Sequence

<220>
<223> Description of Artificial Sequence: Synthetic
      peptide

<400> 3
Asn Leu Gln His Leu Gly Glu
  1               5


<210> 4
<211> 7
<212> PRT
<213> Artificial Sequence

<220>
<223> Description of Artificial Sequence: Synthetic
      peptide

<400> 4
Arg Ala Asp Asn Leu Thr Glu
1               5


<210> 5
<211> 7
<212> PRT
<213> Artificial Sequence

<220>
<223> Description of Artificial Sequence: Synthetic
      peptide

<400> 5
Arg Ala Asp Asn Leu Ala Ile
1               5


<210> 6
<211> 7
<212> PRT
<213> Artificial Sequence

<220>
<223> Description of Artificial Sequence: Synthetic
      peptide

<400> 6
Asn Thr Thr His Leu Glu His
1               5


<210> 7
<211> 7
<212> PRT
<213> Artificial Sequence

<220>
<223> Description of Artificial Sequence: Synthetic
      peptide

<400> 7
Ser Lys Lys His Leu Ala Glu
1               5


<210> 8
<211> 7
<212> PRT
<213> Artificial Sequence

<220>
<223> Description of Artificial Sequence: Synthetic
      peptide

<400> 8
Arg Asn Asp Thr Leu Thr Glu
1               5


<210> 9
<211> 7
<212> PRT
<213> Artificial Sequence

<220>

<223> Description of Artificial Sequence: Synthetic
      peptide

<400> 9
Arg Asn Asp Thr Leu Asn Ala
1               5


<210> 10
<211> 7
<212> PRT
<213> Artificial Sequence

<220>
<223> Description of Artificial Sequence: Synthetic
      peptide

<400> 10
Gln Ser Gly His Leu Thr Glu
1               5


<210> 11
<211> 7
<212> PRT
<213> Artificial Sequence

<220>
<223> Description of Artificial Sequence: Synthetic
      peptide

<400> 11
Gln Leu Ala His Leu Lys Glu
1               5


<210> 12
<211> 7
<212> PRT
<213> Artificial Sequence

<220>
<223> Description of Artificial Sequence: Synthetic
      peptide

<400> 12
Gln Arg Ala His Leu Thr Glu
1               5


<210> 13
<211> 7
<212> PRT
<213> Artificial Sequence

<220>
<223> Description of Artificial Sequence: Synthetic
      peptide

<400> 13
His Thr Gly His Leu Leu Glu
1               5


<210> 14
<211> 7

<212> PRT
<213> Artificial Sequence

<220>
<223> Description of Artificial Sequence: Synthetic
      peptide

<400> 14
Arg Ser Asp His Leu Thr Glu
  1               5


<210> 15
<211> 7
<212> PRT
<213> Artificial Sequence

<220>
<223> Description of Artificial Sequence: Synthetic
      peptide

<400> 15
Arg Ser Asp Lys Leu Thr Glu
  1               5


<210> 16
<211> 7
<212> PRT
<213> Artificial Sequence

<220>
<223> Description of Artificial Sequence: Synthetic
      peptide

<400> 16
Arg Ser Asp His Leu Thr Glu
  1               5


<210> 17
<211> 7
<212> PRT
<213> Artificial Sequence

<220>
<223> Description of Artificial Sequence: Synthetic
      peptide

<400> 17
Arg Ser Asp His Leu Thr Asn
  1               5


<210> 18
<211> 7
<212> PRT
<213> Artificial Sequence

<220>
<223> Description of Artificial Sequence: Synthetic
      peptide

<400> 18
Ser Arg Arg Thr Cys Arg Ala
  1               5

<210> 19
<211> 7
<212> PRT
<213> Artificial Sequence

<220>
<223> Description of Artificial Sequence: Synthetic
      peptide

<400> 19
Gln Leu Arg His Leu Arg Glu
  1               5

<210> 20
<211> 7
<212> PRT
<213> Artificial Sequence

<220>
<223> Description of Artificial Sequence: Synthetic
      peptide

<400> 20
Gln Arg His Ser Leu Thr Glu
  1               5

<210> 21
<211> 7
<212> PRT
<213> Artificial Sequence

<220>
<223> Description of Artificial Sequence: Synthetic
      peptide

<400> 21
Gln Leu Ala His Leu Lys Glu
  1               5

<210> 22
<211> 7
<212> PRT
<213> Artificial Sequence

<220>
<223> Description of Artificial Sequence: Synthetic
      peptide

<400> 22
Asn Leu Gln His Leu Gly Glu
  1               5

<210> 23
<211> 7
<212> PRT
<213> Artificial Sequence

<220>
<223> Description of Artificial Sequence: Synthetic
      peptide

<400> 23
Arg Asn Asp Ala Leu Thr Glu
1               5

<210> 24
<211> 7
<212> PRT
<213> Artificial Sequence

<220>
<223> Description of Artificial Sequence: Synthetic
      peptide

<400> 24
Thr Lys Asn Thr Leu Thr Glu
1               5

<210> 25
<211> 7
<212> PRT
<213> Artificial Sequence

<220>
<223> Description of Artificial Sequence: Synthetic
      peptide

<400> 25
Gln Ser Gly Asp Leu Thr Glu
1               5

<210> 26
<211> 7
<212> PRT
<213> Artificial Sequence

<220>
<223> Description of Artificial Sequence: Synthetic
      peptide

<400> 26
Arg Ser Asp Glu Leu Lys Arg
1               5

<210> 27
<211> 7
<212> PRT
<213> Artificial Sequence

<220>
<223> Description of Artificial Sequence: Synthetic
      peptide

<400> 27
Arg Ser Asp Glu Arg Lys Arg
1               5

<210> 28
<211> 7

<212> PRT
<213> Artificial Sequence

<220>
<223> Description of Artificial Sequence: Synthetic
      peptide

<400> 28
Thr Ser Gly Asn Leu Val Arg
  1               5


<210> 29
<211> 9
<212> DNA
<213> Artificial Sequence

<220>
<223> Description of Artificial Sequence: Synthetic
      primer

<220>
<221> modified_base
<222> (5)..(6)
<223> a, c, g, or t

<400> 29
gatcnngcg                                                          9


<210> 30
<211> 5
<212> PRT
<213> Artificial Sequence

<220>
<223> Description of Artificial Sequence: Synthetic
      peptide

<400> 30
Thr Gly Glu Lys Pro
  1               5


<210> 31
<211> 46
<212> DNA
<213> Artificial Sequence

<220>
<223> Description of Artificial Sequence: Synthetic
      primer

<400> 31
gaggaagttt gccaccagtg gcaacctggt gaggcatacc aaaatc                 46


<210> 32
<211> 24
<212> DNA
<213> Artificial Sequence

<220>
<223> Description of Artificial Sequence: Synthetic
      primer

<400> 32
gtaaaacgac ggccagtgcc aagc                                         24

<210> 33
<211> 33
<212> DNA
<213> Artificial Sequence

<220>
<223> Description of Artificial Sequence: Synthetic
      primer

<220>
<221> modified_base
<222> (7)..(9)
<223> a, c, g, or t and this region is complementary
        to nucleotides 26-28

<220>
<221> modified_base
<222> (25)..(27)
<223> a, c, g, or t

<400> 33
ggccgcnnna tcgagttttc tcgannngcg gcc                                33


<210> 34
<211> 9
<212> DNA
<213> Artificial Sequence

<220>
<223> Description of Artificial Sequence: Synthetic
      primer

<220>
<221> modified_base
<222> (5)..(6)
<223> a, c, g, or t

<400> 34
gatcnngcg                                                            9



<210> 35
<211> 7
<212> PRT
<213> Artificial Sequence

<220>
<223> Description of Artificial Sequence: Synthetic
      peptide

<400> 35
His Arg Thr Thr Leu Thr Asn
  1               5


<210> 36
<211> 7
<212> PRT
<213> Artificial Sequence

<220>
<223> Description of Artificial Sequence: Synthetic
      peptide

<400> 36
Gln Arg Ala His Leu Glu Arg
1               5


<210> 37
<211> 7
<212> PRT
<213> Artificial Sequence

<220>
<223> Description of Artificial Sequence: Synthetic
      peptide

<400> 37
Asp Pro Gly Ala Leu Val Arg
1               5


<210> 38
<211> 7
<212> PRT
<213> Artificial Sequence

<220>
<223> Description of Artificial Sequence: Synthetic
      peptide

<400> 38
Arg Ser Asp Lys Leu Val Arg
1               5


<210> 39
<211> 7
<212> PRT
<213> Artificial Sequence

<220>
<223> Description of Artificial Sequence: Synthetic
      peptide

<400> 39
Thr Ser Gly Glu Leu Val Arg
1               5


<210> 40
<211> 7
<212> PRT
<213> Artificial Sequence

<220>
<223> Description of Artificial Sequence: Synthetic
      peptide

<400> 40
Arg Ser Asp His Leu Thr Asn
1               5


<210> 41
<211> 7
<212> PRT
<213> Artificial Sequence

<220>
<223> Description of Artificial Sequence: Synthetic
      peptide

<400> 41
Asp Pro Gly His Leu Val Arg
  1               5


<210> 42
<211> 7
<212> PRT
<213> Artificial Sequence

<220>
<223> Description of Artificial Sequence: Synthetic
      peptide

<400> 42
Asp Cys Arg Asp Leu Ala Arg
  1               5



<210> 43
<211> 11
<212> PRT
<213> Artificial Sequence

<220>
<223> Description of Artificial Sequence: Synthetic
      peptide

<400> 43
Asp Ala Leu Asp Asp Phe Asp Leu Asp Met Leu
  1               5                   10


<210> 44
<211> 7
<212> PRT
<213> Artificial Sequence

<220>
<223> Description of Artificial Sequence: Synthetic
      peptide

<400> 44
Lys Ser Ala Asp Leu Lys Arg
  1               5


<210> 45
<211> 7
<212> PRT
<213> Artificial Sequence

<220>
<223> Description of Artificial Sequence: Synthetic
      peptide

<400> 45
Arg Ser Asp His Leu Thr Thr
  1               5

<210> 46
<211> 9
<212> DNA
<213> Artificial Sequence

<220>
<223> Description of Artificial Sequence: Synthetic
      primer

<400> 46
gcgtgggcg                                                              9

<210> 47
<211> 9
<212> DNA
<213> Artificial Sequence

<220>
<223> Description of Artificial Sequence: Synthetic
      primer

<400> 47
gattgggcg                                                              9

<210> 48
<211> 7
<212> PRT
<213> Artificial Sequence

<220>
<223> Description of Artificial Sequence: Synthetic
      peptide

<400> 48
Thr Ser Gly Ser Leu Val Arg
  1               5

<210> 49
<211> 36
<212> DNA
<213> Artificial Sequence

<220>
<223> Description of Artificial Sequence: Synthetic
      primer

<220>
<221> modified_base
<222> (2)..(3)
<223> a, c, g, or t

<220>
<221> modified_base
<222> (5)..(6)
<223> a, c, g, or t

<220>
<221> modified_base
<222> (8)..(9)
<223> a, c, g, or t

<220>

<221> modified_base
<222> (11)..(12)
<223> a, c, g, or t

<220>
<221> modified_base
<222> (14)..(15)
<223> a, c, g, or t

<220>
<221> modified_base
<222> (17)..(18)
<223> a, c, g, or t

<220>
<221> modified_base
<222> (20)..(21)
<223> a, c, g, or t

<220>
<221> modified_base
<222> (23)..(24)
<223> a, c, g, or t

<220>
<221> modified_base
<222> (26)..(27)
<223> a, c, g, or t

<220>
<221> modified_base
<222> (29)..(30)
<223> a, c, g, or t

<220>
<221> modified_base
<222> (32)..(33)
<223> a, c, g, or t

<220>
<221> modified_base
<222> (35)..(36)
<223> a, c, g, or t

<220>
<223> This sequence may encompass 2 to 12 "CNN" repeating
      units

<400> 49
cnncnncnnc nncnncnncn ncnncnncnn cnncnn                                36


<210> 50
<211> 18
<212> DNA
<213> Artificial Sequence

<220>
<223> Description of Artificial Sequence: Synthetic
      primer

<220>
<221> modified_base
<222> (2)..(3)
<223> a, c, g, or t

<220>
<221> modified_base

<222> (5)..(6)
<223> a, c, g, or t

<220>
<221> modified_base
<222> (8)..(9)
<223> a, c, g, or t

<220>
<221> modified_base
<222> (11)..(12)
<223> a, c, g, or t

<220>
<221> modified_base
<222> (14)..(15)
<223> a, c, g, or t

<220>
<221> modified_base
<222> (17)..(18)
<223> a, c, g, or t

<220>
<223> This sequence may encompass 2 to 6 "CNN" repeating
      units

<400> 50
cnncnncnnc nncnncnn                                                    18


<210> 51
<211> 36
<212> DNA
<213> Artificial Sequence

<220>
<223> Description of Artificial Sequence: Synthetic
      primer

<220>
<221> modified_base
<222> (2)..(3)
<223> a, c, g, or t

<220>
<221> modified_base
<222> (5)..(6)
<223> a, c, g, or t

<220>
<221> modified_base
<222> (8)..(9)
<223> a, c, g, or t

<220>
<221> modified_base
<222> (11)..(12)
<223> a, c, g, or t

<220>
<221> modified_base
<222> (14)..(15)
<223> a, c, g, or t

<220>
<221> modified_base
<222> (17)..(18)

<223> a, c, g, or t

<220>
<221> modified_base
<222> (20)..(21)
<223> a, c, g, or t

<220>
<221> modified_base
<222> (23)..(24)
<223> a, c, g, or t

<220>
<221> modified_base
<222> (26)..(27)
<223> a, c, g, or t

<220>
<221> modified_base
<222> (29)..(30)
<223> a, c, g, or t

<220>
<221> modified_base
<222> (32)..(33)
<223> a, c, g, or t

<220>
<221> modified_base
<222> (35)..(36)
<223> a, c, g, or t

<220>
<223> This sequence may encompass 2 to 12 "GNN" repeating
      units

<400> 51
gnngnngnng nngnngnngn ngnngnngnn gnngnn                                36


<210> 52
<211> 36
<212> DNA
<213> Artificial Sequence

<220>
<223> Description of Artificial Sequence: Synthetic
      primer

<220>
<221> modified_base
<222> (2)..(3)
<223> a, c, g, or t

<220>
<221> modified_base
<222> (5)..(6)
<223> a, c, g, or t

<220>
<221> modified_base
<222> (8)..(9)
<223> a, c, g, or t

<220>
<221> modified_base
<222> (11)..(12)
<223> a, c, g, or t

<220>
<221> modified_base
<222> (14)..(15)
<223> a, c, g, or t

<220>
<221> modified_base
<222> (17)..(18)
<223> a, c, g, or t

<220>
<221> modified_base
<222> (20)..(21)
<223> a, c, g, or t

<220>
<221> modified_base
<222> (23)..(24)
<223> a, c, g, or t

<220>
<221> modified_base
<222> (26)..(27)
<223> a, c, g, or t

<220>
<221> modified_base
<222> (29)..(30)
<223> a, c, g, or t

<220>
<221> modified_base
<222> (32)..(33)
<223> a, c, g, or t

<220>
<221> modified_base
<222> (35)..(36)
<223> a, c, g, or t

<220>
<223> This sequence may encompass 2 to 12 "ANN" repeating
      Units

<400> 52
annannanna nnannannan nannannann annann                                36


<210> 53
<211> 36
<212> DNA
<213> Artificial Sequence

<220>
<223> Description of Artificial Sequence: Synthetic
      primer

<220>
<221> modified_base
<222> (2)..(3)
<223> a, c, g, or t

<220>
<221> modified_base
<222> (5)..(6)
<223> a, c, g, or t

<220>
<221> modified_base
<222> (8)..(9)
<223> a, c, g, or t

<220>
<221> modified_base
<222> (11)..(12)
<223> a, c, g, or t

<220>
<221> modified_base
<222> (14)..(15)
<223> a, c, g, or t

<220>
<221> modified_base
<222> (17)..(18)
<223> a, c, g, or t

<220>
<221> modified_base
<222> (20)..(21)
<223> a, c, g, or t

<220>
<221> modified_base
<222> (23)..(24)
<223> a, c, g, or t

<220>
<221> modified_base
<222> (26)..(27)
<223> a, c, g, or t

<220>
<221> modified_base
<222> (29)..(30)
<223> a, c, g, or t

<220>
<221> modified_base
<222> (32)..(33)
<223> a, c, g, or t

<220>
<221> modified_base
<222> (35)..(36)
<223> a, c, g, or t

<220>
<223> This sequence may encompass 2 to 12 "TNN" repeating
      Units

<400> 53
tnntnntnnt nntnntnntn ntnntnntnn tnntnn                                36


<210> 54
<211> 7
<212> PRT
<213> Artificial Sequence

<220>
<223> Description of Artificial Sequence: Synthetic
      peptide

<400> 54

Gln Ser Thr Asn Leu Lys Ser
1               5


<210> 55
<211> 34
<212> DNA
<213> Artificial Sequence


<220>
<223> Description of Artificial Sequence: Synthetic
      primer

<400> 55
ggctgcttga ggaagtataa aatgaagttg gaag                                34


<210> 56
<211> 23
<212> DNA
<213> Artificial Sequence

<220>
<223> Description of Artificial Sequence: Synthetic
      primer

<400> 56
ctttatgttt ttggcgtctt cca                                            23


<210> 57
<211> 7
<212> PRT
<213> Artificial Sequence

<220>
<223> Description of Artificial Sequence: Synthetic
      peptide

<400> 57
Arg Ala Asp Glu Arg Lys Arg
1               5


<210> 58
<211> 7
<212> PRT
<213> Artificial Sequence

<220>
<223> Description of Artificial Sequence: Synthetic
      peptide

<400> 58
Thr Ser Gly Asn Leu Val Arg
1               5


<210> 59
<211> 7
<212> PRT
<213> Artificial Sequence

<220>
<223> Description of Artificial Sequence: Synthetic
      peptide

<400> 59
Ser Pro Ala Asp Leu Thr Asn
1               5


<210> 60
<211> 7
<212> PRT
<213> Artificial Sequence

<220>
<223> Description of Artificial Sequence: Synthetic
      peptide

<400> 60
Arg Ala Asp His Leu Ala Ile
1               5


<210> 61
<211> 7
<212> PRT
<213> Artificial Sequence

<220>
<223> Description of Artificial Sequence: Synthetic
      peptide

<400> 61
Arg Ser Asp His Leu Thr Asp
1               5


<210> 62
<211> 7
<212> PRT
<213> Artificial Sequence

<220>
<223> Description of Artificial Sequence: Synthetic
      peptide

<400> 62
Arg Asn Asp Thr Leu Gln Ala
1               5


<210> 63
<211> 7
<212> PRT
<213> Artificial Sequence

<220>
<223> Description of Artificial Sequence: Synthetic
      peptide



<400> 63
Ser Lys Lys His Leu Ala Glu
1               5


<210> 64
<211> 7
<212> PRT
<213> Artificial Sequence

<220>
<223> Description of Artificial Sequence: Synthetic
      peptide

<400> 64
Ser Val Arg Asn Leu Arg Glu
  1               5


<210> 65
<211> 7
<212> PRT
<213> Artificial Sequence

<220>
<223> Description of Artificial Sequence: Synthetic
      peptide

<400> 65
Arg Asn Asp Thr Leu Gln Ala
  1               5


<210> 66
<211> 7
<212> PRT
<213> Artificial Sequence

<220>
<223> Description of Artificial Sequence: Synthetic
      peptide

<400> 66
His Thr Gly His Leu Leu Glu
  1               5


<210> 67
<211> 7
<212> PRT
<213> Artificial Sequence

<220>
<223> Description of Artificial Sequence: Synthetic
      peptide



<400> 67
Arg Ser Asp His Leu Thr Glu
  1               5


<210> 68
<211> 7
<212> PRT
<213> Artificial Sequence

<220>
<223> Description of Artificial Sequence: Synthetic
      peptide

<400> 68
Gln Leu Arg His Leu Arg Glu
  1               5


<210> 69

<211> 7
<212> PRT
<213> Artificial Sequence

<220>
<223> Description of Artificial Sequence: Synthetic
      peptide

<400> 69
Ser Arg Arg Thr Cys Arg Ala
  1               5


<210> 70
<211> 7
<212> PRT
<213> Artificial Sequence

<220>
<223> Description of Artificial Sequence: Synthetic
      peptide

<400> 70
Gln Arg His Ser Leu Thr Glu
  1               5


<210> 71
<211> 7
<212> PRT
<213> Artificial Sequence

<220>
<223> Description of Artificial Sequence: Synthetic
      peptide

<400> 71
Arg Asn Asp Ala Leu Thr Glu
  1               5


<210> 72
<211> 7
<212> PRT
<213> Artificial Sequence

<220>
<223> Description of Artificial Sequence: Synthetic
      peptide

<400> 72
Arg Ala Asp Asn Leu Thr Glu
  1               5


<210> 73
<211> 7
<212> PRT
<213> Artificial Sequence

<220>
<223> Description of Artificial Sequence: Synthetic
      peptide

<400> 73
Arg Asn Asp Thr Leu Thr Glu
  1               5

<210> 74
<211> 7
<212> PRT
<213> Artificial Sequence

<220>
<223> Description of Artificial Sequence: Synthetic
      peptide

<400> 74
Arg Ser Asp Lys Leu Thr Glu
  1               5


<210> 75
<211> 7
<212> PRT
<213> Artificial Sequence

<220>
<223> Description of Artificial Sequence: Synthetic
      peptide

<400> 75
Gln Ser Gly His Leu Thr Glu
  1               5


<210> 76
<211> 7
<212> PRT
<213> Artificial Sequence

<220>
<223> Description of Artificial Sequence: Synthetic
      peptide

<400> 76
Gln Ser Gly Asp Leu Arg Arg
  1               5


<210> 77
<211> 7
<212> PRT
<213> Artificial Sequence

<220>
<223> Description of Artificial Sequence: Synthetic
      peptide

<400> 77
Ser Lys Lys Ala Leu Thr Glu
  1               5


<210> 78
<211> 7
<212> PRT
<213> Artificial Sequence

<220>
<223> Description of Artificial Sequence: Synthetic
      peptide

<400> 78
Thr Ser Gly Asn Leu Thr Glu

1               5


<210> 79
<211> 7
<212> PRT
<213> Artificial Sequence

<220>
<223> Description of Artificial Sequence: Synthetic
      peptide

<400> 79
Gln Asn Ser Thr Leu Thr Glu
1               5


<210> 80
<211> 7
<212> PRT
<213> Artificial Sequence

<220>
<223> Description of Artificial Sequence: Synthetic
      peptide

<400> 80
Thr Ser His Ser Leu Thr Glu
1               5


<210> 81
<211> 7
<212> PRT
<213> Artificial Sequence

<220>
<223> Description of Artificial Sequence: Synthetic
      peptide

<400> 81
Thr Lys Asn Ser Leu Thr Glu
1               5


<210> 82
<211> 7
<212> PRT
<213> Artificial Sequence

<220>
<223> Description of Artificial Sequence: Synthetic
      peptide

<400> 82
Thr Thr Gly Ala Leu Thr Glu
1               5


<210> 83
<211> 18
<212> DNA
<213> Artificial Sequence

<220>
<223> Description of Artificial Sequence: Synthetic
      primer

<400> 83
cggggggctc ccctggtt                                                    18

<210> 84
<211> 18
<212> DNA
<213> Artificial Sequence

<220>
<223> Description of Artificial Sequence: Synthetic
      primer

<400> 84
gtcaaggcaa ggctggcc                                                    18

<210> 85
<211> 7
<212> PRT
<213> Artificial Sequence

<220>
<223> Description of Artificial Sequence: Synthetic
      peptide

<400> 85
Gln Arg His His Leu Val Glu
  1               5

<210> 86
<211> 7
<212> PRT
<213> Artificial Sequence

<220>
<223> Description of Artificial Sequence: Synthetic
      peptide

<400> 86
Ser Pro Gly His Leu Ile Glu
  1               5

<210> 87
<211> 7
<212> PRT
<213> Artificial Sequence

<220>
<223> Description of Artificial Sequence: Synthetic
      peptide

<400> 87
Ser Cys Ser His Leu Ala Glu
  1               5

<210> 88
<211> 7
<212> PRT
<213> Artificial Sequence

<220>
<223> Description of Artificial Sequence: Synthetic
      peptide

<400> 88
Arg Lys Asp Asn Met Thr Ala
1               5


<210> 89
<211> 7
<212> PRT
<213> Artificial Sequence

<220>
<223> Description of Artificial Sequence: Synthetic
      peptide

<400> 89
Gln Leu Gly His Leu Ile Gln
1               5


<210> 90
<211> 7
<212> PRT
<213> Artificial Sequence

<220>
<223> Description of Artificial Sequence: Synthetic
      peptide

<400> 90
Asn Thr Lys Gln Leu Arg Asn
1               5


<210> 91
<211> 7
<212> PRT
<213> Artificial Sequence

<220>
<223> Description of Artificial Sequence: Synthetic
      peptide

<400> 91
Asn Thr Thr His Leu Glu His
1               5


<210> 92
<211> 7
<212> PRT
<213> Artificial Sequence

<220>
<223> Description of Artificial Sequence: Synthetic
      peptide

<400> 92
Ser Cys Gly His Leu Thr Glu
1               5


<210> 93
<211> 7
<212> PRT
<213> Artificial Sequence

<220>
<223> Description of Artificial Sequence: Synthetic

peptide

<400> 93
Thr His Arg Asp Leu Arg Gln
1               5


<210> 94
<211> 7
<212> PRT
<213> Artificial Sequence

<220>
<223> Description of Artificial Sequence: Synthetic
      peptide

<400> 94
Gln Arg His Ser Leu Thr Glu
1               5


<210> 95
<211> 7
<212> PRT
<213> Artificial Sequence

<220>
<223> Description of Artificial Sequence: Synthetic
      peptide

<400> 95
Asn Cys Asn His Leu Ala Glu
1               5


<210> 96
<211> 7
<212> PRT
<213> Artificial Sequence

<220>
<223> Description of Artificial Sequence: Synthetic
      peptide

<400> 96
Gly Pro Gly Gly Leu Ile Arg
1               5


<210> 97
<211> 7
<212> PRT
<213> Artificial Sequence

<220>
<223> Description of Artificial Sequence: Synthetic
      peptide

<400> 97
Ser Pro Gly His Leu Val Glu
1               5


<210> 98
<211> 7
<212> PRT
<213> Artificial Sequence

<220>
<223> Description of Artificial Sequence: Synthetic
      peptide

<400> 98
Gly Leu Lys Trp Leu Val Val
  1               5


<210> 99
<211> 7
<212> PRT
<213> Artificial Sequence

<220>
<223> Description of Artificial Sequence: Synthetic
      peptide

<400> 99
Asp Leu Arg Trp Leu Val Val
  1               5


<210> 100
<211> 7
<212> PRT
<213> Artificial Sequence

<220>
<223> Description of Artificial Sequence: Synthetic
      peptide

<400> 100
Asn Cys Phe His Leu Thr Glu
  1               5


<210> 101
<211> 7
<212> PRT
<213> Artificial Sequence

<220>
<223> Description of Artificial Sequence: Synthetic
      peptide

<400> 101
Asn Val Arg Thr Leu Asp Thr
  1               5


<210> 102
<211> 7
<212> PRT
<213> Artificial Sequence

<220>
<223> Description of Artificial Sequence: Synthetic
      peptide

<400> 102
Arg Leu Asn Asn Leu Leu Val
  1               5


<210> 103
<211> 7
<212> PRT

<213> Artificial Sequence

<220>
<223> Description of Artificial Sequence: Synthetic
      peptide

<400> 103
Asp Arg Lys Val Leu Ala Thr
  1               5


<210> 104
<211> 7
<212> PRT
<213> Artificial Sequence

<220>
<223> Description of Artificial Sequence: Synthetic
      peptide

<400> 104
His Val Ile Asp Leu Asp His
  1               5


<210> 105
<211> 7
<212> PRT
<213> Artificial Sequence

<220>
<223> Description of Artificial Sequence: Synthetic
      peptide

<400> 105
Asn Thr Thr His Leu Glu His
  1               5


<210> 106
<211> 7
<212> PRT
<213> Artificial Sequence

<220>
<223> Description of Artificial Sequence: Synthetic
      peptide

<400> 106
Asn Val Asn Pro Leu Pro Pro
  1               5


<210> 107
<211> 7
<212> PRT
<213> Artificial Sequence

<220>
<223> Description of Artificial Sequence: Synthetic
      peptide

<400> 107
Asn His Thr Arg Leu Ala Ser
  1               5


<210> 108

<211> 7
<212> PRT
<213> Artificial Sequence

<220>
<223> Description of Artificial Sequence: Synthetic
      peptide

<400> 108
Ile Pro Ala Asp Leu Lys Gln
  1               5


<210> 109
<211> 7
<212> PRT
<213> Artificial Sequence

<220>
<223> Description of Artificial Sequence: Synthetic
      peptide

<400> 109
Arg Arg Arg Gly Trp Arg Lys
  1               5


<210> 110
<211> 7
<212> PRT
<213> Artificial Sequence

<220>
<223> Description of Artificial Sequence: Synthetic
      peptide

<400> 110
Glu Thr Arg Thr Leu Thr Ala
  1               5


<210> 111
<211> 7
<212> PRT
<213> Artificial Sequence

<220>
<223> Description of Artificial Sequence: Synthetic
      peptide

<400> 111
His Ile Ile Asp Leu Asp His
  1               5


<210> 112
<211> 7
<212> PRT
<213> Artificial Sequence

<220>
<223> Description of Artificial Sequence: Synthetic
      peptide

<400> 112
Thr Arg Lys Glu Leu Arg Ser
  1               5

<210> 113
<211> 7
<212> PRT
<213> Artificial Sequence

<220>
<223> Description of Artificial Sequence: Synthetic
      peptide

<400> 113
Asn His Gly His Leu Thr Glu
  1               5


<210> 114
<211> 7
<212> PRT
<213> Artificial Sequence

<220>
<223> Description of Artificial Sequence: Synthetic
      peptide

<400> 114
Gly Arg Leu Ile Leu Leu Gly
  1               5


<210> 115
<211> 7
<212> PRT
<213> Artificial Sequence

<220>
<223> Description of Artificial Sequence: Synthetic
      peptide

<400> 115
Ala Met Gly His Leu Val Glu
  1               5


<210> 116
<211> 7
<212> PRT
<213> Artificial Sequence

<220>
<223> Description of Artificial Sequence: Synthetic
      peptide

<400> 116
Gln Trp His His Leu Thr Asp
  1               5


<210> 117
<211> 7
<212> PRT
<213> Artificial Sequence

<220>
<223> Description of Artificial Sequence: Synthetic
      peptide

<400> 117
Ser Cys Val His Leu Ala Glu

1               5


<210> 118
<211> 7
<212> PRT
<213> Artificial Sequence

<220>
<223> Description of Artificial Sequence: Synthetic
      peptide

<400> 118
Thr Cys Gly His Leu Val Glu
  1               5


<210> 119
<211> 7
<212> PRT
<213> Artificial Sequence

<220>
<223> Description of Artificial Sequence: Synthetic
      peptide

<400> 119
Ser Cys Tyr Lys Leu Arg Glu
  1               5


<210> 120
<211> 7
<212> PRT
<213> Artificial Sequence

<220>
<223> Description of Artificial Sequence: Synthetic
      peptide

<400> 120
Ser Cys Gly Gly Leu Thr Ala
  1               5


<210> 121
<211> 7
<212> PRT
<213> Artificial Sequence

<220>
<223> Description of Artificial Sequence: Synthetic
      peptide

<400> 121
Gln Cys Gly His Leu Val Ala
  1               5


<210> 122
<211> 7
<212> PRT
<213> Artificial Sequence

<220>
<223> Description of Artificial Sequence: Synthetic
      peptide

<400> 122
Gly Cys Ser Lys Leu Arg Glu
1               5


<210> 123
<211> 7
<212> PRT
<213> Artificial Sequence

<220>
<223> Description of Artificial Sequence: Synthetic
      peptide

<400> 123
Ser Cys Gly His Leu Gln Glu
1               5


<210> 124
<211> 7
<212> PRT
<213> Artificial Sequence

<220>
<223> Description of Artificial Sequence: Synthetic
      peptide

<400> 124
Cys Cys Ala His Leu Ser Glu
1               5


<210> 125
<211> 7
<212> PRT
<213> Artificial Sequence

<220>
<223> Description of Artificial Sequence: Synthetic
      peptide

<400> 125
Asp His Gln Asp Leu Thr Ile
1               5


<210> 126
<211> 7
<212> PRT
<213> Artificial Sequence

<220>
<223> Description of Artificial Sequence: Synthetic
      peptide

<400> 126
Asp Leu Arg Ser Cys Arg Ser
1               5


<210> 127
<211> 7
<212> PRT
<213> Artificial Sequence

<220>
<223> Description of Artificial Sequence: Synthetic

peptide

<400> 127
Gln Arg Arg His Leu Leu Ser
1               5


<210> 128
<211> 7
<212> PRT
<213> Artificial Sequence

<220>
<223> Description of Artificial Sequence: Synthetic
      peptide

<400> 128
Leu Arg Arg Gln Leu Ala His
1               5


<210> 129
<211> 7
<212> PRT
<213> Artificial Sequence

<220>
<223> Description of Artificial Sequence: Synthetic
      peptide

<400> 129
Arg Met Arg Asn Leu Gln Lys
1               5


<210> 130
<211> 7
<212> PRT
<213> Artificial Sequence

<220>
<223> Description of Artificial Sequence: Synthetic
      peptide

<400> 130
Asn Leu Gln His Leu Gly Glu
1               5


<210> 131
<211> 18
<212> DNA
<213> Artificial Sequence

<220>
<223> Description of Artificial Sequence: Synthetic
      primer

<400> 131
aaccagggga gccccccg                                                18


<210> 132
<211> 18
<212> DNA
<213> Artificial Sequence

<220>

<223> Description of Artificial Sequence: Synthetic
      primer

<400> 132
   ttggtcccct cggggggc                                                    18


<210> 133
<211> 15
<212> PRT
<213> Artificial Sequence

<220>
<223> Description of Artificial Sequence: Synthetic
      peptide

<400> 133
Thr Gly Gly Gly Gly Ser Gly Gly Gly Gly Thr Gly Glu Lys Pro
  1               5                  10                  15


<210> 134
<211> 7
<212> PRT
<213> Artificial Sequence

<220>
<223> Description of Artificial Sequence: Synthetic
      peptide

<400> 134
Asn Leu Gln Asn Leu Gly Glu
  1               5


<210> 135
<211> 7
<212> PRT
<213> Artificial Sequence

<220>
<223> Description of Artificial Sequence: Synthetic
      peptide

<400> 135
Gln Ser Gly His Cys Arg Ala
  1               5


<210> 136
<211> 7
<212> PRT
<213> Artificial Sequence

<220>
<223> Description of Artificial Sequence: Synthetic
      peptide

<400> 136
Arg Ser Asp Lys Cys Arg Ala
  1               5


<210> 137
<211> 7
<212> PRT
<213> Artificial Sequence

<220>
<223> Description of Artificial Sequence: Synthetic
      peptide

<400> 137
His Lys Asn Ala Cys Arg Ala
  1               5


<210> 138
<211> 7
<212> PRT
<213> Artificial Sequence

<220>
<223> Description of Artificial Sequence: Synthetic
      peptide

<400> 138
Thr Ser Gly Ala Leu Thr Glu
  1               5


<210> 139
<211> 7
<212> PRT
<213> Artificial Sequence

<220>
<223> Description of Artificial Sequence: Synthetic
      peptide

<400> 139
Thr Ser Gly Thr Leu Thr Glu
  1               5


<210> 140
<211> 7
<212> PRT
<213> Artificial Sequence

<220>
<223> Description of Artificial Sequence: Synthetic
      peptide

<400> 140
Thr Thr Gly Gly Leu Thr Glu
  1               5


<210> 141
<211> 7
<212> PRT
<213> Artificial Sequence

<220>
<223> Description of Artificial Sequence: Synthetic
      peptide

<400> 141
Asp Cys Arg Thr Leu Ala Glu
  1               5


<210> 142
<211> 7
<212> PRT

<213> Artificial Sequence

<220>
<223> Description of Artificial Sequence: Synthetic
      peptide

<400> 142
Asp Cys Arg Ala Leu Thr Glu
  1               5


<210> 143
<211> 7
<212> PRT
<213> Artificial Sequence

<220>
<223> Description of Artificial Sequence: Synthetic
      peptide

<400> 143
Asp Cys Arg Thr Leu Thr Glu
  1               5


<210> 144
<211> 7
<212> PRT
<213> Artificial Sequence

<220>
<223> Description of Artificial Sequence: Synthetic
      peptide

<400> 144
Ser Cys Arg Thr Leu Ala Glu
  1               5


<210> 145
<211> 7
<212> PRT
<213> Artificial Sequence

<220>
<223> Description of Artificial Sequence: Synthetic
      peptide

<400> 145
Ser Cys Arg Ala Leu Thr Glu
  1               5


<210> 146
<211> 7
<212> PRT
<213> Artificial Sequence

<220>
<223> Description of Artificial Sequence: Synthetic
      peptide

<400> 146
Ser Cys Arg Thr Leu Thr Glu
  1               5


<210> 147

<211> 7
<212> PRT
<213> Artificial Sequence

<220>
<223> Description of Artificial Sequence: Synthetic
      peptide

<400> 147
Asp Lys Lys Ala Leu Ala Glu
  1               5


<210> 148
<211> 7
<212> PRT
<213> Artificial Sequence

<220>
<223> Description of Artificial Sequence: Synthetic
      peptide

<400> 148
Asp Lys Lys Thr Leu Ala Glu
  1               5


<210> 149
<211> 7
<212> PRT
<213> Artificial Sequence

<220>
<223> Description of Artificial Sequence: Synthetic
      peptide

<400> 149
Asp Lys Lys Ala Leu Thr Glu
  1               5


<210> 150
<211> 7
<212> PRT
<213> Artificial Sequence

<220>
<223> Description of Artificial Sequence: Synthetic
      peptide

<400> 150
Asp Lys Lys Thr Leu Thr Glu
  1               5


<210> 151
<211> 7
<212> PRT
<213> Artificial Sequence

<220>
<223> Description of Artificial Sequence: Synthetic
      peptide

<400> 151
Ser Lys Lys Ala Leu Ala Glu
  1               5

<210> 152
<211> 7
<212> PRT
<213> Artificial Sequence

<220>
<223> Description of Artificial Sequence: Synthetic
      peptide

<400> 152
Ser Lys Lys Thr Leu Ala Glu
  1               5


<210> 153
<211> 7
<212> PRT
<213> Artificial Sequence

<220>
<223> Description of Artificial Sequence: Synthetic
      peptide

<400> 153
Ser Lys Lys Thr Leu Thr Glu
  1               5


<210> 154
<211> 7
<212> PRT
<213> Artificial Sequence

<220>
<223> Description of Artificial Sequence: Synthetic
      peptide

<400> 154
His Thr Gly Ala Leu Leu Glu
  1               5


<210> 155
<211> 7
<212> PRT
<213> Artificial Sequence

<220>
<223> Description of Artificial Sequence: Synthetic
      peptide

<400> 155
His Thr Gly Asn Leu Leu Glu
  1               5


<210> 156
<211> 7
<212> PRT
<213> Artificial Sequence

<220>
<223> Description of Artificial Sequence: Synthetic
      peptide

<400> 156
Asp Pro Gly Asn Leu Val Arg

1               5

<210> 157
<211> 7
<212> PRT
<213> Artificial Sequence

<220>
<223> Description of Artificial Sequence: Synthetic
      peptide

<400> 157
Asp Ser Gly Asn Leu Val Arg
  1               5

<210> 158
<211> 5
<212> PRT
<213> Artificial Sequence

<220>
<223> Description of Artificial Sequence: Synthetic
      peptide

<400> 158
Ala Ala Ala Arg Ala
  1               5